# EUROPÄISCHE PATENTANMELDUNG

(11) **EP 2 033 971 A1**
(43) Veröffentlichungstag der Anmeldung: **11.03.2009**
(21) Anmeldenummer: 07115856.2
(22) Anmeldetag: 06.09.2007
(51) Int. Cl.: C07K 14/705, C07K 16/28

(54) **Bone Morphogenetic Protein (BMP)-bindende Domänen von Proteinen der Repulsive Guidance Molecule (RGM) Proteinfamilie und funktionale Fragmente davon sowie deren Verwendung**

(71) Anmelder: Abbott GmbH & Co. KG, 65205 Wiesbaden (DE)
(72) Erfinder: Müller, Bernhard, 67345 Neustadt (DE); Schaffar, Gregor, 68185 Mannheim (DE); Schmidt, Martin, 64625 Bensheim (DE); Meyer, Axel, 68723 Schwetzingen (DE)
(74) Vertreter: Reitstötter - Kinzebach

(57) **Zusammenfassung**

Die vorliegende Erfindung betrifft die Identifizierung und Verwendung von Bone Morphogenetic Protein (BMP)-bindenden Domänen von Mitgliedern der Repulsive Guidance Molecule (RGM) Proteinfamilie sowie davon abgeleitete Polypeptidfragmente und Fusionsproteine. Die erfindungsgemäßen Domänen beziehungsweise Peptidfragmente und Fusionsproteine eignen sich als Mittel zur aktiven oder passiven Immunisierung von Individuen bzw. als diagnostische und therapeutische Mittel zur Verwendung bei Krankheiten oder Krankheitszuständen, an deren Entstehung oder Verlauf ein Mitglied der RGM-Familie und ein diesem Molekül zugeordneter zellulärer Rezeptor wie insbesondere Neogenin und/oder BMP beteiligt ist. Die Erfindung betrifft außerdem monoklonale und polyklonale Antikörper gegen die erfindungsgemäßen Bindungsdomänen und gegen die davon abgeleiteten Polypeptide sowie Verfahren zur Herstellung der erfindungsgemäßen Domänen, Polypeptide, Fusionsproteine und Antikörper.

## Beschreibung

Die vorliegende Erfindung betrifft die Identifizierung und Verwendung von Bone Morphogenetic Protein (BMP)-bindenden Domänen von Mitgliedern der Repulsive Guidance Molecule (RGM) Proteinfamilie sowie davon abgeleitete Polypeptidfragmente und Fusionsproteine. Die erfindungsgemäßen Domänen beziehungsweise Peptidfragmente und Fusionsproteine eignen sich als Mittel zur aktiven oder passiven Immunisierung von Individuen bzw. als diagnostische und therapeutische Mittel zur Verwendung bei Krankheiten oder Krankheitszuständen, an deren Entstehung oder Verlauf ein Mitglied der RGM-Familie und ein diesem Molekül zugeordneter zellulärer Rezeptor wie insbesondere Neogenin und/oder BMP beteiligt ist. Die Erfindung betrifft außerdem monoklonale und polyklonale Antikörper gegen die erfindungsgemäßen Bindungsdomänen und gegen die davon abgeleiteten Polypeptide sowie Verfahren zur Herstellung der erfindungsgemäßen Domänen, Polypeptide, Fusionsproteine und Antikörper.

### Stand der Technik

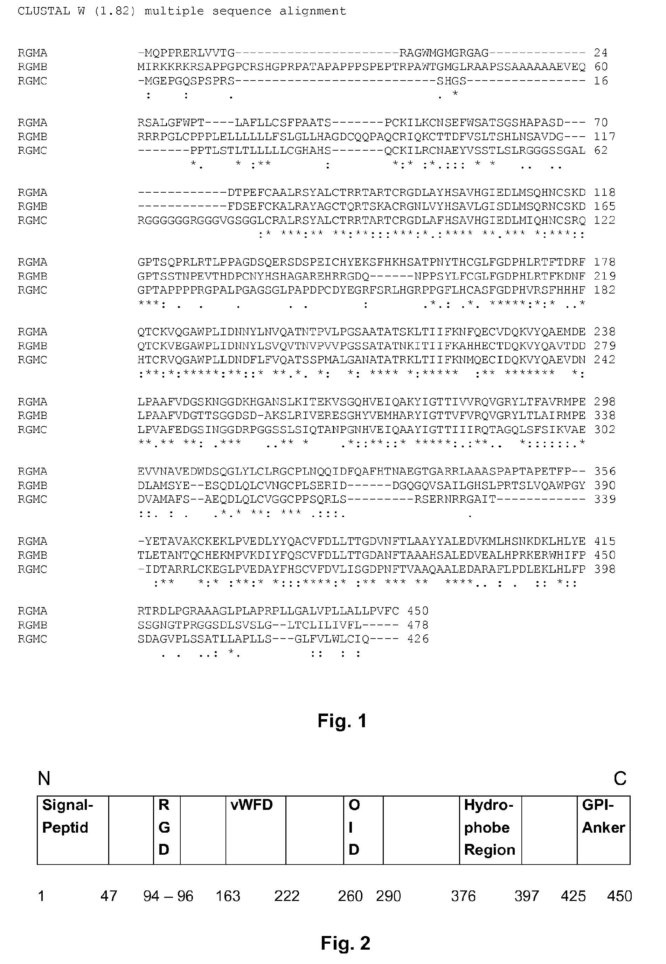

Die Funktion der Mitglieder der RGM-Proteinfamilie wurde erstmals von Monnier, P.P. et al, Nature, 419, p392-395, 2002 beschrieben. Diese Familie umfasst drei bisher bekannte Mitglieder, die als RGM A, RGM B (auch bezeichnet als DRAGON) und RGM C (auch bezeichnet als Hemojuvelin) bezeichnet werden (Niederkofler V. et al. J. Neurosci. 24, 808-18, 2004). Es handelt sich dabei um Glykoproteine die über einen Lipid-Anker (Glycosylphosphatidylinositol-Anker = GPI-Anker) an die Plasmamembran gebunden sind. Die Mitglieder dieser Proteinfamilie weisen keine ausgedehnte Sequenzhomologie zu anderen Proteinen auf und als Strukturmerkmale sind im Wesentlichen folgende Bereiche identifiziert worden: ein N-terminales Signalpeptid; eine RGD-Sequenz; eine proteolytische Spaltstelle um die Aminosäuresequenz GDPH; eine Struktur-homologe von-Willebrand-Faktor-Domäne (vWF D); eine hydrophobe Sequenz in Nähe des C-Terminus und eine C-terminale GPI-Anker Consensus Sequenz (vgl. auch Figur 2).

Beim Menschen sind die kodierenden Sequenzen für RGM A auf Chromosom 15, für RGM B auf Chromosom 5 und für RGM C auf Chromosom 1 lokalisiert. Es ist ein charakteristisches Expressionsmuster zu beobachten: RGM A und B werden insbesondere in adultem Gehirn und Rückenmark, RGM C wird insbesondere in Skelettmuskel, Leber und Herzmuskel, und RGM B außerdem im Knorpelgewebe exprimiert.

RGM Proteine wurden ursprünglich identifiziert als Kandidatenproteine, die bei der Bildung topographischer neuronaler Projektionen eine wichtige Rolle spielen (Stahl B. et al. Neuron 5:p 735-43, 1990; Mueller B.K. et al. Curr. Biol. 6, p. 1497-1502, 1996; Mueller BK in Molecular Basis of Axon Growth and Nerve Pattern Formation, Edited by H. Fujisawa, Japan Scientific Societies Press, 215-229, 1997). Ihre Fähigkeit abstoßend (repulsiv) oder hemmend (inhibitorisch) auf wachsende Nervenfasern zu wirken, war ein entscheidendes funktionelles Merkmal, welches bei ihrer Isolierung, Klonierung und Charakterisierung eine wichtige Rolle spielte. In einfachen zellulären Testsystemen war die Aktivität gut nachweisbar. In zwei unterschiedlichen zellulären Assays wirken RGM Proteine hemmend oder repulsiv. Im Kollapstest werden zu auswachsenden Nervenfasern RGM-Proteine gegeben. Die Bindung von RGM and den RGM-Rezeptor löst eine heftige Reaktion aus, bei der sämtliche membranösen Elemente des neuronalen Wachstumskegels eingezogen werden. Der ursprüngliche ausgedehnte hand-ähnliche Wachstumskegel wird dabei zu einem dünnen Faden umgewandelt. In Gegenwart von RGM bleiben die Nervenfasern gehemmt, ziehen sich stark zurück und können ihr Wachstum nicht mehr weiter fortsetzen.

RGM-Proteine üben einen Teil ihrer Wirkung durch Bindung an den RGM-Rezeptor Neogenin aus (Rajagopalan S. et al., Nat Cell Biol. 6 p.756-62, 2004). Neogenin ist nahe verwandt mit dem DCC Rezeptor (Deleted in Colorecal Cancer). Beide Rezeptoren sind Mitglieder der Immunoglobulin-Superfamilie und besitzen eine extrazelluläre, eine Transmembran- und eine intrazelluläre Domäne. Beide wurden als Rezeptoren für einen weiteren Liganden, das Netrin-1, beschrieben, aber lediglich Neogenin, nicht jedoch DCC, bindet RGM Proteine. Die extrazellulären Domänen dieser Rezeptoren bestehen aus 4 Immunoglobulin-ähnlichen Domänen, auf die 6 Fibronektin-Repeat Domänen folgen.

Am besten verstanden ist die Funktion von RGM A im Nervensystem und besonders auffällig ist dessen Wirkung, in niedrigsten Konzentrationen das Wachstum von Nervenfasern zu hemmen. Eine Verletzung des zentralen Nervensystems beim erwachsenen Menschen und bei der adulten Ratte führt zu einer Akkumulation von RGM-Proteinen an der Läsionsstelle (Schwab JM. et al. Arch. Neurol. Bd,62, 1561-1568, 2005; Schwab J.M. et al. Eur. J. Neurosci. 21 :p. 387-98, 2005). Dadurch wird das erneute Auswachsen der verletzten Nervenfasern verhindert und es kommt zu permanenten, in Abhängigkeit vom Ort der Läsionsstelle, mehr oder weniger schweren Funktionsausfällen. Diese für das Nervenfaserwachstum inhibitorische Aktivität von RGM wird durch Bindung an den Rezeptor Neogenin vermittelt (Rajagopalan S. et al., a.a.O.). Der gleiche Rezeptor vermittelt über die Bindung von Netrin-1 aber auch eine gegenteilige, das Nervenfaserwachstum stimulierende Wirkung. Wird das RGM A Protein an der Läsionsstelle im Rückenmark der Ratte durch einen polyklonalen Antikörper neutralisiert, regenerieren die Nervenfasern über die Verletzungsstelle hinweg, bilden neue synaptische Kontakte aus und führen dadurch zu signifikanten funktionalen Verbesserungen (Hata K. et al. J. Cell Biol. 173: 47 - 58, 2006).

Neueste Ergebnisse weisen darauf hin, dass die RGM-Proteine auch wichtige Aufgaben erfüllen im zentralen und peripheren Nervensystem, bei der Regulation des Eisenstoffwechsels, bei Tumorerkrankungen, bei Entzündungsvorgängen und bei der Bildung von Knochen und Knorpelgewebe.

Aus der WO 2007/039256 sind die Neuritenwachstum-inhibierenden Domänen von Proteinen der RGM-Familie bekannt. So wurde z. B. für RGM A im Sequenzbereich 260-290 signifikante inhibitorische Aktivität lokalisiert.

Weiterhin ist bekannt, dass RGM A, B und C mit verschiedenen Mitgliedern der BMP-Familie interagieren können. BMPs sind Mitglieder der TGF-β-Superfamilie von Liganden, welche an zahlreichen physiologischen und pathophysiologischen Prozessen beteiligt sind. BMPs üben ihre Funktion über einen speziellen Signaltransduktionsweg aus, an dessen Anfang die Bindung des BMP-Liganden an eine Kombination zweier Typen von Serin/Threonin-Kinase-Rezeptoren steht. Eine Interaktion mit RGM konnte bisher für BMP-2, -4, -5, -6 und -12 nachgewiesen werden (vgl. z. B. Babitt, J. L. et al., Nature Genetics, 2006, Bd. 48, 5, 531-539; Babitt, J. L. et al., J. Biol. Chem., 2005, Bd. 280, 33, 29820-29827; Babitt, J. L. et al., The Journal of Clinical Investigation, 2007, Bd. 117, 7, 1933-1939; Samad, T. A. et al., J. Biol. Chem., 2005, Bd. 280, 14, 14122-14129; Halbrooks et al. J. Molecular Signaling 2, 4: 2007: publiziert in elektronischer Form).

BMP-Bindungsdomänen von RGM-Proteinen konnten bisher noch nicht beschrieben werden.

Aufgabe der Erfindung ist daher die Lokalisierung von BMP-Bindungsdomänen der RGM-Proteine und deren Charakterisierung.

### Kurze Beschreibung der Erfindung

Obige Aufgabe wurde überraschenderweise durch Isolierung und Charakterisierung der BMP-Bindungsdomänen von humanen RGM Proteinen, insbesondere RGM A, und aktiver Polypeptidfragmente davon, gelöst.

### Figurenbeschreibung

Figur 1 zeigt das Sequenz-Alignment der humanen Formen von RGM A (GenBank # NP_064596.1) RGM B (GenBank # NP_001012779) und RGM C (GenBank # NP_998818.1).

Figur 2 zeigt in schematischer Darstellung den Aufbau von RGM-Molekülen. Zwischen dem N-terminalen Signalpeptid und dem C-terminalen GPI-Anker sind die RGD-Sequenz, die von Willebrand-Faktor-Domäne (vWF D) sowie eine hydrophobe Sequenz im Bereich des C-Terminus vor der Ankerregion dargestellt. Zwischen vWFD und hydrophober Region im Bereich um die Positionen 260-290 befindet sich die Neuritenwachstum-inhibierenden Domäne (OID). Unterhalb des Diagramms sind die entsprechenden Aminosäurepositionen für humanes RGM angegeben; die proteolytische Spaltstelle des humanem RGM Aliegt zwischen den Aminosäuren 168 und 169.

Figur 3 zeigt das Ergebnis eines In-vitro-Interaktionsassays zwischen BMP 4 und verschiedenen immobilisierten RGM A-Fc-Fusionsproteinen.

Figur 4 zeigt das Ergebnis eines In-Vitro-Interaktionsassays zum Vergleich der Wechselwirkung verschiedener immobilisierter RGM A-Fc-Fusionsproteine mit BMP-4- bzw. BMP-2.

Figur 5 zeigt das Ergebnis eines In-Vitro-Interaktionsassays zwischen immobilisiertem BMP-4 und verschiedenen RGM A-Fc-Fusionsproteinen.

Figur 6 zeigt die Ergebnisse von In-Vitro-Interaktionsassays zwischen immobilisiertem BMP-4 und unterschiedlichen Konzentrationen erfindungsgemäßer Fusionsproteine 47-90-Fc (Figur 6A), 47-168-Fc (Figur 6B) und 316-386-Fc (Figur 6C).

Figur 7 zeigt die Ergebnisse von zwei unterschiedlichen Neuritenwachstumstests mit humanen Nervenzellen (SH-SY5Y in Figur 7A, NTera in Fig. 7B). Beide hRGM A Fragmente - 786 (47 - 168) und 790 (316 - 386) - hemmen das Neuritenwachstum, wobei das Fragment 47 - 168-Fc deutlich stärker wirksam ist.

### Detaillierte Beschreibung der Erfindung

### I. Erläuterungen allgemeiner Begriffe

Als "Rezeptoren" bezeichnet man im Rahmen der vorliegenden Erfindung insbesondere an einer Zellmembran gebundene Oberflächenmoleküle, welche mit einem, beispielsweise löslichen, Liganden in Wechselwirkung treten können und als Folge dieser Wechselwirkung ein beispielsweise in das Zellinnere gerichtetes Signal oder eine Signalkaskade (auch als Signalling bezeichnet) auslösen können.

Als "Ligand" bezeichnet man einen natürlichen, d.h. in vivo gebildeten oder künstlich erzeugten, nieder- oder hochmolekularen Bindungspartner für einen "Rezeptor". Der Ligand ist vorzugsweise in der extrazellulären Umgebung frei beweglich.

Als "Immunogen" bezeichnet man ein erfindungsgemäßes Peptidfragment in glycosylierter oder nicht-glycosylierter Form, welches zur Induktion der Bildung von Antikörpern gegen das Immunogen geeignet ist. Gegebenenfalls kann eine Bindung des Immunogens (als Hapten) an einen makromolekularen Träger von Vorteil sein.

Als "Epitop" oder antigene Determinante bezeichnet man den die Spezifität eines Antikörpers bestimmenden Bereich eines Antigens, wie z.B. eines Proteins. Wird dieses Epitop, beispielsweise durch äußere Einflüsse, wie z.B. eine Wechselwirkung eines Proteins mit einem Liganden, in einem Abschnitt des Proteins neu gebildet oder auf der zugänglichen Moleküloberfläche exprimiert, so spricht man von einem "Neoepitop".

Als "Domäne" eines Proteins oder Antikörpers bezeichnet man eine durch alpha-Helix- und/oder beta-Faltblattelemente gebildete komplexe, innerhalb des Proteins abgegrenzte Struktur.

Werden keine anderen Angaben gemacht, so umfasst der Begriff "erfindungsgemäßes RGM-Protein" sowohl die BMP-Bindungsdomänen als auch davon abgeleitete Polypeptide eines Mitglieds der Familie von RGM-Molekülen, insbesondere RGM A, B und C. Insbesondere werden auch funktionale, einen BMP-Signaltransduktionsweg "stimulierende" Polypeptidfragmente mit umfasst. Zusätzlich können die erfindungsgemäßen Polypeptide auch "inhibitorisch aktive" Polypeptide mit umfassen, insbesondere solche, welche an Neogenin binden, bzw. eine das Nervenfaserwachstum inhibierende Aktivität (veranschaulicht durch einen hierin beschriebenen Neuritenwachstumstest) besitzen

Ein "Binden" der erfindungsgemäßen Domänen oder Polypeptide ist im weitesten Sinne als eine zeitlich gegebenenfalls begrenzte Wechselwirkung jeglicher Art mit einem Bindungspartner, wie z.B. BMP oder Neogenin, zu verstehen. Die Bindung kann dabei spezifisch oder unspezifisch, vorzugsweise aber spezifisch erfolgen. Ein solches Binden ist mit Hilfe geeigneter Bindungsassays, wie z.B. den hierin im experimentellen Teil beschriebenen Bindungstests, nachzuweisen. Insbesondere können dabei die erfindungsgemäßen Domänen oder Polypeptide durch Ausbildung kovalenter oder nichtkovalenter, wie z.B. ionischer und/oder hydrophober Wechselwirkungen mit dem jeweiligen Bindungspartner in Kontakt kommen. Insbesondere kann die Wechselwirkung ausreichend sein, um eine durch den Bindungspartner vermittelte Eigenschaft, wie z.B. eine biologische Funktion, beispielsweise eine Wechselwirkung des Bindungspartners mit einem dritten Partner, zu modulieren, d.h. positiv oder negativ zu beeinflussen, wie z.B. zu fördern oder partiell oder vollständig zu inhibieren.

"Spezifisch" ist eine erfindungsgemäße Bindung insbesondere dann, wenn die Anzahl der verschiedenen Bindungspartner oder verschiedenen Bindungspartnerklassen zahlenmäßig begrenzt ist. Insbesondere sollte die Bindung nicht mit mehr als 10, wie z.B. 1, 2, 3, 4 oder 5 verschiedenen Bindungspartnern oder Bindungspartnerklassen erfolgen. Beispielsweise stellt BMP eine Bindungspartner-Klasse dar. Spezifität ist ebenfalls gegeben, wenn eine Wechselwirkung zwar mit mehreren Bindungspartnern erfolgt, die Wechselwirkung aber nur mit einer begrenzten Anzahl von Bindungspartnern davon ausreichend intensiv ist, um eine biologische Funktion in obigem Sinne zu beeinflussen. Beispielsweise ist erfindungsgemäß Spezifität gegeben, wenn eine erfindungsgemäße Domänen oder ein Polypeptid mit wenigstens einem BMP, insbesondere ausgewählt unter BMP-2, BMP-4, BMP-5, BMP-6 und BMP-12, insbesondere an BMP-2 und/oder BMP-4 bindet; und/oder an Neogenin bindet.

"Inhibierende" oder "inhibitorisch aktive" Polypeptide sind solche, die das Nervenzellenwachstum in einen hierin beschriebenen Nervenzellenwachstumstest reduzieren oder vollständig inhibieren.

Die oben beschriebene "stimulierende" und die "inhibitorische" Aktivität können unabhängig voneinander für ein bestimmtes Polypeptid gegeben sein; vorzugsweise ist aber die BMP-Signalweg "stimulierende" Aktivität immer, die Nervenzellenwachstum "inhibierende" Aktivität nur optional vorhanden.

Die "BMP-Signaltransduktion" stimulierende Aktivität ist eine Aktivität, die von mindestens einem BMP-Protein, ausgewählt unter BMP-2, -4, -5, -6 und -12, ausgelöst werden kann. Diese stimulierende Aktivität ist gegeben, wenn ein erfindungsgemäßes BMP-bindendes Polypeptid, veranschaulicht durch einen hierin beschriebenen In-vitro-Bindungstest, mit wenigstens einem BMP-Molekül, ausgewählt unter BMP-2, -4, -5, -6 und -12 wechselwirkt.

Werden keine anderen Angaben gemacht so steht "RGM" für RGM A, B und C insbesondere RGM A.

"Neogenin" oder "Neogenin-Rezeptor" sind synonyme Begriffe und bezeichnen insbesondere Neogenin aus einem Säuger, insbesondere humanes Neogenin.

Unter einer "funktionalen Verknüpfung" einer BMP-bindenden Domäne oder eines BMP-bindenden Polypetids mit einer weiteren Aminosäurensequenz versteht man insbesondere eine kovalente, peptidische Verknüpfung, welche eine Bindung der Domäne oder des Polypeptids an wenigstens ein BMP-Molekül ausgewählt unter BMP-2, -4, -5, -6 und -12 erlaubt.

### II. Spezielle Gegenstände der Erfindung

Ein erster Gegenstand der Erfindung betrifft Bone Morphogenetic Protein (BMP)-bindende Domänen oder bindende Peptidfragmente des Repulsive Guidance Molecule (RGM) in glykosylierter oder insbesondere nicht-glykosylierter Form, vorzugsweise abgeleitet von RGM von Säugern, wie z.B. Mensch, Ratte oder Maus, oder Geflügel, wie z.B. Huhn. Werden keine anderen Angaben gemacht, so umfasst der Begriff "bindende Domäne" jegliche an wenigstens ein BMP bindende Polypeptide, die von einem RGM abgeleitet sind.

Eine bevorzugte Ausführungsform betrifft BMP-bindende Domänen, abgeleitet von einem humanen RGM A gemäß SEQ ID NO: 2, humanen RGM B gemäß SEQ ID NO:4 oder humanen RGM C gemäß SEQ ID NO: 6. Dabei umfassen die bindenden Domänen insbesondere eine Aminosäuresequenz mit einer Länge von bis zu 170, wie z.B. bis zu 125, bis zu 100, bis zu 80, bis zu 60, bis zu 50, oder bis zu 40 vorzugsweise zusammenhängenden Aminosäureresten aus einen Aminosäuresequenzbereich von RGM und zwar N-terminal zur vWF-Domäne oder C-terminal zur Nervenfaserwachstum-inhibierenden Domäne (OID) des RGM, wie insbesondere RGM A, oder aus den durch ein Sequenzalignment ableitbaren korrespondierenden Sequenzbereichen von RGM B und C.

Gegenstand der Erfindung sind insbesondere solche BMP-bindenden Domänen mit einer Länge von etwa 30 bis 150 zusammenhängenden Aminosäureresten, sowie funktionale Derivate davon und Fusionsproteine, enthaltend wenigstens eine BMP-bindende Domäne in funktionaler Verknüpfung mit wenigstens einer weiteren, davon verschiedenen, Aminosäuresequenz.

Gegenstand der Erfindung sind auch solche BMP-bindenden Domänen, gekennzeichnet durch wenigstens eine der folgenden Teilsequenzen gemäß SEQ ID NO: 7 und 8:
X₁C(K/R)IX₂(K/R)CX₃(S/T/A)(E/D)(FN)X₄SX₅T (SEQ ID NO:7)
worin X₁ bis X₅ beliebige Aminosäurereste bedeuten; oder
X₆CX₇ALRX₈YAX₉CTX₁₀RTX₁₁ (SEQ ID NO:8)
worin X₆ bis X₁₁ beliebige Aminosäurereste bedeuten;
oder eine Teilsequenz der Formel
(SEQ ID NO:7) -Link1- (SEQ ID NO:8)

worin Link1 für eine SEQ ID NO: 7 und 8 verbrückende Aminosäuresequenz steht, umfassend 10 bis 45 wie etwa 13 bis 28 zusammenhängende beliebige Aminosäurereste.

Insbesondere steht dabei
X₁ für Pro oder Gln
X₂ für Leu oder Gln
X₃ für Asn oder Thr
X₄ für Val oder Trp
X₅ für Ser, Ala oder Leu
X₆ für Phe oder Leu
X₇ für Ala, Lys oder Arg
X₈ für Ser oder Ala
X₉ für Leu oder Gly
X₁₀ für Arg oder Gln und/oder
X₁₁ für Ala oder Ser.

Als konkrete Beispiele für SEQ IQ NO: 7 sind zu nennen:
PCKILKCNSEFWSAT
OCRIOKCTTDFVSLT
OCKILRCNAEYVSST

Als konkrete Beispiele für SEQ IQ NO: 8 sind zu nennen:
FCAALRSYALCTRRTA
FCKALRAYAGCTORTS
LCRALRSYALCTRRTA

Als konkrete Beispiele für den Linker Link1 sind zu nennen:
SGSHAPASDDTPE
SHLNSAVDGFDSE
LSLRGGGSSGALRGGGGGGRGGGVGSGG

Beispiele für BMP-bindende Domänen umfassen eine Aminosäuresequenz im Bereich der Aminosäurepositionen 30 bis 180 gemäß SEQ ID NO:2, im Bereich der Aminosäurepositionen 80 bis 230 gemäß SEQ ID NO:4, oder im Bereich der Aminosäurepositionen 20 bis 150 gemäß SEQ ID NO:6, oder funktionale Neogenin-Rezeptor-bindende Fragmente davon. Diese Bindungsdomäne (und davon abgeleitete Fragmente) wird auch als hoch-affine BMP-Bindungsdomäne bezeichnet. Insbesondere kann die hoch-affine BMP-Bindungsdomäne auch eine hoch-affine Wechselwirkung zu Neogenin aufweisen und kann somit auch als hoch-affine Neogenin-Bindungsdomäne bezeichnet werden. Ein Beispiel für eine niedrig affine Neogenin-Bindungsdomäne ist dagegen z.B. das RGM A Fragment 218-284 gemäß SEQ ID NO:2 beschrieben in der WO 2007/039256, auf deren Offenbarung hiermit ausdrücklich Bezug genommen wird. Eine niedrig-affine Bindung liegt beispielsweise, ohne darauf beschränkt zu sein, vor, wenn die KD > 1 µM, wie z.B. 2 bis 10 µM, ist; eine hoch-affine Bindung kann z.B. vorliegen, wenn die KD < 10 nM, wie z.B. 1 bis 9 mM, ist.

Beispiele für hoch affine BMP-bindende Domänen sind solche, umfassend eine der folgenden Aminosäuresequenzen von SEQ ID NO:2:
Aminosäureposition von etwa 47 bis etwa 168 oder etwa 41 bis etwa 168
Aminosäureposition von etwa 47 bis etwa 90 oder etwa 41 bis etwa 90 oder
Aminosäureposition von etwa 75 bis etwa 121;
oder eine der folgenden Aminosäuresequenzen von SEQ ID NO:4:
Aminosäureposition von etwa 94 bis etwa 209
Aminosäureposition von etwa 94 bis etwa 137 oder
Aminosäureposition von etwa 122 bis etwa 168;
eine der folgenden Aminosäuresequenzen von SEQ ID NO:6:
Aminosäureposition von etwa 36 bis etwa 172
Aminosäureposition von etwa 36 bis etwa 94 oder
Aminosäureposition von etwa 80 bis etwa 125;
oder ein funktionales, BMP-bindendes Fragment davon, wie z.B. ein Fragment einer der obigen Sequenzen, die C- und /oder N-terminal um 1, 2, 3, 4, 5, 6, 7, 8, 9, 10, 12, 15, oder 20 Aminosäurereste verkürzt sein kann, ohne die Fähigkeit zur BMP-Bindung (nachweisbar in einem hierin beschriebenen Bindungstest) zu verlieren.

Weitere konkrete Beispiele erfindungsgemäßer Domänen sind BMP-bindende Domänen oder bindende Fragmente davon, welche wenigstens 10 zusammenhängende Aminosäurereste aus dem Sequenzbereich etwa von Position 47 bis etwa 168 gemäß SEQ ID NO: 2, aus dem Sequenzbereich etwa von Position 94 bis etwa 209 gemäß SEQ ID NO: 4 oder aus dem Sequenzbereich etwa von Position 36 bis 172 gemäß SEQ ID NO: 6 umfassen.

Ein weiterer Gegenstand der Erfindung betrifft BMP-Bindungsdomänen, welche etwa C-terminal zur OID (vgl. Figur 2) gelegen sind. Diese Bindungsdomänen (und davon abgeleitete Fragmente) werden auch als niedrig-affine BMP-Bindungsdomänen bezeichnet, da deren Bindungsaffinität relativ zu den oben genannten hoch-affinen BMP-Bindungsdomänen unter vergleichbaren Versuchsbedingungen geringer sein kann.

Gegenstand der Erfindung sind somit auch solche BMP-bindenden Domänen, gekennzeichnet durch wenigstens eine der folgenden Teilsequenzen gemäß SEQ ID NO: 26 und 27:
LX₂₀LC(V/L)X₂₁GCP (SEQ ID NO:26)
worin X₂₀ und X₂₁ beliebige Aminosäurereste bedeuten; oder
TAX₂₂X₂₃X₂₄C(K/H)EX₂₅(L/M)PV(E/K)DX₂₆Y(FN)(Q/H)(A/S)CVFD(V/L)LX₂₇(T/S)G (SEQ ID NO:27)
worin X₂₂ bis X₂₇ beliebige Aminosäurereste bedeuten;
oder eine Teilsequenz der Formel
(SEQ ID NO:26)-Link2- (SEQ ID NO:27)
worin Link2 für eine SEQ ID NO: 26 und 27 verbrückende Aminosäuresequenz steht, umfassend 10 bis 45 wie etwa 19 bis 38 zusammenhängende beliebige Aminosäurereste.

Insbesondere steht dabei
X₂₀ für Tyr oder Gln
X₂₁ für Arg, Asn oder Gly
X₂₂ für Arg, Asn oder Val
X₂₃ für Ala, Thr oder Arg
X₂₄ für Lys, Gln oder Leu
X₂₅ für Lys oder Gly
X₂₆ für Leu, Ile oder Ala und/oder
X₂₇ für Thr oder Ile

Als konkrete Beispiele für SEQ IQ NO: 26 sind zu nennen:
LYLCLRGCP
LQLCVNGCP
LQLCVGGCP

Als konkrete Beispiele für SEQ IQ NO: 27 sind zu nennen:
TAVAKCKEKLPVEDLYYQACVFDLLTTG
TANTOCHEKMPVKDIYFOSCVFDLLTTG
TARRLCKEGLPVEDAYFHSCVFDVLISG

Als konkrete Beispiele für den Linker Link2 sind zu nennen:
LNOOIDFOAFHTNAEGTGARRLAAASPAPTAPETFPYE
LSERIDDGOGOVSAILGHSLPRTSLVOAWPGYTLE
PSQRLSRSERNRRGAITID

Beispiele erfindungsgemäßer niedrig-affiner Domänen sind BMP-bindende Domänen oder bindende Fragmente davon, welche wenigstens 10 zusammenhängende Aminosäurereste aus dem Sequenzbereich etwa von Position 316 bis etwa 386 gemäß SEQ ID NO: 2, aus dem Sequenzbereich etwa von Position 350 bis etwa 421 gemäß SEQ ID NO: 4 oder aus dem Sequenzbereich etwa von Position 314 bis 369 gemäß SEQ ID NO: 6 umfassen.

Beispiele für oben genannte "bindende Fragmente mit wenigstens 10 Aminosäureresten" sind z.B. solche, die z.B. 10-50, 10-30, 10-25, 10-20 oder 10-15, wie insbesondere 10, 11, 12, 13, 14, oder 15, zusammenhängende Aminosäurereste aus einem der oben genannten Peptide oder Sequenzbereiche aufweisen.

Gegenstand der Erfindung sind insbesondere solche BMP-bindenden Domänen, welche wenigstens ein BMP ausgewählt unter BMP-2, BMP-4, BMP-5, BMP-6 und BMP-12, insbesondere BMP-2 und/oder BMP-4 binden; sowie BMP-bindende Domänen, welche auch an Neogenin binden.

Ein weiterer Gegenstand der Erfindung betrifft antigene Polypeptid-Fragmente der BMP-bindenden Domänen nach obiger Definition. Insbesondere sind solche antigenen Polypeptid-Fragmente Gegenstand der Erfindung, welche zur Herstellung von Immunglobulin-Molekülen brauchbar sind und die Bindung von RGM an ein BMP-Rezeptormolekül modulieren, insbesondere teilweise oder vollständig agonisieren oder antagonisieren und gegebenenfalls die Bindung an den Neogenin-Rezeptor modulieren, insbesondere teilweise oder vollständig antagonisieren. Beispielsweise seien solche antigenen Polypeptid-Fragmente genannt, welche wenigstens 10, wie z.B. 10-30, 10-25, 10-20 oder 10-15, wie insbesondere 10, 11, 12, 13, 14, oder 15, zusammenhängende Aminosäurereste eines der oben genannten Peptide umfassen.

Ein weiterer Gegenstand der Erfindung betrifft die Verwendung einer BMP-bindenden Domäne nach obiger Definition oder eines Polypeptid-Fragments nach obiger Definition zur Herstellung eines polyklonalen Antiserums oder eines monoklonalen Antikörpers gegen RGM, wobei das Antiserum oder der Antikörper insbesondere die Bindung von RGM an den Neogenin-Rezeptor moduliert, vorzugsweise teilweise oder vollständig antagonisiert.

Gegenstand der Erfindung sind auch solche polyklonalen Antiseren oder monoklonale Antikörper gegen RGM gemäß obiger Definition zur Verwendung in der Diagnose oder Therapie.

Ein weiterer Gegenstand der Erfindung betrifft Fusionsproteine umfassend wenigstens ein erstes biologisch aktives Polypeptid, ausgewählt unter BMP-bindenden Domänen nach obiger Definition, operativ (d.h. N- oder C-terminal) verknüpft mit einem zweiten Polypeptid, ausgewählt unter einem mono- oder polyvalenten Trägerpolypeptid oder einem zweiten biologisch aktiven Polypeptid. Insbesondere umfasst der polyvalente Träger wenigstens ein Fc oder Fc" Molekül eines Immunglobulins, insbesondere eines Säugers, wie insbesondere aus einem humanen Immunglobulin abgeleitet, wobei jede der beiden Polypetidketten davon mit gleichen oder verschiedenen BMP-bindenden Domänen nach obiger Definition operativ verknüpft ist.

Ein weiterer Gegenstand der Erfindung betrifft die Verwendung einer BMP-bindenden Domäne nach obiger Definition oder eines Polypeptid-Fragments nach obiger Definition zur Herstellung eines polyklonaler Antiserums oder monoklonalen Antikörpers gegen RGM, wobei das Antiserum oder der Antikörper die Bindung von RGM an Neogenin (d.h. den Neogenin-Rezeptor) moduliert.

Gegenstand der Erfindung ist auch ein polyklonales Antiserum oder ein monoklonaler Antikörper gegen RGM gemäß obiger Definition zur Verwendung in der Diagnose oder Therapie.

Gegenstand der Erfindung ist auch die Verwendung eines polyklonalen Antiserums oder eines monoklonalen Antikörpers nach obiger Definition zur Herstellung eines pharmazeutischen Mittels zur Diagnose oder Therapie von Krankheiten oder Krankheitsstadien welche durch eine Wechselwirkung des Neogenin-Rezeptors (Neogenins) mit RGM oder einem RGM-Fragment vermittelt werden, insbesondere von Krankheiten oder Krankheitsstadien, ausgewählt unter
a) mechanischen Verletzungen von Schädel, Gehirn und Rückenmark,
b) chronische Erkrankungen, ausgewählt unter neurodegenerativen, inflammatorischen oder Autoimmun-Erkrankungen,
c) Störungen der neuronalen Regeneration, des axonalen Sproutings, der Neuritenextension und der neuronalen Plastizität,
d) Tumorerkrankungen und Tumormetastasierung.

Gegenstand der Erfindung ist weiterhin die Verwendung einer BMP-bindenden Domäne nach obiger Definition oder eines Fusionsproteins nach obiger Definition zur Herstellung eines Mittels zur Diagnose oder Therapie von Krankheiten oder Krankheitsstadien welche durch eine gestörte oder beeinträchtigte Wechselwirkung von RGM oder einem RGM-Fragment mit dem zugeordneten Rezeptor (Neogenin oder BMP) mediiert werden, wobei die Krankheiten oder Krankheitsstadien insbesondere ausgewählt sind unter
a) veränderten Neuritogeneseprozessen bei psychotischen Erkrankungen und chronischen Schmerzzuständen die durch exzessive Neuritensprossung und/oder pathologische Synaptogenese hervorgerufen werden.
b) Erkrankungen, assoziiert mit einem gestörten Eisenstoffwechsel;
c) Erkrankungen, assoziiert mit einem gestörten Knochenwachstum
d) Erkrankungen, assoziiert mit degenerativen Knorpelveränderungen
e) Erkrankungen, assoziiert mit Bandscheiben - und Wirbelkörperschäden
f) Erkrankungen, assoziiert mit deregulierten, unkontrollierten Zellmigrationsvorgängen.

Ein weiterer Gegenstand der Erfindung betrifft die Verwendung einer BMP-bindenden Domäne oder eines bindenden Fragments davon nach obiger Definition oder eines Fusionsproteins nach obiger Definition zur Herstellung eines Mittels zur Diagnose oder Therapie von Krankheiten oder Krankheitsstadien welche durch Stimulation oder Verstärkung des BMP-Signalwegs (durch Bindung an BMP-2, BMP-4, BMP-5, BMP-6, und/oder BMP, insbesondere an BMP-2 und /oder 4) behandelbar sind, insbesondere zur Behandlung von Erkrankungen mit gestörtem Knochenwachstum oder zur Behandlung von Knochenfrakturen.

Gegenstand der Erfindung ist außerdem die Verwendung einer BMP-bindenden Domäne oder eines bindenden Fragments davon nach obiger Definition oder eines Fusionsproteins nach obiger Definition zur Herstellung eines Mittels zur Diagnose oder Therapie von Autoimmunerkrankungen, insbesondere ausgewählt unter Spondylitis ankylosans, Antiphospholipid syndrome, Addison Krankheit, autoimmuner hämolytischer Anämie, Autoimmun-Hepatitis, autoimmunes lymphoproliferatives Syndrom (ALPS), autoimmunes thrombocytopenische Purpura (ATP), Behcet Krankheit, bullöses Pemphigoid, Kardiomyopathie, Zöliakie. Dermatitis Herpetiformis, Chronic Fatigue Immune Dysfunction Syndrom (CFIDS), Chronic Inflammatory Demyelinating Polyneuropathie (CIDP vernarbendes Pemphigoid, systemische Sclerose (CREST Syndrom), Kälteagglutinationskrankheit, Crohn's Krankheit, cutane Vasculitis, Degos Krankheit, Dermatomyositis, juvenile Dermatomyositis, Lupus erythematosus discoides, essentielle gemischte Kryoglubulinämie, Fibromyalgie, Goodpasture Syndrom, Graves Krankheit, Guillain-Barre Syndrom, Hashimoto Thyroiditis, idiopathische pulmonäre Fibrose, idiopathische Thrombocytopenia purpura (ITP), Immunoglobulin A Nephropathie, Insulinabhängiger Diabetes mellitus, juvenile Arthritis, Kawasaki Krankheit, Lichen planus, membranöse Glomerulonephritis, Meniere Krankheit, gemischte Bindegewebserkrankung, multifocale motorische Neuropathy, Multiple Sklerose, Myasthenia gravis, Pemphigus vulgaris, perniciöse Anämie, Polyarteritis nodosa, Polychondritis, Polyglanduläres Syndrom, Polymyalgia rheumatica, Polymyositis, Dermatomyositis, primäre Agammaglobulinemia, primäre Cirrhosis biliaris, Psoriasis, Raynaud Phänomen, Reiter Syndrome, rheumatisches Fieber, rheumatoide Arthritis, Sarcoidose, Scleroderma, Sjogren Syndrom, Stiff-Man Syndrom, systemischer Lupus erythematosus, Takayasu Arthritis, temporäre Arthritis/Riesenzellenarthritis, ulcerative Colitis, Uveitis, Vasculitis, Vitiligo, und Wegener Granulomatose; oder
Haarausfallerkrankungen, insbesondere ausgewählt unter Alopecia areata, Alopecia totalis, Alopecia universalis, androgener Alopecie, telogenem Effluvium, anagenem Effluvium, und Chemotherapie-induzierter Alopecie.

Gegenstand der Erfindung ist auch die Verwendung einer BMP-bindenden Domäne nach obiger Definition als Target zum Nachweis oder zur Identifizierung von RGM-bindenden Liganden.

Gegenstand der Erfindung ist weiterhin die Verwendung einer BMP-bindenden Domäne nach obiger Definition oder eines Fragments nach obiger Definition als Immunogen zur aktiven oder passiven Immunisierung.

Gegenstand der Erfindung ist weiterhin ein polyklonales Antiserum, erhältlich durch Immunisierung eines Säugers mit einer antigenen Menge einer BMP-bindenden Domäne nach obiger Definition oder eines Polypeptid-Fragments nach obiger Defintion.

Gegenstand der Erfindung ist auch ein monoklonaler Antikörper gegen eine BMP-bindende Domäne nach obiger Definition oder gegen ein Polypeptid-Fragment nach obiger Definition, oder ein Antigen-bindendes Fragment davon, gegebenenfalls in humanisierter Form.

Gegenstand der Erfindung sind pharmazeutische Mittel, umfassend in einem pharmazeutisch verträglichen Träger wenigstens einen aktiven Bestandteil ausgewählt unter:
a) einer BMP-bindenden Domäne nach obiger Definition oder einem Polypeptid-Fragment nach obiger Definition oder ein Fusionsprotein nach obiger Definition,
b) monoklonale oder polyklonale Antikörpern nach obiger Definition.

Insbesondere sind erfindungsgemäße pharmazeutische Mittel zur intrathecalen, intravenösen, subkutanen, oralen oder parenteralen, perkutanen, subdermalen, intraossealen, nasalen, extrakorporalen und Inhalations-Verabreichung geeignet.

Weitere erfindungsgemäße pharmazeutische Mittel eignen sich zur Behandlung von Knochenfrakturen und umfassen wenigstens eine BMP-bindende Domäne nach obiger Definition oder ein Fusionsprotein nach obiger Definition in einem flüssigen, halbfesten, oder festen Träger.

Ein weiterer Gegenstand der Erfindung betrifft einen Expressionsvektor, umfassend wenigstens eine kodierende Nukleinsäuresequenz für eine BMP-bindende Domäne nach obiger Definition, ein Fusionsprotein nach obiger Definition oder ein Polypeptid-Fragment nach obiger Definition, operativ verknüpft mit wenigstens einer regulativen Nukleinsäuresequenz.

Weiterhin betrifft die Erfindung:
- Rekombinante Mikroorganismen, welche wenigstes einen Vektor nach obiger Definition tragen.
- Hybridomzelllinien, welche einen monoklonalen Antikörper nach obiger Definition produzieren. Dabei wird eine Hybridomzelllinie nach obiger Definition kultiviert und das produzierte Proteinprodukt aus der Kultur isoliert.
- Verfahren zur Herstellung einer BMP-bindenden Domäne nach obiger Definition oder eines Polypeptid-Fragments oder Fusionsproteins nach obiger Definition, wobei man einen rekombinanten Miroorganismus nach obiger Definition kultiviert und das produzierte Proteinprodukt aus der Kultur isoliert.
   Ein weiterer Gegenstand der Erfindung ist ein Verfahren zur Herstellung eines monoklonalen Antikörpers nach obiger Definition, wobei man eine Hybridomzelllinie nach obiger Defintion kultiviert und das produzierte Proteinprodukt aus der Kultur isoliert.
   Gegenstand der Erfindung ist ferner die Verwendung einer BMP-bindenden Domäne nach obiger Definition oder eines Fusionsproteins nach obiger Definition zur Herstellung eines pharmazeutischen Mittels zur Stimulierung eines RGM-Rezeptors, insbesondere von Neogenin oder eines BMP ausgewählt unter BMP-2, BMP-4, BMP-5, BMP-6 und BMP-12.
   Schließlich betrifft die Erfindung die Verwendung eines monoklonalen Antikörpers nach obiger Definition, zur Herstellung eines pharmazeutischen Mittels zur Blockierung der Aktivierung eines RGM-Rezeptors, wie insbesondere Neogenin.

### III. Weitere Angaben zur Ausführung der Erfindung

### 1. Polypeptide

Gegenstand der Erfindung sind insbesondere BMP-Bindungsdomänen von Proteinen der RGM Familie und von diesen Domänen abgeleitete Peptidfragmente. Während erfindungsgemäß insbesondere RGM A und dessen Bindungsdomäne und davon abgeleitete Fragmente untersucht wurden, sind Gegenstand der Erfindung auch entsprechende Domänen und Fragmente homologer Proteine, wie insbesondere homologer Mitglieder der RGM Familie, wie insbesondere RGM B und RGM C.

"Funktionale Äquivalente" oder Analoga der konkret offenbarten RGM-Domänen oder Polypeptide sind im Rahmen der vorliegenden Erfindung davon verschiedene Polypeptide, wie z. B. solche mit einem Homologiegrad von weniger als 100% zu den BMP-Bindungsdomänen von Proteinen gemäß SEQ ID NO: 2, 4 oder 6, welche aber weiterhin die gewünschte biologische Aktivität besitzen. Insbesondere sollten diese zur Bindung an wenigstens ein BMP befähigt sein und/oder in einem hierin beschriebenen Bindungstest eine Bindung zeigen und gegebenenfalls auch in einem hierin beschriebenen Nervenfaserwachstumstest einen inhibitorischen Effekt zeigen und dabei das Nervenfaserwachstum teilweise oder vollständig, statistisch signifikant (p <= 0,05), inhibieren

Unter "funktionalen Äquivalenten" versteht man erfindungsgemäß insbesondere Mutanten, welche in wenigstens einer der Sequenzpositionen der oben genannten konkreten Sequenzen eine andere als die konkret genannte Aminosäure aufweisen aber trotzdem eine der hierin genannten biologischen Aktivitäten besitzen. "Funktionale Äquivalente" umfassen somit die durch eine oder mehrere Aminosäure-Additionen, -Substitutionen, - Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche biologische Effekte nur unterschiedlich stark ausgeprägt zu beobachten sind. Beispiele für geeignete Substitutionen von Aminosäureresten sind folgende:

| **Ursprünglicher Rest** | **Beispiele der Substitution** |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn; Gln |
| Ile | Leu; Val |
| Leu | Ile; Val |
| Lys | Arg ; Gln ; Glu |
| Met | Leu ; Ile |
| Phe | Met ; Leu ; Tyr |
| Ser | Thr |
| Thr | Ser |
| Trp | Tyr |
| Tyr | Trp ; Phe |
| Val | Ile; Leu |

"Funktionale Äquivalente" im obigen Sinne sind auch Präkursoren der beschriebenen Polypeptide sowie funktionale Derivate und Salze der Polypeptide. Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Erfindung. "Funktionale Derivate" erfindungsgemäßer Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

"Funktionale Äquivalente" umfassen natürlich auch Polypeptide welche aus anderen Organismen, zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" sind außerdem Fusionsproteine, welche ein der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Enzyme und Immunoglobuline.

Erfindungsgemäß mit umfasste "funktionale Äquivalente" sind Homologe zu den konkret offenbarten Proteinen bzw. Peptiden. Diese besitzen wenigstens 40 % oder wenigstens 50%, oder wenigstens 60 %, wie z.B. wenigstens 75% oder insbesondere wenigstens 85 %, wie z.B. 90%, 95% oder 99%, Homologie zu einer der konkret offenbarten Sequenzen, z.B. berechnet nach dem Algorithmus von Pearson und Lipman, Proc. Natl. Acad, Sci. (USA) 85(8), 1988, 2444-2448. Eine prozentuale Homologie eines erfindungsgemäßen homologen Polypeptids bedeutet insbesondere prozentuale Identität der Aminosäurereste bezogen auf die Gesamtlänge einer der hierin konkret beschriebenen Aminosäuresequenzen.

Unter einer "abgeleiteten" Aminosäuresequenz wird erfindungsgemäß, wenn keine anderen Angaben gemacht werden, eine Sequenz verstanden, die mit der Ausgangssequenz eine Identität von mindestens 80% oder mindestens 90%, insbesondere 91 %, 92%, 93%, 94%, 95%, 96%, 97%, 98% und 99% aufweist.

Unter "Identität" bzw. "Homologie" zwischen zwei Sequenzen wird Identität der Aminosäurereste über die jeweils gesamte Sequenzlänge verstanden, wie z.B. die Identität, die durch Vergleich mit Hilfe der Vector NTI Suite 7.1 Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:
Multiple alignment parameter:

| | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighing | 0 |

| | |
|---|---|
| Pairwise alignment parameter: | |
| FAST algorithm | on |
| K-tuple size | 1 |
| Gap penalty | 3 |
| Window size | 5 |
| Number of best diagonals | 5 |

Im Falle einer möglichen Proteinglykosylierung umfassen die erfindungsgemäßen Äquivalente Proteine des oben bezeichneten Typs in deglykosylierter bzw. glykosylierter Form sowie durch Veränderung des Glykosylierungsmusters erhältliche abgewandelte Formen.

Homologe der erfindungsgemäßen Peptide können durch Screening kombinatorischer Banken von Mutanten, wie z.B. Verkürzungsmutanten, identifiziert werden. Beispielsweise kann eine variegierte Bank von Peptid-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen codieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

### 2. Nukleinsäuren

Gegenstand der Erfindung sind weiterhin die kodierenden Nukleinsäuresequenzen für die oben beschriebenen BMP-Bindungsdomänen und -Polypetide, wie insbesondere gemäß SEQ ID NO: 1, 3 und 5, sowie davon abgeleitete Nukleinsäuresequenzen oder -teilsequenzen, die für die oben beschriebenen Peptidfragmente kodieren.

Alle erfindungsgemäßen Nukleinsäuresequenzen (einzel- und doppelsträngige DNA-und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Unter einer "abgeleiteten" Nukleinsäuresequenz wird erfindungsgemäß, wenn keine anderen Angaben gemacht werden, eine Sequenz verstanden, die mit der Ausgangssequenz eine Identität von mindestens 80% oder mindestens 90%, insbesondere 91 %, 92%, 93%, 94%, 95%, 96%, 97%, 98% und 99% aufweist.

Unter "Identität" zwischen zwei Nukleinsäuren wird die Identität der Nukleotide über die jeweils gesamte Nukleinsäurelänge verstanden, insbesondere die Identität, die durch Vergleich mit Hilfe der Vector NTI Suite 7.1 Software der Firma Informax (USA) unter Anwendung der Clustal Methode (siehe oben).

Gegenstand der Erfindung sind auch Nukleinsäuresequenzen, kodierend für eines der obigen Peptide und deren funktionalen Äquivalenten, welche z.B. unter Verwendung künstlicher Nukleotidanaloga zugänglich sind.

Die Erfindung betrifft sowohl isolierte Nukleinsäuremoleküle, welche für erfindungsgemäße Peptide oder biologisch aktive Abschnitte davon kodieren, sowie Nukleinsäurefragmente, die z.B. als Hybridisierungssonden oder Primer zur Identifizierung oder Amplifizierung von erfindungsgemäßer kodierenden Nukleinsäuren verwendet werden können.

Die erfindungsgemäßen Nukleinsäuremoleküle können zudem untranslatierte Sequenzen vom 3'- und/oder 5'-Ende des kodierenden Genbereichs enthalten

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül kann mittels molekularbiologischer Standard-Techniken und der erfindungsgemäß bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der erfindungsgemäßen Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die erfindungsgemäßen Oligonukleotide können ferner durch Standard-Syntheseverfahren, z.B. mit einem automatischen DNA-Synthesegerät, hergestellt werden.

Die Erfindung umfasst weiterhin die zu den konkret beschriebenen Nukleotidsequenzen komplementären Nukleinsäuremoleküle oder einen Abschnitt davon.

Die erfindungsgemäßen Nukleotidsequenzen ermöglichen die Erzeugung von Sonden und Primern, die zur Identifizierung und/oder Klonierung von homologen Sequenzen in anderen Zelltypen und Organismen verwendbar sind. Solche Sonden bzw. Primer umfassen gewöhnlich einen Nukleotidsequenzbereich, der unter stringenten Bedingungen an mindestens etwa 12, vorzugsweise mindestens etwa 25, wie z.B. etwa 40, 50 oder 75 aufeinanderfolgende Nukleotide eines Sense-Stranges einer erfindungsgemäßen Nukleinsäuresequenz oder eines entsprechenden Antisense-Stranges hybridisiert.

Weitere erfindungsgemäße Nukleinsäuresequenzen sind abgeleitet von kodierenden Sequenzen zu den erfindungsgemäßen RGM-Domänen und -Peptiden und unterscheiden sich davon durch Addition, Substitution, Insertion oder Deletion einzelner oder mehrerer Nukleotide, kodieren aber weiterhin für Peptide mit dem gewünschten Eigenschaftsprofil.

Erfindungsgemäß umfasst sind auch solche Nukleinsäuresequenzen, die sogenannte stumme Mutationen umfassen oder entsprechend der Codon-Nutzung eines speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon. Gegenstand sind ebenso durch konservative Nukleotid-Substitutionen (d.h. die betreffende Aminosäure wird durch eine Aminosäure gleicher Ladung, Größe, Polarität und/oder Löslichkeit ersetzt) erhältliche Sequenzen.

Gegenstand der Erfindung ist auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Weiterhin umfasst die Erfindung auch Nukleinsäuresequenzen, welche mit oben genannten kodierenden Sequenzen hybridisieren oder dazu komplementär sind. Diese Polynukleotide lassen sich bei Durchmusterung von genomischen oder cDNA-Banken auffinden und gegebenenfalls daraus mit geeigneten Primern mittels PCR vermehren und anschließend beispielsweise mit geeigneten Sonden isolieren. Eine weitere Möglichkeit bietet die Transformation geeigneter Mikroorganismen mit erfindungsgemäßen Polynukleotiden oder Vektoren, die Vermehrung der Mikroorganismen und damit der Polynukleotide und deren anschließende Isolierung. Darüber hinaus können erfindungsgemäße Polynukleotide auch auf chemischem Wege synthetisiert werden.

Unter der Eigenschaft, an Polynukleotide "hybridisieren" zu können, versteht man die Fähigkeit eines Poly- oder Oligonukleotids unter stringenten Bedingungen an eine nahezu komplementäre Sequenz zu binden, während unter diesen Bedingungen unspezifische Bindungen zwischen nicht-komplementären Partnern unterbleiben. Dazu sollten die Sequenzen zu 70-100%, insbesondere zu 90-100%, wie z.B. 95%, 96%, 97%, 98% oder 99%, komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze. Üblicherweise werden dazu Oligonukleotide ab einer Länge von 30 Basenpaaren eingesetzt. Unter stringenten Bedingungen versteht man beispielsweise in der Northern-Blot-Technik die Verwendung einer 50 - 70 °C, vorzugsweise 60 - 65 °C warmen Waschlösung, beispielsweise 0,1x SSC-Puffer mit 0,1% SDS (20x SSC: 3M NaCl, 0,3M Na-Citrat, pH 7,0) zur Elution unspezifisch hybridisierter cDNA-Sonden oder Oligonukleotide. Dabei bleiben, wie oben erwähnt, nur in hohem Maße komplementäre Nukleinsäuren aneinander gebunden. Die Einstellung stringenter Bedingungen ist dem Fachmann bekannt und ist z.B. in Ausubel et al., Current Protocols in Molecular Biology, John Wiley & Sons, N.Y. (1989), 6.3.1-6.3.6. beschrieben.

Ein weiterer Aspekt der Erfindung betrifft "Antisense-"Nukleinsäuren. Diese umfasst eine Nukleotidsequenz, die zu einer kodierenden "Sense-"Nukleinsäure, komplementär ist. Die Antisense-Nukleinsäure kann zum gesamten kodierenden Strang oder nur zu einem Abschnitt davon komplementär sein. Bei einer weiteren Ausführungsform ist das Antisense-Nukleinsäuremolekül antisense zu einem nicht-kodierenden Bereich des kodierenden Stranges einer Nukleotidsequenz. Der Begriff "nicht-kodierender Bereich" betrifft die als 5'- und 3'-untranslatierte Bereiche bezeichneten Sequenzabschnitte.

Ein Antisense-Oligonukleotid kann z.B. etwa 5, 10, 15, 20, 25, 30, 35, 40, 45 oder 50 Nukleotide lang sein. Eine erfindungsgemäße Antisense-Nukleinsäure kann durch chemische Synthese und enzymatische Ligationsreaktionen mittels im Fachgebiet bekannter Verfahren konstruiert werden. Eine Antisense-Nukleinsäure kann chemisch synthetisiert werden, wobei natürlich vorkommende Nukleotide oder verschieden modifizierte Nukleotide verwendet werden, die so gestaltet sind, dass sie die biologische Stabilität der Moleküle erhöhen, oder die physikalische Stabilität des Duplexes erhöhen, der zwischen der Antisense- und Sense-Nukleinsäure entstanden ist. Beispielsweise können Phosphorthioat-Derivate und acridinsubstituierte Nukleotide verwendet werden. Beispiele modifizierter Nukleotide, die zur Erzeugung der Antisense-Nukleinsäure verwendet werden können, sind u.a. 5-Fluoruracil, 5-Bromuracil, 5-Chloruracil, 5-loduracil, Hypoxanthin, Xanthin, 4-Acetylcytosin, 5-(Carboxyhydroxylmethyl)uracil, 5-Carboxymethylaminomethyl-2-thiouridin, 5-Carboxymethylaminomethyluracil, Dihydrouracil, Beta-D-Galactosylqueosin, Inosin, N6-Isopentenyladenin, 1-Methylguanin, 1-Methylinosin, 2,2-Dimethylguanin, 2-Methyladenin, 2-Methylguanin, 3-Methylcytosin, 5-Methylcytosin, N6-Adenin, 7-Methylguanin, 5-Methylaminomethyluracil, 5-Methoxyaminomethyl-2-thiouracil, Beta-D-Mannosylqueosin, 5'-Methoxycarboxymethyluracil, 5-Methoxyuracil, 2-Methylthio-N6-isopentenyladenin, Uracil-5-oxyessigsäure (v), Wybutoxosin, Pseudouracil, Queosin, 2-Thiocytosin, 5-Methyl-2-thiouracil, 2-Thiouracil, 4-Thiouracil, 5-Methyluracil, Uracil-5-oxyessigsäuremethylester, Uracil-5-oxyessigsäure (v), 5-Methyl-2-thiouracil, 3-(3-Amino-3-N-2-carboxypropyl)uracil, (acp3)w und 2,6-Diaminopurin. Die Antisense-Nukleinsäure kann auch biologisch hergestellt werden, indem ein Expressionsvektor verwendet wird, in den eine Nukleinsäure in Antisense-Richtung subkloniert worden ist.

### 3. Expressionskonstrukte und Vektoren

Gegenstand der Erfindung sind außerdem Expressionskonstrukte, enthaltend unter der genetischen Kontrolle regulativer Nukleinsäuresequenzen eine für ein erfindungsgemäßes RGM-Peptid oder funktionales Äquivalent oder Immunglobulin kodierende Nukleinsäuresequenz; sowie Vektoren, umfassend wenigstens eines dieser Expressionskonstrukte.

Vorzugsweise umfassen solche erfindungsgemäßen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz. Unter einer "operativen Verknüpfung" versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind Targeting-Sequenzen sowie Enhancer, Polyadenylierungssignale und dergleichen. Weitere regulative Elemente umfassen selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Zusätzlich zu den artifiziellen Regulationssequenzen kann die natürliche Regulationssequenz vor dem eigentlichen Strukturgen noch vorhanden sein. Durch genetische Veränderung kann diese natürliche Regulation gegebenenfalls ausgeschaltet und die Expression der Gene erhöht oder erniedrigt werden. Das Genkonstrukt kann aber auch einfacher aufgebaut sein, das heißt es werden keine zusätzlichen Regulationssignale vor das Strukturgen insertiert und der natürliche Promotor mit seiner Regulation wird nicht entfernt. Statt dessen wird die natürliche Regulationssequenz so mutiert, dass keine Regulation mehr erfolgt und die Genexpression gesteigert oder verringert wird. Die Nukleinsäuresequenzen können in einer oder mehreren Kopien im Genkonstrukt enthalten sein.

Beispiele für brauchbare Promotoren sind: cos-, tac-, trp-, tet-, trp-tet-, lpp-, lac-, Ipplac-, laclq-, T7-, T5-, T3-, gal-, trc-, ara-, SP6-, lambda-PR- oder lambda-PL-Promotor, die vorteilhafterweise in gram-negativen Bakterien Anwendung finden; sowie die grampositiven Promotoren amy und SPO2, die Hefepromotoren ADC1, MFalpha, AC, P-60, CYC1, GAPDH oder die Pflanzenpromotoren CaMV/35S, SSU, OCS, lib4, usp, STLS1, B33, not oder der Ubiquitin- oder Phaseolin-Promotor. Besonders bevorzugt ist die Verwendung induzierbarer Promotoren, wie z.B. licht- und insbesondere temperaturinduzierbarer Promotoren, wie der PᵣPₗ-Promotor. Prinzipiell können alle natürlichen Promotoren mit ihren Regulationssequenzen verwendet werden. Darüber hinaus können auch synthetische Promotoren vorteilhaft verwendet werden.

Die genannten regulatorischen Sequenzen sollen die gezielte Expression der Nukleinsäuresequenzen und der Proteinexpression ermöglichen. Dies kann beispielsweise je nach Wirtsorganismus bedeuten, dass das Gen erst nach Induktion exprimiert oder überexprimiert wird, oder dass es sofort exprimiert und/oder überexprimiert wird.

Die regulatorischen Sequenzen bzw. Faktoren können dabei vorzugsweise die Expression positiv beeinflussen und dadurch erhöhen oder erniedrigen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Die Herstellung einer Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden. Unter Vektoren sind außer Plasmiden auch alle anderen dem Fachmann bekannten Vektoren, wie beispielsweise Phagen, Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden.

Als Beispiele für geeignete Expressionsvektoren können genannt werden:

Übliche Fusionsexpressionsvektoren, wie pGEX (Pharmacia Biotech Inc; Smith, D.B. und Johnson, K.S. (1988) Gene 67:31-40), pMAL (New England Biolabs, Beverly, MA) und pRIT 5 (Pharmacia, Piscataway, NJ), bei denen Glutathion-S-Transferase (GST), Maltose E-bindendes Protein bzw. Protein A an das rekombinante Zielprotein fusioniert wird.

Nicht-Fusionsprotein-Expressionsvektoren wie pTrc (Amann et al., (1988) Gene 69:301-315) und pET 11d (Studier et al. Gene Expression Technology: Methods in Enzymology 185, Academic Press, San Diego, Kalifornien (1990) 60-89).

Hefe-Expressionsvektor zur Expression in der Hefe S. *cerevisiae*, wie pYepSec1 (Baldari et al., (1987) Embo J. 6:229-234), pMFa (Kurjan und Herskowitz (1982) Cell 30:933-943), pJRY88 (Schultz et al. (1987) Gene 54:113-123) sowie pYES2 (Invitrogen Corporation, San Diego, CA). Vektoren und Verfahren zur Konstruktion von Vektoren, die sich zur Verwendung in anderen Pilzen, wie filamentösen Pilzen, eignen, umfassen diejenigen, die eingehend beschrieben sind in: van den Hondel, C.A.M.J.J. & Punt, P.J. (1991) "Gene transfer systems and vector development for filamentous fungi, in: Applied Molecular Genetics of Fungi, J.F. Peberdy et al., Hrsg., S. 1-28, Cambridge University Press: Cambridge.

Baculovirus-Vektoren, die zur Expression von Proteinen in gezüchteten Insektenzellen (bspw. Sf9-Zellen) verfügbar sind, umfassen die pAc-Reihe (Smith et al., (1983) Mol. Cell Biol.. 3:2156-2165) und die pVL-Reihe (Lucklow und Summers (1989) Virology 170:31-39).

Pflanzen-Expressionsvektoren, wie solche, die eingehend beschrieben sind in: Becker, D., Kemper, E., Schell, J. und Masterson, R. (1992) "New plant binary vectors with selectable markers located proximal to the left border", Plant Mol. Biol. 20:1195-1197; und Bevan, M.W. (1984) "Binary Agrobacterium vectors for plant transformation", Nucl. Acids Res. 12:8711-8721.

Säugetier-Expressionsvektoren, wie pCDM8 (Seed, B. (1987) Nature 329:840) und pMT2PC (Kaufman et al. (1987) EMBO J. 6:187-195).

Weitere geeignete Expressionssysteme für prokaryontische und eukaryotische Zellen sind in Kapitel 16 und 17 von Sambrook, J., Fritsch, E.F. und Maniatis, T., Molecular cloning: A Laboratory Manual, 2. Auflage, Cold Spring Harbor Laboratory, Cold Spring Harbor Laboratory Press, Cold Spring Harbor, NY, 1989 beschrieben.

### 4. Rekombinante Wirtsorganismen

Mit Hilfe der erfindungsgemäßen Vektoren sind rekombinante Organismen herstellbar, welche beispielsweise mit wenigstens einem erfindungsgemäßen Vektor transformiert sind und zur Produktion der erfindungsgemäßen Domänen oder Polypeptide eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungsund Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben.

Als Wirtsorganismen sind prinzipiell alle Organismen geeignet, die eine Expression der erfindungsgemäßen Nukleinsäuren, ihrer Allelvarianten, ihrer funktionellen Äquivalente oder Derivate ermöglichen. Unter Wirtsorganismen sind beispielsweise Bakterien, Pilze, Hefen, pflanzliche oder tierische Zellen zu verstehen. Bevorzugte Organismen sind Bakterien, wie solche der Gattungen Escherichia, wie z. B. Escherichia coli, Streptomyces, Bacillus oder Pseudomonas, eukaryotische Mikroorganismen, wie Saccharomyces cerevisiae, Aspergillus, höhere eukaryotische Zellen aus Tieren oder Pflanzen, beispielsweise Sf9-, CHO- oder HEK293-Zellen.

Die Selektion erfolgreich transformierter Organismen kann durch Markergene erfolgen, die ebenfalls im Vektor oder in der Expressionskassette enthalten sind. Beispiele für solche Markergene sind Gene für Antibiotikaresistenz und für Enzyme, die eine farbgebende Reaktion katalysieren, die ein Anfärben der transformierten Zelle bewirkt. Diese können dann mittels automatischer Zellsortierung selektiert werden. Erfolgreich mit einem Vektor transformierte Mikroorganismen, die ein entsprechendes Antibiotikaresistenzgen (z.B. G418 oder Hygromycin) tragen, lassen sich durch entsprechende Antibiotika-enthaltende Medien oder Nährböden selektieren. Markerproteine, die an der Zelloberfläche präsentiert werden, können zur Selektion mittels Affinitätschromatographie genutzt werden.

Gewünschtenfalls kann das Genprodukt auch in transgenen Organismen, wie transgenen Tieren, insbesondere Mäusen, Schafen oder transgenen Pflanzen zur Expression, gebracht werden.

Gegenstand der Erfindung sind weiterhin Verfahren zur rekombinanten Herstellung erfindungsgemäßer RGM-Domänen oder -Polypeptide oder funktioneller, biologisch aktiver Fragmente davon, wobei man einen Peptide-produzierenden rekombinanten Wirtsorganismus kultiviert, gegebenenfalls die Expression der Polypeptide induziert und diese aus der Kultur isoliert. Die Peptide können so auch in großtechnischem Maßstab produziert werden, falls dies erwünscht ist.

Der rekombinante Wirt kann nach bekannten Verfahren kultiviert und fermentiert werden. Bakterien können beispielsweise in TB- oder LB-Medium und bei einer Temperatur von 20 bis 40°C und einem pH-Wert von 6 bis 9 vermehrt werden. Im Einzelnen werden geeignete Kultivierungsbedingungen beispielsweise in T. Maniatis, E.F. Fritsch and J. Sambrook, Molecular Cloning: A Laboratory Manual, Cold Spring Harbor Laboratory, Cold Spring Harbor, NY (1989) beschrieben.

Die Zellen werden dann, falls die Polypeptide nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Peptide kann mit bekannten, chromatographischen Verfahren erzielt werden, wie Molekularsieb-Chromatographie (Gelfiltration), wie Q-Sepharose-Chromatographie, lonenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Besonders geeignet ist es, zur Isolierung des rekombinanten Peptids Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Peptide an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Peptide verwendet werden. Zur Erkennung der Peptidekönnen außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Peptide verwendet werden.

### 5. Immunglobuline

### 5.1 Definition

Gegenstand der vorliegenden Erfindung sind monoklonale oder polyklonale Antikörper, die an ein erfindungsgemäßes RGM-Protein oder Derivat/Äquivalent davon spezifisch binden, d.h. Antikörper mit Spezifität für ein erfindungsgemäßes RGM-Protein oder Derivat/Äquivalent davon. Gegenstand der vorliegenden Erfindung sind auch Teile dieser Antikörper, insbesondere antigenbindende Teile davon, d.h. Antikörperfragmente, die ein erfindungsgemäßes RGM-Protein oder ein Derivat/Äquivalent davon binden.

Vorzugsweise wählt man den erfindungsgemäßen Antikörper so, dass er eine bestimmte Bindungskinetik aufweist (z. B. hohe Affinität, geringe Dissoziation, niedrige off-Geschwindigkeit (k_{off}), starke neutralisierende Aktivität) für die spezifische Bindung an erfindungsgemäßes RGM-Protein oder Derivat/Äquivalent davon.

So können Antikörper mit einer Affinität für das erfindungsgemäße RGM-Protein oder Derivat/Äquivalent davon im Bereich von K_{D}=10⁻⁶-10⁻¹² M bereitgestellt werden.

Einem weiteren Aspekt zufolge kann man die erfindungsgemäßen Antikörper so wählen, dass sie das RGM-Protein oder Derivat/Äquivalent davon mit einer k_{off}-Geschwindigkeitskonstanten von 0,1 S⁻¹ oder weniger binden.

Bei den Antikörpern handelt es sich vorzugsweise um isolierte Antikörper. Einem weiteren Aspekt zufolge sind die Antikörper neutralisierende Antikörper. Zu den erfindungsgemäßen Antikörpern gehören insbesondere monoklonale und rekombinante Antikörper. Der erfindungsgemäße Antikörper kann eine Aminosäuresequenz umfassen, die vollständig von einer einzigen Spezies abstammt, kann also z.B. ein humaner Antikörper ein Rattenantikörper oder ein Mausantikörper sein. Weiteren Ausführungsformen zufolge kann der Antikörper ein chimärer Antikörper oder ein CDR-Graft-Antikörper oder eine andere Form eines humanisierten Antikörpers sein.

Der Begriff "Antikörper" soll sich auf Immunglobulinmoleküle beziehen, die aus 4 Polypeptidketten, zwei schweren (H) Ketten und zwei leichten (L) Ketten, gebildet sind. Die Ketten sind in der Regel durch Disulfid-Bindungen miteinander verknüpft. Jede schwere Kette setzt sich aus einer variablen Region der schweren Kette (hier als HCVR oder VH abgekürzt) und einer konstanten Region der schweren Kette zusammen. Die konstante Region der schweren Kette wird aus drei Domänen CH1, CH2 und CH3 gebildet. Jede leichte Kette setzt sich aus einer variablen Region der leichten Kette (hier als LCVR oder VL abgekürzt) und einer konstanten Region der leichten Kette zusammen. Die konstante Region der leichten Kette wird aus einer Domäne CL gebildet. Die VH-und VL-Regionen können weiter unterteilt werden in hypervariable Regionen, die als komplementaritätsbestimmende Regionen (CDR für Complementarity Determining Region) bezeichnet werden und mit konservierteren Regionen, die als Gerüstregionen (FR für Frame Work Region) bezeichnet werden, durchsetzt sind. Jede VH- und VL-Region wird aus drei CDRs und vier FRs gebildet, die vom N-Terminus zum C-Terminus in folgender Reihenfolge angeordnet sind: FR1, CDR1, FR2, CDR2, FR3, CDR3, FR4.

Der Begriff "antigenbindender Teil" eines Antikörpers (oder einfach "Antikörperteil") bezieht sich auf ein oder mehrere Fragmente eines Antikörpers mit Spezifität für ein erfindungsgemäßes RGM-Protein oder Derivat/Äquivalent davon, wobei das oder die Fragmente nach wie vor die Fähigkeit besitzen, das RGM-Protein oder Derivat/Äquivalent davon spezifisch zu binden. Es ist gezeigt worden, dass die antigenbindende Funktion eines Antikörpers von Fragmenten eines vollständigen Antikörpers wahrgenommen werden können. Zu Beispielen bindender Fragmente gehören im Sinne des Begriffs "antigenbindender Teil" eines Antikörpers (i) ein Fab-Fragment, d.h. ein aus den VL-, VH-, CL- und CH1-Domänen zusammengesetztes monovalentes Fragment; (ii) ein F(ab')₂-Fragment, d.h. ein bivalentes Fragment, welches zwei in der Hinge-Region über eine Disulfid-Brücke miteinander verknüpfte Fab-Fragmente beinhaltet; (iii) ein Fd-Fragment, das sich aus den VH- und CH1-Domänen zusammengesetzt; (iv) ein Fv-Fragment, das sich aus den VL- und VH-Domänen eines einzelnen Arms eines Antikörpers zusammengesetzt; (v) ein dAb-Fragment (Ward et al., (1989) Nature 341:544-546 ), das aus einer VH-Domäne oder VH, CH1, CH2, DH3, oder VH, CH2, CH3 besteht; und (vi) eine isolierte komplementaritätsbestimmente Region (CDR).

Wenngleich die beiden Domänen des Fv-Fragments, nämlich VL und VH, von getrennten Genen kodiert sind, können sie des weiteren mit einem synthetischen Linker unter Verwendung rekombinanter Verfahren miteinander verbunden werden, wodurch sie als eine einzige Proteinkette hergestellt werden können, worin die VL- und VH-Regionen sich zusammenfinden, um monovalente Moleküle zu bilden (als Einzelkette-Fv (ScFv) gekannt; siehe z.B. Bird et al. (1988) Science 242:423-426; und Huston et al. (1988) Proc. Natl. Acad. Sci. USA 85:5879-5883). Derartige Einzelketten-Antikörper sollen auch von dem Begriff "antigenbindender Teil" eines Antikörpers erfasst werden. Andere Formen von Einzelketten-Antikörpern wie "Diabodies" gehören ebenfalls dazu. Diabodies sind bivalente, bispezifische Antikörper, in denen VH- und VL-Domänen auf einer einzelnen Polypeptidkette exprimiert sind, wobei allerdings ein Linker verwendet wird, der zu kurz ist, als dass sich die beiden Domänen auf derselben Kette zusammenfinden können, wodurch man die Domänen zwingt, mit komplementären Domänen einer anderen Kette zu paaren und zwei antigenbindende Stellen zu bilden (siehe z.B. Holliger, P., et al. (1993) Proc. Natl. Acad. Sci. USA 90:6444-6448; Poljak, R.J., et al. (1994) Structure 2:1121-1123).

Des weiteren kann ein Antikörper oder antigenbindender Teil davon Teil eines größeren Immunadhäsionsmoleküls sein, das durch kovalente oder nicht kovalente Assoziierung des Antikörpers oder Antikörperteils mit einem oder mehreren weiteren Proteinen oder Peptiden gebildet wird. Zu solchen Immunadhäsionsmolekülen gehört die Verwendung der Streptavidin-Kernregion, um ein tetrameres scFv-Molekül herzustellen (Kipriyanov, S.M., et al. (1995) Human Antibodies und Hybridomas 6:93-101) und die Verwendung eines Cysteinrestes, eines Markerpeptids und eines C-terminalen Polyhistidin-Tags, um bivalente und biotinylierte scFv-Moleküle zu machen (Kipriyanov, S.M., et al. (1994) Mol. Immunol. 31:1047-1058).

Antikörperteile, wie Fab und F(ab')₂-Fragmente, können aus ganzen Antikörpern hergestellt werden, indem man konventionelle Techniken verwendet, wie die Verdauung mit Papain bzw. Pepsin. Darüber hinaus können Antikörper, Antikörperteile und Immunadhäsionsmoleküle erhalten werden, indem man standardisierte rekombinante DNA-Techniken verwendet. Ein "isolierter Antikörper mit Spezifität für ein erfindungsgemäßes RGM-Protein oder Derivat/Äquivalent davon" umschreibt einen Antikörper mit Spezifität für ein erfindungsgemäßes RGM-Protein oder Derivat/Äquivalent davon, der im wesentlichen frei von anderen Antikörpern mit unterschiedlichen Antigenspezifitäten ist.

Der Begriff "neutralisierender Antikörper" beschreibt einen Antikörper, dessen Bindung an ein bestimmtes Antigen zur Inhibierung der biologischen Aktivität des Antigens führt. Diese Inhibierung der biologischen Aktivität des Antigens kann beurteilt werden, indem man einen oder mehrere Indikatoren für die biologische Aktivität des Antigens misst, wobei man einen geeigneten in vitro- oder in vivo-Assay verwendet.

Der Begriff "monoklonaler Antikörper" beschreibt einen Antikörper, der von einem Hybridom abstammt (z. B. ein Antikörper, der von einem mittels Hybridomtechnologie, wie der standardisierten Hybridommethodik nach Köhler und Milstein, hergestellten Hybridom sekretiert wird). Ein von einem Hybridom abstammender Antikörper mit Spezifität für ein erfindungsgemäßes RGM-Protein oder Derivat/Äquivalent davon wird daher als monoklonaler Antikörper bezeichnet.

Der Begriff "rekombinanter Antikörper" beschreibt Antikörper, die mit rekombinanten Mitteln hergestellt, exprimiert, erzeugt oder isoliert werden, wie Antikörper, die unter Verwendung eines in eine Wirtszelle transfizierten, rekombinanten Expressionsvektors exprimiert werden; Antikörper, die aus einer rekombinanten kombinatorischen Antikörperbank isoliert sind; Antikörper, die aus einem Tier (z. B. einer Maus), das durch humane Immunglobulingene transgen ist, isoliert sind (siehe z. B. Taylor, L.D., et al. (1992) Nucl. Acids Res. 20:6287-6295); oder Antikörper, die auf irgend eine andere Weise, bei der bestimmte Immunoglobulin-Gensequenzen (wie humane Immunglobulin-Gensequenzen) mit anderen DNA-Sequenzen zusammengesetzt werden, hergestellt, exprimiert, erzeugt oder isoliert werden. Zu rekombinanten Antikörpern gehören beispielsweise chimäre, CDR-Graft- und humanisierte Antikörper.

Der Begriff "humaner Antikörper" beschreibt Antikörper, deren variable und konstante Regionen Immunglobulinsequenzen der Humankeimbahn, wie beispielsweise von Kabat *et al*. (siehe Kabat, et al. (1991) Sequences of Proteins of Immunological Interest, Fifth Edition, U.S. Department of Health und Human Services, NIH Publication Nr. 91-3242) beschrieben, entsprechen oder davon abstammen. Die erfindungsgemäßen humanen Antikörper können allerdings Aminosäurereste beinhalten, die nicht von humanen Keimbahn-Immunglobulinsequenzen kodiert sind (z. B. Mutationen, die durch zufällige oder ortsspezifische Mutagenese in vitro oder durch somatische Mutation in vivo eingeführt sind), beispielsweise in den CDRs, und insbesondere in CDR3. Erfindungsgemäße rekombinante humane Antikörper haben variable Regionen und können auch konstante Regionen beinhalten, die von Immunglobulinsequenzen der Humankeimbahn abstammen (siehe Kabat, E.A., et al. (1991) Sequences of Proteins of Immunological Interest, Fifth Edition, U.S. Department of Health und Human Services, NIH Publication Nr. 91-3242). Bestimmten Ausführungsformen zufolge werden derartige rekombinante humane Antikörper allerdings einer in vitro-Mutagenese (oder falls ein durch humane Ig-Sequenzen transgenes Tier verwendet wird, einer somatischen in vivo-Mutagenese) unterzogen, so dass die Aminosäuresequenzen der VH- und VL-Regionen der rekombinanten Antikörper Sequenzen sind, die, wenngleich sie mit VH- und VL-Sequenzen der Humankeimbahn verwandt sind oder davon abstammen, innerhalb des humanen Antikörper-Keimbahnrepertoirs in vivo natürlicherweise nicht existieren. Bestimmten Ausführungsformen zufolge sind derartige rekombinante Antikörper das Resultat einer selektiven Mutagenese oder Rückmutation, oder beides.

Der Begriff "Rückmutation" bezieht sich auf ein Verfahren, bei dem einige oder sämtliche der somatisch mutierten Aminosäuren eines humanen Antikörpers mit den entsprechenden Keimbahnresten einer homologen Keimbahnantikörpersequenz ersetzt werden. Die Sequenzen für die schwere und leichte Kette eines erfindungsgemäßen humanen Antikörpers werden getrennt mit den Keimbahnsequenzen in der VBASE-Datenbank verglichen, um die Sequenzen mit der größten Homologie zu identifizieren. Abweichungen im erfindungsgemäßen humanen Antikörper werden auf die Keimbahnsequenz zurückgeführt, indem man an definierten Nukleotidpositionen, die solche abweichenden Aminosäuren kodieren, mutiert. Die direkte oder indirekte Bedeutung jeder auf diese Weise als Kandidat für eine Rückmutation identifizierten Aminosäure für die Antigenbindung sollte untersucht werden, und eine Aminosäure, die nach Mutation eine wünschenswerte Eigenschaft des humanen Antikörpers beeinträchtigt, sollte in den endgültigen humanen Antikörper nicht miteinbezogen werden. Um die Anzahl der Aminosäuren für eine Rückmutation so gering wie möglich zu halten, können diejenigen Aminosäurepositionen unverändert bleiben, die zwar von der am nächsten kommenden Keimbahnsequenz abweichen aber mit der entsprechenden Aminosäuresequenz einer zweiten Keimbahnsequenz identisch sind, vorausgesetzt, daß die zweite Keimbahnsequenz mit der Sequenz des erfindungsgemäßen humanen Antikörpers zumindest in 10 und vorzugsweise in 12 Aminosäuren auf beiden Seiten der fraglichen Aminosäure identisch und co-linear ist. Rückmutationen können auf beliebiger Stufe der Antikörperoptimierung vorgenommen werden.

Der Begriff "chimärer Antikörper" umfasst Antikörper, in denen einzelne Teile des Moleküls aus verschiedenen Spezies abgeleitet sind. So sind chimäre Antikörper, ohne darauf beschränkt zu sein, z.B. Antikörper, die Sequenzen für die variable Region der schweren und leichten Kette aus einer Spezies beinhalten, in denen aber die Sequenzen einer oder mehrerer der CDR-Regionen aus VH und/oder VL mit CDR-Sequenzen einer anderen Spezies ersetzt sind. In solchen Antikörpern können die variablen Regionen der schweren und leichten Kette aus Maus aufweisen, in denen eine oder mehrere der Maus-CDRs (z. B. CDR3) durch humane CDR-Sequenzen ersetzt sind.

Der Begriff "humanisierter Antikörper" beschreibt Antikörper, die Sequenzen der variablen Region schwerer und leichter Kette aus einer nichthumanen Spezies (z. B. Maus, Ratte, Kaninchen, Huhn, Kamelid, Ziege) beinhalten, in denen jedoch wenigstens ein Teil der VH- und/oder VL-Sequenz verändert worden ist, um "humanähnlicher" zu sein, d. h. variablen Sequenzen der humanen Keimbahn ähnlicher zu sein. Eine Art eines humanisierten Antikörpers ist ein CDR-Graft-Antikörper, bei dem humane CDR-Sequenzen in nichthumane VH- und VL-Sequenzen eingesetzt sind, um die entsprechenden nichthumanen CDR-Sequenzen zu ersetzen.

Eine Methode, die Bindungskinetik eines Antikörpers zu messen, basiert auf der sogenannten Oberflächenplasmonresonanz. Der Begriff "Oberflächenplasmonresonanz" bezieht sich auf ein optisches Phänomen, mit dem sich biospezifische Wechselwirkungen analysieren lassen, indem man Veränderungen von Proteinkonzentrationen mit einer Biosensormatrix nachweist, wobei man beispielsweise das BlAcore-System verwendet (Pharmacia Biosensor AB, Uppsala, Sweden und Piscataway, NJ). Für weitere Beschreibungen siehe Jönsson, U., et al. (1993) Ann. Biol. Clin. 51:19-26; Jönsson, U., et al. (1991) Biotechniques 11:620-627; Johnsson, B., et al. (1995) J. Mol. Recognit. 8:125-131; und Johnnson, B., et al. (1991) Anal. Biochem. 198:268-277.

Der Begriff "K_{off}" beschreibt die off-Geschwindigkeitskonstante für die Dissoziation eines Antikörpers aus dem Antikörper/Antigen-Komplex.

Der Begriff "K_{d}" beschreibt die Dissoziationskonstante einer bestimmten AntikörperAntigen-Wechselwirkung.

Die Bindungsaffinität der erfindungsgemäßen Antikörper kann beurteilt werden, indem man standardisierte in vitro-Immunoassays, wie ELISA- oder BlAcore-Analysen, verwendet.

### 5.2 Herstellung von Immunglobulinen

### 5.2.1 Herstellung von polyklonalen Antikörpern

Die vorliegende Erfindung betrifft polyklonale Antikörper gegen erfindungsgemäße RGM-Domänen und -Polypeptide und deren Herstellung.

Dazu wird ein Wirt mit wenigstens einem erfindungsgemäßen RGM-Protein oder Derivat/Äquivalent davon immunisiert; und ein als Antwort auf die Immunisierung gebildetes Antikörper-haltiges Serum des Wirts gewonnen.

Sind die zu verwendenden RGM-Polypetide nicht oder nur schwach immunogen, so kann man ihre Immunogenität dadurch erhöhen, daß man sie an Träger, vorzugsweise ein Trägerprotein, wie Keyhole Limpet Hemocyanin (KLH), Limulus Polyphenus Hemocyanin ((LPH), Rinderserumalbumin (BSA) oder Ovalbumin (OVA), koppelt. Dazu stehen dem Fachmann eine Reihe von Kopplungsmöglichkeiten zur Verfügung, die allgemein bekannt sind. Zweckmäßig kann beispielsweise die Umsetzung mit Glutardialdehyd sein, beispielsweise durch Inkubation von RGM-Protein mit einem geeigneten Peptid oder Peptid-Gemisch in Wasser oder einem wässrigen Lösungsmittel. Diese Umsetzung kann bequemerweise bei Umgebungstemperatur, also in der Regel Raumtemperatur, durchgeführt werden. Es kann allerdings auch zweckmäßig sein, zu kühlen oder leicht zu erwärmen. In der Regel führt die Umsetzung innerhalb weniger Stunden zum erwünschten Ergebnis, eine Reaktionsdauer von beispielsweise 2 h liegt im üblichen Bereich. Die Glutardialdehyd-Konzentration liegt in der Regel im ppm- bis %-Bereich, zweckmäßigerweise von 10 ppm bis zu 1 %, vorzugsweise von 100 ppm bis 0,5 %. Die Optimierung der Reaktionsparameter liegt im Bereich des fachmännischen Könnens.

Zusätzlich zum Antigen enthalten die Zusammensetzungen in der Regel weitere Hilfsstoffe, insbesondere üblicherweise zur Immunisierung eingesetzte Adjuvantien, z.B. Freund-Adjuvanz. Insbesondere verwendet man komplettes Freund-Adjuvanz für die erste Immunisierung wohingegen alle weiteren Immunisierungen mit inkomplettem Freund-Adjuvanz durchgeführt werden. Zur Herstellung der Immunisierungscocktails wird das Antigen (Immunogen), vorzugsweise als oben beschriebenes Komponenten-Gemisch, zu dem oder den Hilfsstoffen gegeben. In der Regel wird dabei das Antigen emulgiert.

Als Wirt eignen sich insbesondere Nager oder auch Kaninchen. Diesen oder anderen geeigneten Wirten werden die Immunisierungscocktails, vorzugsweise subkutan, injiziert. Die Antikörpertiter können mit einem Immunoassay, beispielsweise kompetitiv mit einem gegen Wirt-IgG gerichteten Schaf-Antiserum und markiertem RGM-Protein, bestimmt werden. So kann gegen Ende der Immunisierung entschieden werden, ob ein bestimmter Wirt zur Antikörpergewinnung geeignet ist. Werden beispielsweise vier Immunisierungen durchgeführt, so kann man nach der dritten Immunisierung den Antikörpertiter bestimmen und dann aus Tieren, die einen ausreichenden Antikörpertiter aufweisen, Antikörper gewinnen.

Zur Gewinnung gebildeter Antikörper ist es bevorzugt, den Wirten über mehrere Wochen oder Monate Blut abzunehmen. Abschließend kann man den Wirt ausbluten lassen. Aus dem so gewonnenen Blut kann in an sich bekannter Weise Serum gewonnen werden, das die erwünschten Antikörper enthält. Das so erhaltene Vollserum kann erforderlichenfalls in fachmännischer Weise weiter aufgereinigt werden, um die darin enthaltene Antikörperfraktion und insbesondere die RGM-Protein-erkennenden Antikörper anzureichern.

Gemäß einer besonderen Ausführungsform dieses Verfahrens selektiert man wenigstens einen Antikörper des Serums, der das als Immunogen verwendete RGM-Protein oder ein Derivat/Äquivalent davon spezifisch erkennt. Spezifität meint in diesem Zusammenhang eine höhere Bindungsaffinität des Antikörpers für das Immunogen als für andere, insbesondere immunogen verwandte Proteine.

### 5.2.2 Herstellung von monoklonalen Antikörpern

Erfindungsgemäß brauchbare Immunglobuline sind unter Anwendung an sich bekannter Methoden erhältlich. So gestattet die Hybridomtechnologie die Herstellung von monospezifischen Antikörpern für ein interessierendes Antigen. Weiterhin sind rekombinante Antikörpertechniken, wie das in-vitro-Screenen von Antikörperbanken entwickelt worden,mit deren Hilfe sich ebenfalls derartige spezifische Antikörper herstellen lassen.

So kann man beispielsweise ein Tier mit dem interessierenden Antigen immunisieren. Dieser in-vivo-Ansatz kann des weiteren beinhalten, dass man aus den Lymphozyten oder Milzzellen eines Tieres eine Reihe von Hybridomen etabliert und ein Hybridom selektiert, das einen das Antigen spezifisch bindenden Antikörper sekretiert. Bei dem zu immunisierenden Tier kann es sich beispielsweise um eine Maus, Ratte, Kaninchen, Huhn, Kamelid oder Schaf handeln, oder um eine transgene Version eines der zuvor genannten Tiere, beispielsweise eine transgene Maus mit humanen Immunglobulingenen, die nach einem antigenen Stimulus humane Antikörper macht. Zu weiteren Typen von Tieren, die immunisiert werden können, zählen Mäuse mit schwerer kombinierter Immundefizienz (SCID), die mit humanen peripheren mononukleären Blutzellen (chimäre hu-PBMC-SCID-Mäuse) oder mit lymphoiden Zellen oder Vorläufern davon rekonstituiert worden sind, genauso wie Mäuse, die mit einer letalen Gesamtkörperbestrahlung behandelt, anschließend mit Knochenmarkszellen aus einer Maus mit schwerer kombinierter Immundefizienz (SCID) gegen Strahlung geschützt und anschließend mit funktionalen humanen Lymphozyten transplantiert worden sind (das sogenannte Trimera-System). Ein weiterer Typus eines zu immunisierenden Tieres ist ein Tier (z.B. eine Maus), in dessen Genom ein endogenes Gen, welches das interessierende Antigen kodiert, ausgeschaltet wurde ("knocked out"), z.B. durch homologe Rekombination, so dass dieses Tier nach Immunisierung mit dem Antigen das Antigen als fremd erkennt. Für den Fachmann ist klar, dass die mit diesen Verfahren hergestellten polyklonalen oder monoklonalen Antikörper charakterisiert und selektiert werden, indem man bekannte Screening-Verfahren verwendet, zu denen ELISA-Techniken gehören, ohne jedoch darauf beschränkt zu sein.

Einer weiteren Ausführungsform zufolge, screent man eine rekombinante Antikörperbank mit dem Antigen. Die rekombinante Antikörperbank kann beispielsweise auf der Oberfläche von Bakteriophagen oder auf der Oberfläche von Hefezellen oder auf der Oberfläche von bakteriellen Zellen exprimiert sein. Die rekombinante Antikörperbank kann beispielsweise eine scFv-Bank oder eine Fab-Bank sein. Gemäß einer weiteren Ausführungsform sind Antikörperbänke als RNA-Protein-Fusionen exprimierbar.

Ein weiterer Ansatz zur Herstellung erfindungsgemäßer Antikörper beinhaltet eine Kombination aus *in vivo-* und *in vitro*-Ansätzen. Beispielsweise kann man das Antigen auf das Antikörper-Repertoire einwirken lassen, indem man ein Tier mit dem Antigen *in vivo* immunisiert und anschließend eine aus lymphoiden Zellen des Tieres hergestellte rekombinante Antikörperbank oder Einzeldomänen-Antikörperbank (z.B. mit schwerer und/oder leichter Kette) mit dem Antigen *in vitro* screent. Einem weiteren Ansatz zufolge lässt man das Antigen auf das Antikörper-Repertoire einwirken, indem man ein Tier mit dem Antigen *in vivo* immunisiert und anschließend eine aus lymphoiden Zellen des Tieres hergestellte rekombinante Antikörperbank oder Einzeldomänen-Bank einer Affinitätsreifung unterzieht. Einem weiteren Ansatz zufolge lässt man das Antigen auf das Antikörper-Repertoire einwirken, indem man ein Tier mit dem Antigen *in vivo* immunisiert, anschließend einzelne Antikörper-produzierende Zellen, die einen interessierenden Antikörper sekretieren, selektiert und aus diesen selektierten Zellen cDNAs für die variable Region der schweren und leichten Kette gewinnt (z.B. mittels PCR) und die variablen Regionen der schweren und leichten Kette *in vitro* in Säugerwirtszellen exprimiert (was als das Lymphozyten-Antikörper-Selektionsverfahren, oder SLAM für "Selected Lymphocyte Antibody Method", bezeichnet wird), wodurch sich die selektierten Antikörper-Gensequenzen weiter selektieren und manipulieren lassen. Außerdem können monoklonale Antikörper durch Expressionsklonierung selektiert werden, indem man die Antikörpergene für die schwere und leichte Kette in Säugerzellen exprimiert und diejenigen Säugerzellen selektiert, die einen Antikörper mit der gewünschten Bindungsaffinität sekretieren.

Mit vorliegender Erfindung werden definierte Antigene in Form von RGM-Bindungsdomänen oder -Polypeptiden zum Screenen und Gegenscreenen zur Verfügung gestellt. So können erfindungsgemäß diejenigen polyklonalen und monoklonalen Antikörper selektiert werden, die ein erfindungsgemäß gewünschtes Eigenschaftsprofil gemäß obiger Definition aufweisen.

Mit den erfindungsgemäßen Verfahren zur Herstellung von Antikörpern lassen sich verschiedene Antikörpertypen herstellen. Hierzu gehören im Wesentlichen humane Antikörper, chimäre Antikörper, humanisierte Antikörper und CDR-Graft-Antikörper sowie Antigen-bindende Teile davon.

Im Folgenden werden Verfahren zur Herstellung erfindungsgemäßer Antikörper beschrieben. Dabei wird zwischen In-vivo-Ansätzen, In-vitro-Ansätzen, oder einer Kombination aus beiden, unterschieden.

In-vivo-Ansätze:

Ausgehend von den in-vivo erzeugten Antikörper produzierenden Zellen können monoklonale Antikörper mittels standardisierter Techniken, wie der ursprünglich von Köhler und Milstein (1975, Nature 256:495-497) (siehe auch Brown et al. (1981) J. Immunol 127:539-46; Brown et al. (1980) J Biol Chem 255:4980-83; Yeh et al. (1976) PNAS 76:2927-31; und Yeh et al. (1982) Int. J. Cancer 29:269-75) beschriebenen Hybridomtechnik, hergestellt werden. Die Technologie zur Produktion monoklonaler Antikörperhybridome ist hinlänglich bekannt (siehe allgemein R. H. Kenneth, in Monoclonal Antibodies: A New Dimension In Biological Analyses, Plenum Publishing Corp., New York, New York (1980); E. A. Lerner (1981) Yale J. Biol. Med., 54:387-402; M. L. Gefter et al. (1977) Somatic Cell Genet., 3:231-36). Eine immortalisierte Zelllinie (typischerweise ein Myelom) wird dazu mit Lymphozyten (typischerweise Splenozyten oder Lymphknotenzellen oder periphere Blutlymphozyten) eines mit dem erfindungsgemäßen RGM-Protein oder Derivat/Äquivalent davon immunisierten Säugers fusioniert, und die Kulturüberstände der resultierenden Hybridomzellen werden gescreent, um ein Hybridom zu identifizieren, das einen monoklonalen Antikörper mit Spezifität für erfindungsgemäßes RGM-Protein oder für ein Derivat/Äquivalent davon produziert. Dazu kann man ein beliebiges, von vielen hinlänglich bekannten Protokollen für die Fusion von Lymphozyten und immortalisierten Zelllinien anwenden (siehe auch G. Galfre et al. (1977) Nature 266:550-52; Gefter *et al*. *Somatic Cell Genet.,* cited *supra*; Lerner, *Yale J. Biol*. *Med.,* cited *supra*; Kenneth, *Monoclonal Antibodies,* cited *supra*). Darüber hinaus sind dem Fachmann mannigfaltige Variationen solcher Verfahren, die ebenfalls brauchbar sind, bekannt. Typischerweise stammten die immortalisierten Zelllinien (z.B. eine Myelomzelllinie) von der gleichen Säugerspezies ab wie die Lymphozyten. Beispielsweise kann man murine Hybridome etablieren, indem man Lymphozyten aus einer mit einer erfindungsgemäßen immunogenen Zubereitung immunisierten Maus mit einer immortalisierten Mauszelllinie fusioniert. Bevorzugte immortalisierte Zelllinien sind Maus-Myelomzelllinien, welche für Hypoxanthin, Aminopterin und Thymidin enthaltendes Kulturmedium (HAT-Medium) sensitiv sind. Man kann eine beliebige von vielen Myelomzelllinien als Fusionspartner standardmäßig verwenden, z.B. die P3-NS1/1-Ag4-1-, P3-x63-Ag8.653- or Sp2/O-Ag14-Myelomlinie. Diese Myelomzelllinien sind von der American Type Culture Collection (ATCC), Rockville, MD, erhältlich. Typischerweise werden HAT-sensitive Maus-Myelomzellen mit Maus-Splenozyten unter Verwendung von Polyethylenglykol (PEG) fusioniert. Die aus der Fusion resultierenden Hybridomzellen werden dann unter Verwendung von HAT-Medium selektiert, wodurch nicht fusionierte und nicht produktiv fusionierte Myelomzellen abgetötet werden (nicht fusionierte Splenozyten sterben nach mehreren Tagen ab, da sie nicht transformiert sind). Monoklonale Antikörper-produzierende Hybridomzellen, die ein erfindungsgemäßes RGM-Protein oder ein Derivat/Äquivalent davon spezifisch erkennen, werden identifiziert, indem man die Hybridomkulturüberstände auf solche Antikörper screent, z.B. indem man einen Standard-ELISA-Assay verwendet, um diejenigen Antikörper zu selektieren, die erfindungsgemäßes RGM-Protein oder ein Derivat/Äquivalent davon spezifisch binden können.

Je nach Art des gewünschten Antikörpers, können verschiedene Wirtstiere für die in-vivo-Immunisierung verwendet werden. Ein Wirt, der eine endogene Version des interessierenden Antigens selbst exprimiert, kann verwendet werden. Alternativ kann ein Wirt verwendet werden, der für eine endogene Version des interessierenden Antigens defizient gemacht wurde. Es wurde beispielsweise gezeigt, dass Mäuse, die über homologe Rekombination am entsprechenden endogenen Gen für ein bestimmtes endogenes Protein defizient gemacht wurden (d.h. Knockout-Mäuse) eine humorale Antwort gegen das Protein, mit dem sie immunisiert wurden, erzeugen und daher zur Herstellung hochaffiner monoklonaler Antikörper gegen das Protein verwendet werden können (siehe z.B. Roes, J. et al. (1995) J. Immunol. Methods 183:231-237; Lunn, M.P. et al. (2000) J. Neurochem. 75:404-412).

Für die Herstellung nicht humaner Antikörper gegen erfindungsgemäßes RGM-Protein oder ein Derivat/Äquivalent davon sind viele nicht-menschliche Säuger als Wirte für die Antikörperherstellung geeignet. Hierzu gehören Mäuse, Ratten, Hühner, Kamelide, Kaninchen und Ziegen (und Knockout-Versionen davon), wenngleich Mäuse zur Hybridomherstellung bevorzugt sind. Desweiteren kann man zur Herstellung im Wesentlichen humaner Antikörper gegen ein humanes Antigen mit dualer Spezifität ein nicht-humanes Wirtstier verwenden, das ein humanes Antikörper-Repertoire exprimiert. Zu solchen nicht-humanen Tieren gehören transgene Tiere (z.B. Mäuse), die humane Immunglobulin-Transgene tragen (chimäre hu-PBMC-SCID-Mäuse) und Mensch/Maus-Bestrahlungschimären, die nachstehend eingehender beschrieben sind.

Einer Ausführungsform zufolge ist das Tier, das mit einem erfindungsgemäßen RGM-Protein oder Derivat/Äquivalent davon immunisiert wird, ein nicht-humaner Säuger, vorzugsweise eine Maus, der durch humane Immunglobulingene transgen ist, so dass der nicht-humane Säuger nach einem antigenen Stimulus humane Antigkörper macht. Typischerweise werden in solche Tiere Immunglobulin-Transgene für schwere und leichte Kette mit humaner Keimbahnkonfiguration eingeführt, wobei die Tiere so verändert wurden, dass ihre endogenen Loci für schwere und leichte Kette inaktiv sind. Stimuliert man solche Tiere mit Antigen (z.B. mit einem humanen Antigen), werden Antikörper, die von den humanen Immunglobulinsequenzen abstammen (d.h. humane Antikörper), produziert. Aus den Lymphozyten solcher Tiere können mittels standardisierter Hypridomtechnologie humane monoklonale Antikörper gemacht werden. Zur weiteren Beschreibung transgener Mäuse mit humanen Immunglobulinen und ihre Verwendung bei der Herstellung humaner Antikörper siehe beispielsweise US-Patent Nr. 5,939,598, WO 96/33735, WO 96/34096, WO 98/24893 und WO 99/53049 (Abgenix Inc.), und US-Patent Nr. 5,545,806, Nr. 5,569,825, Nr. 5,625, 126, Nr. 5,633, 425, Nr. 5,661,016, Nr. 5,770,429, Nr. 5,814,318, Nr. 5,877,397 und WO 99/45962 (Genpharm Inc.); siehe ebenfalls MacQuitty, J.J. und Kay, R.M. (1992) Science 257:1188; Taylor, L.D. et al. (1992) Nucleic Acids Res. 20:6287-6295; Lonberg, N. et al. (1994) Nature 368:856-859; Lonberg, N. und Huszar, D. (1995) Int. Rev. Immunol. 13:65-93; Harding, F.A. und Lonberg, N. (1995) Ann. N.Y. Acad. Sci. 764:536-546; Fishwild, D. M. et al. (1996) Nature Biotechnology 14:845-851; Mendez, M. J. et al. (1997) Nature Genetics 15:146-156; Green, L.L. und Jakobovits, A. (1998) J. Exp. Med. 188:483-495; Green, L.L. (1999) J. Immunol. Methods 231:11-23; Yang, X.D. et al. (1999) J. Leukoc. Biol. 66:401-410; Gallo, M.L. et al. (2000) Eur. J. Immunol. 30:534-540.

Gemäß einer weiteren Ausführungsform kann das Tier, das mit erfindungsgemäßem RGM-Protein oder einem Derivat/Äquivalent davon immunisiert wird, eine Maus mit schwerer kombinierter Immundefizienz (SCID) sein, die mit humanen peripheren mononukleären Blutzellen oder lymphoiden Zellen oder Vorläufern davon rekonstituiert wurde. Solche Mäuse, die als chimäre hu-PBMC-SCID-Mäuse bezeichnet werden, produzieren nachgewiesenermaßen humane Immunglobulinantworten nach einem antigenen Stimulus. Zur weiteren Beschreibung dieser Mäuse und ihrer Verwendung für die Erzeugung von Antikörpern siehe beispielsweise Leader, K.A. et al. (1992) Immunology 76:229-234; Bombil, F. et al. (1996) Immunobiol. 195:360-375; Murphy, W.J. et al. (1996) Semin. Immunol. 8:233-241; Herz, U. et al. (1997) Int. Arch. Allergy Immunol. 113:150-152; Albert, S.E. et al. (1997) J. Immunol. 159:1393-1403; Nguyen, H. et al. (1997) Microbiol. Immunol. 41:901-907; Arai, K. et al. (1998) J. Immunol. Methods 217:79-85; Yoshinari, K. und Arai, K. (1998) Hybridoma 17:41-45; Hutchins, W.A. et al. (1999) Hybridoma 18:121-129; Murphy, W.J. et al. (1999) Clin. Immunol. 90:22-27; Smithson, S.L. et al. (1999) Mol. Immunol. 36:113-124; Chamat, S. et al. (1999) J. Infect. Diseases 180:268-277; und Heard, C. et al. (1999) Molec. Med. 5:35-45.

Gemäß einer weiteren Ausführungsform ist das Tier, das mit erfindungsgemäßem RGM-Protein oder einem Derivat/Äquivalent davon immunisiert wird, eine Maus, die mit einer letalen Ganzkörperbestrahlung behandelt, anschließend mit Knochenmarkszellen aus Mäusen mit schwerer kombinierter Immundefizienz (SCID) gegen Strahlung geschützt, und anschließend mit funktionalen humanen Lymphozyten transplantiert worden ist. Dieser als das Trimera-System bezeichnete Chimärentyp wird verwendet, um humane monoklonale Antikörper herzustellen, indem man die Mäuse mit dem interessierenden Antigen immunisiert und anschließend monoklonale Antikörper unter Verwendung von standardisierter Hybridomtechnologie herstellt. Zur weiteren Beschreibung dieser Mäuse und ihrer Verwendung für die Erzeugung von Antikörpern siehe beispielsweise Eren, R. et al. (1998) Immunology 93:154-161; Reisner, Y und Dagan, S. (1998) Trends Biotechnol. 16:242-246; Ilan, E. et al. (1999) Hepatology 29:553-562; und Bocher, W.O. et al. (1999) Immunology 96:634-641.

In-vitro-Ansätze:

Alternativ zur Herstellung erfindungsgemäßer Antikörper durch Immunisierung und Selektion können erfindungsgemäße Antikörper identifiziert und isoliert werden, indem man eine rekombinante kombinatorische Immunglobulin-Bank mit einem erfindungsgemäßen RGM-Protein oder Derivat/Äquivalent davon screent, um so Mitglieder der Immunoglobulin-Bank, die spezifisch an das RGM-Protein oder Derivat/Äquivalent davon binden, zu isolieren. Kits zur Erzeugung und zum Screenen von Display-Banken sind kommerziell erhältlich (z.B. das Recombinant Phage Antibody System von Pharmacia, Katalog-Nr. 27-9400-01; und der SurfZAP® Phage Display Kit von Stratagene, Katalog-Nr. 240612). In vielen Ausführungsformen ist die Display-Bank eine scFv-Bank oder eine Fab-Bank. Die Phagendisplay-Technik zum Screenen rekombinanter Antikörperbänke ist hinlänglich beschrieben worden. Beispiele für Verfahren und Verbindungen, die bei der Erzeugung und dem Screenen von Antikörper-Display-Banken besonders vorteilhaft verwendet werden können, sind beispielsweise in McCafferty et *al*. WO 92/01047, US-Patent Nr. 5,969,108 und EP 589 877 (beschreibt insbesondere den Display von scFv), Ladner et al. US-Patent Nr. 5,223,409, Nr. 5,403,484, Nr. 5,571,698, Nr. 5,837,500 und EP 436 597 (beschreibt beispielsweise die plll-Fusion); Dower *et al*. WO 91/17271, US-Patent Nr. 5,427,908, US-Patent Nr. 5,580,717 und EP 527 839 (beschreibt insbesondere den Display von Fab); Winter *et al*. International Publication WO 92/20791 und EP 368,684 (beschreibt insbesondere die Klonierung von Sequenzen für variable Immunoglobulindomänen); Griffiths et al. US-Patent Nr. 5,885,793 und EP 589 877 (beschreibt insbesondere die Isolierung von humanen Antikörpern gegen humane Antigene unter Verwendung rekombinanter Bänke); Garrard et al. WO 92/09690 (beschreibt insbesondere Phagenexpressionstechniken); Knappik et al. WO 97/08320 (beschreibt die humane rekombinante Antikörperbank HuCal); Salfeld et al. WO 97/29131, (beschreibt die Herstellung eines rekombinanten humanen Antikörpers gegen ein humanes Antigen (humanen Tumor-Nekrose-Faktor alpha), sowie in-vitro-Affinitätsreifung des rekombinanten Antikörpers) und Salfeld et al. U.S. Provisional Application Nr. 60/126,603 und die hierauf basierenden Patentanmeldungen (beschreibt ebenfalls die Herstellung rekombinanter humaner Antikörper gegen humanes Antigen (humanes Interleukin-12), sowie die *in*-*vitro*-Affinitätsreifung des rekombinanten Antikörpers) zu finden.

Weitere Beschreibungen von Screenings rekombinanter Antikörperbänke sind in wissenschaftlichen Publikationen zu finden, wie Fuchs et al. (1991) Bio/Technology 9:1370-1372; Hay et al. (1992) Hum Antibod Hybridomas 3:81-85; Huse et al. (1989) Science 246:1275-1281; Griffiths et al. (1993) EMBO J 12:725-734; Hawkins et al. (1992) J Mol Biol 226:889-896; Clarkson et al. (1991) Nature 352:624-628; Gram et al. (1992) PNAS 89:3576-3580; Garrad et al. (1991) Bio/Technology 9:1373-1377; Hoogenboom et al. (1991) Nuc Acid Res 19:4133-4137; Barbas et al. (1991) PNAS 88:7978-7982; McCafferty et al. Nature (1990) 348:552-554; und Knappik et al. (2000) J. Mol. Biol. 296:57-86.

Alternativ zur Verwendung von Bakteriophagen-Display-Systemen können rekombinante Antikörper-Banken auf der Oberfläche von Hefezellen oder bakteriellen Zellen exprimiert werden. Verfahren zur Herstellung und zum Screenen von Banken, die auf der Oberfläche von Hefezellen exprimiert sind, sind in WO 99/36569 beschrieben. Verfahren zur Herstellung und zum Screenen von Banken, die auf der Oberfläche von bakteriellen Zellen exprimiert sind, sind in WO 98/49286 genauer beschrieben.

Sobald ein interessierender Antikörper aus einer kombinatorischen Bank identifiziert ist, werden die DNAs, welche die leichten und schweren Ketten des Antikörpers kodieren, mittels standardisierter molekularbiologischer Techniken isoliert, beispielsweise mittels PCR-Amplifikation von DNA aus der Display-Packung (z.B. dem Phagen), die man während des Screenens der Bank isoliert hat. Nukleotidsequenzen von Genen für leichte und schwere Antikörperketten, mit denen PCR-Primer hergestellt werden können, sind dem Fachmann bekannt. Viele solcher Sequenzen sind beispielsweise in Kabat, E.A., et al. (1991) Sequences of Proteins of Immunological Interest, Fifth Edition, U.S. Department of Health und Human Services, NIH Publication Nr. 91-3242 und der Datenbank für Sequenzen der Humankeimbahn VBASE beschrieben.

Ein erfindungsgemäßer Antikörper oder Antikörperteil kann hergestellt werden, indem man die Gene für leichte und schwere Immunglobulinketten in einer Wirtszelle rekombinant exprimiert. Um einen Antikörper rekombinant zu exprimieren, wird eine Wirtszelle mit einem oder mehreren rekombinanten Expressionsvektoren, die DNA-Fragmente tragen, welche die leichten und schweren Immunglobulinketten des Antikörpers kodieren, transfiziert, so dass die leichten und schweren Ketten in der Wirtszelle exprimiert und vorzugsweise in das Medium, in dem die Wirtszellen kultiviert werden, sekretiert werden. Aus diesem Medium können die Antikörper gewonnen werden. Man verwendet standardisierte rekombinante DNA-Methodik, um Gene für schwere und leichte Antikörperketten zu erhalten, diese Gene in rekombinante Expressionsvektoren einzusetzen und die Vektoren in Wirtszellen einzuführen. Eine derartige Methodik ist beispielsweise in Sambrook, Fritsch und Maniatis (Hrsg.), Molecular Cloning; A Laboratory Manual, Second Edition, Cold Spring Harbor, N.Y., (1989), Ausubel, F.M. et al. (Hrsg.) Current Protocols in Molecular Biology, Greene Publishing Associates, (1989) und im US-Patent Nr. 4,816,397 von Boss et al. beschrieben.

Sobald DNA-Fragmente, die VH- und VL-Segmente des interessierenden Antikörpers kodieren, erhalten werden, können diese DNA-Fragmente mit standardisierten rekombinanten DNA-Techniken weiter manipuliert werden, beispielsweise um die Gene für variable Regionen in Gene für Antikörperketten voller Länge, in Gene für Fab-Fragmente oder in ein scFv-Gen zu überführen. Bei diesen Manipulationen wird ein VL- oder VH-kodierendes DNA-Fragment operativ mit einem weiteren ein weiteres Protein, z.B. eine konstante Antikörperregion oder einen flexiblen Linker, kodierenden DNA-Fragment verknüpft. Der Begriff "operativ verknüpft" soll hier meinen, dass die beiden DNA-Fragmente so miteinander verbunden sind, dass die von den beiden DNA-Fragmenten kodierten Aminosäuresequenzen im Leseraster (in-frame) bleiben.

Die isolierte, die VH-Region kodierende DNA kann in ein Gen für eine schwere Kette voller Länge überführt werden, indem man die VH-Region kodierende DNA mit einem weiteren, konstante Regionen der schweren Kette (CH1, CH2 und CH3) kodierenden DNA-Molekül operativ verknüpft. Die Sequenzen von Genen für konstante Regionen humaner schwerer Ketten sind hinlänglich bekannt (siehe z.B. Kabat, E.A., et al. (1991) Sequences of Proteins of Immunological Interest, Fifth Edition, U.S. Department of Health und Human Services, NIH Publication Nr. 91-3242), und DNA-Fragmente, die diese Regionen überspannen, können mittels standardisierter PCR-Amplifikation erhalten werden. Die konstante Region der schweren Kette kann eine konstante Region aus IgG1, IgG2, IgG3, IgG4, IgA, IgE, IgM or IgD sein, wobei eine konstante Region aus IgG1 oder IgG4 bevorzugt ist. Um ein Gen für ein Fab-Fragment der schweren Kette zu erhalten, kann die VH-kodierende DNA mit einem weiteren, lediglich die konstante Region CH1 der schweren Kette kodierenden DNA-Molekül operativ verknüpft werden.

Die isolierte, die VL-Region kodierende DNA kann in ein Gen für eine leichte Kette voller Länge (sowie ein Gen für eine Fab-leichte Kette) überführt werden, indem man die VL-kodierende DNA mit einem weiteren, die konstante Region CL der leichten Kette kodierenden DNA-Molekül operativ verknüpft. Die Sequenzen von Genen der konstanten Region humaner leichter Kette sind hinlänglich bekannt (siehe Kabat, E.A., et al. (1991) Sequences of Proteins of Immunological Interest, Fifth Edition, U.S. Department of Health und Human Services, NIH Publication Nr. 91-3242), und DNA-Fragmente, die diese Regionen überspannen, können mittels standardisierter PCR-Amplifikation erhalten werden. Die konstante Region der leichten Kette kann eine konstante kappa- oder lambda-Region sein, wobei eine konstante kappa-Region bevorzugt ist.

Um ein scFv-Gen zu erzeugen, können die VH- und VL-kodierenden DNA-Fragmente mit einem weiteren, einen flexiblen Linker, z.B. die Aminosäuresequenz (Gly₄-Ser)₃ kodierenden Fragment operativ verknüpft werden, so dass die VH- und VL-Sequenzen als fortlaufendes einzelkettiges Protein exprimiert werden, wobei die VL- und VH-Regionen über den flexiblen Linker miteinander verbunden sind (siehe Bird et al. (1988) Science 242:423-426; Huston et al. (1988) Proc. Natl. Acad. Sci. USA 85:5879-5883; McCafferty et al., Nature (1990) 348:552-554).

VH- und VL-Einzeldomänen mit Spezifität für erfindungsgemäßes RGM-Protein oder ein Derivat/Äquivalent davon können mit den oben beschriebenen Verfahren aus Einzeldomänen-Banken isoliert werden. Zwei VH-Einzeldomänen-Ketten (mit oder ohne CH1) oder zwei VL-Ketten oder ein Paar aus einer VH- und einer VL-Kette mit der gewünschten Spezifität können verwendet werden, um erfindungsgemäße RGM-Proteine oder Derivate/Äquivalente davon zu binden.

Um die erfindungsgemäßen rekombinanten Antikörper oder Antikörperteile zu exprimieren, können die DNAs, welche die leichten und schweren Ketten von partieller oder voller Länge kodieren, in Expressionsvektoren insertiert werden, so das die Gene mit transkriptionalen und translationalen Kontrollsequenzen operativ verknüpft sind. In diesem Zusammenhang soll der Begriff "operativ verknüpft" meinen, dass ein Antikörpergen in einem Vektor so ligiert ist, dass transkriptionale und translationale Kontrollsequenzen innerhalb des Vektors ihre beabsichtigte Funktion zur Regulation der Transkription und Translation des Antikörpergens erfüllen.

Der Expressionsvektor und die Expressionskontrollsequenzen werden so gewählt, dass sie mit der zur Expression verwendeten Wirtszelle kompatibel sind. Das Gen für die leichte Antikörperkette und das Gen für die schwere Antikörperkette können in getrennte Vektoren insertiert werden, oder beide Gene werden in denselben Expressionsvektor insertiert, was der Regelfall ist. Die Antikörpergene werden in den Expressionsvektor mittels standardisierter Verfahren insertiert (z.B. Ligation komplementärer Restriktionsschnittstellen am Antikörpergenfragment und Vektor, oder Ligation stumpfer Enden, falls keine Restriktionsschnittstellen vorhanden sind). Vor der Insertion der Sequenzen für die leichte und schwere Kette kann der Expressionsvektor bereits Sequenzen für konstante Antikörperregionen tragen. Beispielsweise ist es ein Ansatz, die VH- und VL-Sequenzen in Antikörpergene voller Länge zu überführen, indem man sie in Expressionsvektoren insertiert, die bereits die konstanten Regionen für schwere bzw. leichte Kette kodieren, so dass das VH-Segment mit dem oder den CH-Segment(en) innerhalb des Vektors operativ verknüpft ist, und auch das VL-Segment mit dem CL-Segment innerhalb des Vektors operativ verknüpft ist. Zusätzlich oder alternativ kann der rekombinante Expressionsvektor ein Signalpeptid kodieren, welches die Sekretion der Antikörperkette aus der Wirtszelle erleichtert. Das Gen für die Antikörperkette kann in den Vektor kloniert werden, so dass das Signalpeptid in Leseraster mit dem N-Terminus des Gens für die Antikörperkette verknüpft ist. Das Signalpeptid kann ein Immunglobulin-Signalpeptid oder ein heterologes Signalpeptid sein (d.h. ein Signalpeptid aus einem Nicht-Immunoglobulin-Protein). Zusätzlich zu den Genen für die Antikörperkette können die erfindungsgemäßen Expressionsvektoren regulatorische Sequenzen aufweisen, welche die Expression der Gene für die Antikörperkette in einer Wirtszelle kontrollieren. Der Begriff "regulatorische Sequenz" soll Promotoren, Enhancer und weitere Expressionskontrollelemente (z.B. Polyadenylierungssignale) einschließen, welche die Transkription oder Translation der Gene für die Antikörperkette kontrollieren. Solche regulatorischen Sequenzen sind beispielsweise in Goeddel; Gene Expression Technology: Methods in Enzymology 185, Academic Press, San Diego, CA (1990) beschrieben. Dem Fachmann ist bekannt, dass das Design des Expressionsvektors, wozu die Selektion regulatorischer Sequenzen gehört, von Faktoren abhängen kann, wie der Wahl der zu transformierenden Wirtszelle, der gewünschten Expressionsstärke des Proteins, etc. Zu bevorzugten regulatorischen Sequenzen für eine Expression in Säugerwirtszellen gehören virale Elemente, die zu einer starken Proteinexpression in Säugerzellen führen, wie Promotoren und/oder Enhancer, die vom Cytomegalovirus (CMV) (wie der CMV-Promoter/Enhancer), Simian-Virus 40 (SV40) (wie der SV40-Promotor/Enhancer), Adenovirus (z.B. der Späte Adenovirus-Hauptpromotor (AdMLP für Adenovirus Major Late Promoter) und Polyoma abstammen. Zur weiteren Beschreibung viraler regulatorischer Elemente und Sequenzen davon, siehe z.B. US-Patent Nr. 5,168,062 von Stinski, US-Patent Nr. 4,510,245 von Bell et al. und US-Patent Nr. 4,968,615 von Schaffner et al.

Zusätzlich zu den Genen für die Antikörperkette und die regulatorischen Sequenzen können die erfindungsgemäßen rekombinanten Expressionsvektoren zusätzliche Sequenzen aufweisen, wie Sequenzen, welche die Replikation des Vektors in Wirtszellen regulieren (z.B. Replikationsstartpunkte) und selektierbare Markergene. Die selektierbaren Markergene erleichtern die Selektion von Wirtszellen, in die der Vektor eingeführt wurde (siehe z.B. US-Patents Nr. 4,399,216, 4,634,665 und 5,179,017, alle von Axel *et al*.). Zum Beispiel ist es üblich, dass das selektierbare Markergen eine Wirtszelle, in die der Vektor eingesetzt wurde, gegen Wirkstoffe wie G418, Hygromycin oder Methotrexat resistent macht. Zu bevorzugten selektierbaren Markergenen gehören das Gen für die Dihydrofolatreduktase (DHFR) (zur Verwendung in dhfi-Wirtzellen mit Methotrexat-Selektion/Amplifikation) und das neo-Gen (zur G418-Selektion).

Für die Expression der leichten und schweren Ketten wird der oder werden die schwere und leichte Ketten kodierende(n) Expressionsvektor(en) mittels standardisierter Techniken in eine Wirtszelle transfiziert. Die verschiedenen Formen des Begriffs "Transfektion" sollen eine Vielzahl von Techniken erfassen, die gewöhnlicherweise zur Einführung exogener DNA in eine prokaryotische oder eukaryotische Wirtszelle verwendet werden, z.B. Elektroporation, Calcium-Phosphat-Fällung, DEAE-Dextran-Transfektion und ähnliches. Wenngleich es theoretisch möglich ist, die erfindungsgemäßen Antikörper entweder in prokaryotischen oder eukaryotischen Wirtszellen zu exprimieren, ist die Expression der Antikörper in eukaryotischen Zellen und insbesondere in Säugerwirtszellen bevorzugt, da die Wahrscheinlichkeit, dass ein korrekt gefalteter und immunologisch aktiver Antikörper zusammengesetzt und sekretiert wird, in solchen eukaryotischen Zellen und insbesondere Säugerzellen höher ist als in prokaryotischen Zellen. Über die prokaryotische Expression von Antikörpergenen ist berichtet worden, dass sie für die Produktion großer Ausbeuten aktiven Antikörpers ineffektiv sei (Boss, M.A. und Wood, C. R. (1985) Immunology Today 6:12-13).

Zu Säugerwirtszellen, die für die Expression erfindungsgemäßer rekombinanter Antikörper bevorzugt sind, gehören CHO-Zellen (einschließlich dhfr⁻-CHO-Zellen, die in Urlaub und Chasin, (1980) Proc. Natl. Acad. Sci. USA 77:4216-4220 beschrieben sind und mit einem DHFR-selektierbaren Marker verwendet werden, wie z.B. in R.J. Kaufman und P.A. Sharp (1982) Mol. Biol. 159:601-621 beschrieben ist), NS0-Myelomzellen, COS-Zellen und SP2-Zellen. Werden rekombinante Expressionsvektoren, welche die Antikörpegene kodieren, in Säugerwirtszellen eingeführt, so werden die Antikörper hergestellt, indem man die Wirtszellen so lange kultiviert, bis der Antikörper in den Wirtszellen exprimiert oder vorzugsweise der Antikörper in das Kulturmedium, in dem die Wirtszellen wachsen, sekretiert wird. Die Antikörper können aus dem Kulturmedium gewonnen werden, indem man standardisierte Verfahren zur Aufreinigung von Proteinen verwendet.

Es können ebenfalls Wirtszellen verwendet werden, um Teile intakter Antikörper, wie Fab-Fragmente oder scFv-Moleküle, herzustellen. Variationen der zuvor beschriebenen Vorgehensweise gehören selbstverständlich zur Erfindung. Beispielsweise kann es wünschenswert sein, eine Wirtszelle mit DNA zu transfizieren, die entweder die leichte Kette oder die schwere Kette (aber nicht beide) eines erfindungsgemäßen Antikörpers kodiert. Sind leichte oder schwere Ketten vorhanden, die für die Bindung des interessierenden Antigens nicht erforderlich sind, so kann die DNA, die entweder eine solche leichte oder eine solche schwere Kette oder beide kodiert, mittels rekombinanter DNA-Technologie zum Teil oder vollständig entfernt werden. Moleküle, die von solchen verkürzten DNA-Molekülen exprimiert werden, gehören ebenfalls zu den erfindungsgemäßen Antikörpern. Darüber hinaus können bifunktionale Antikörper hergestellt werden, in denen eine schwere und eine leichte Kette ein erfindungsgemäßer Antikörper sind und die andere schwere und leichte Kette Spezifität für ein anderes als das interessierende Antigen besitzen, indem man einen erfindungsgemäßen Antikörper mit einem zweiten Antikörper mittels standardisierter chemischer Verfahren quervernetzt.

In einem bevorzugten System zur rekombinanten Expression eines erfindungsgemäßen Antikörpers oder Antigen-bindenden Teils davon wird ein rekombinanter Expressionsvektor, der sowohl die schwere Antikörperkette als auch die leichte Antikörperkette kodiert, mittels Calcium-Phosphat-vermittelter Transfektion in dhfr⁻-CHO-Zellen eingeführt. Innerhalb des rekombinanten Expressionsvektors sind die Gene für die schwere und leichte Antikörperkette jeweils mit regulatorischen CMV-Enhancer/AdMLP-Promotor-Elementen operativ verknüpft, um eine starke Transkription der Gene zu bewirken. Der rekombinante Expressionsvektor trägt auch ein DHFR-Gen, mit dem sich CHO-Zellen, die mit dem Vektor transfiziert sind, selektieren lassen, indem man die Methotrexat-Selektion/Amplification verwendet. Die selektierten transformierten Wirtszellen werden kultiviert, so dass die schweren und leichten Antikörperketten exprimiert werden, und intakter Antikörper wird aus dem Kulturmedium gewonnen. Man verwendet standardisierte molekularbiologische Techniken, um den rekombinanten Expressionsvektor herzustellen, die Wirtszellen zu transfizieren, die Transformanten zu selektieren, die Wirtszellen zu kultivieren und den Antikörper aus dem Kulturmedium zu gewinnen. So betrifft die Erfindung ein Verfahren zur Synthese eines erfindungsgemäßen rekombinanten Antikörpers, indem man eine erfindungsgemäße Wirtszelle in einem geeigneten Kulturmedium kultiviert, bis ein erfindungsgemäßer rekombinanter Antikörper synthetisiert ist. Das Verfahren kann des weiteren beinhalten, dass man den rekombinanten Antikörper aus dem Kulturmedium isoliert.

Alternativ zum Screenen rekombinanter Antikörperbänke durch Phage-Display können weitere, dem Fachmann bekannte Methoden zum Screenen großer kombinatorischer Bänke eingesetzt werden, um die erfindungsgemäßen Antikörper zu identifizieren. Bei einer Art eines alternativen Expressionssytems ist die rekombinante Antikörperbank in Form von RNA-Protein-Fusionen exprimiert, wie in WO 98/31700 von Szostak und Roberts, und in Roberts, R.W. und Szostak, J.W. (1997) Proc. Natl. Acad. Sci. USA 94:12297-12302 beschrieben. In diesem System erzeugt man durch in-vitro-Translation synthetischer mRNAs, die an ihrem 3'-Ende Puromycin, ein Peptidylakzeptor-Antibiotikum, tragen, zwischen einer mRNA und dem Peptid oder Protein, das sie kodiert, eine kovalente Fusion. So kann eine spezifische mRNA aus einem komplexen Gemisch von mRNAs (z.B. einer kombinatorischen Bank) anhand der Eigenschaften des kodierten Peptids oder Proteins (z.B. des Antikörpers oder eines Teils davon) wie dem Binden des Antikörpers oder Teils davon an erfindungsgemäßes RGM-Protein oder ein Derivat/Äquivalent davon angereichert werden. Antikörper oder Teile davon kodierende Nukleinsäuresequenzen, die aus dem Screenen solcher Bänke gewonnen werden, können mit rekombinanten Mitteln in der oben beschriebenen Weise exprimiert werden (z.B. in Säugerwirtszellen) und darüber hinaus einer weiteren Affinitätsreifung unterzogen werden, indem man entweder in weiteren Durchgängen mRNA-Peptid-Fusionen screent, wobei man in die ursprünglich selektierte(n) Sequenz(en) Mutationen einführt, oder indem man andere Verfahren zur in-vitro-Affinitätsreifung rekombinanter Antikörper in der oben beschriebenen Weise verwendet.

Kombinationen aus in vivo- und in vitro-Ansätzen:

Die erfindungsgemäßen Antikörper können ebenfalls hergestellt werden, indem man eine Kombination aus in-vivo- und in-vitro-Ansätzen anwendet, wie Verfahren, in denen man zunächst erfindungsgemäßes RGM-Protein oder ein Derivat/Äquivalent davon auf ein Antikörper-Repertoire in vivo in einem Wirtstier einwirken lässt, um die Produktion von RGM-Protein- oder Derivat/Äquivalent-bindenden Antikörpern zu stimulieren, und anschließend die weitere Antikörperselektion und/oder Antikörperreifung (d.h. Optimierung) mit Hilfe einer oder mehrerer in-vitro-Techniken bewerkstelligt wird. Einer Ausführungsform zufolge kann ein solches kombiniertes Verfahren beinhalten, dass man zunächst ein nicht-humanes Tier (z.B. eine Maus, Ratte, Kaninchen, Huhn, Kamelide, Ziege oder eine transgene Version davon oder eine chimäre Maus) mit dem erfindungsgemäßen RGM-Protein oder Derivat/Äquivalent davon immunisiert, um eine Antikörper-Antwort gegen das Antigen zu stimulieren, und anschließend unter Verwendung von Immunglobulinsequenzen aus Lymphozyten, die durch die Einwirkung des RGM-Proteins oder Derivats/Äquivalents in vivo stimuliert worden sind, eine Phage-Display-Antikörperbank herstellt und screent. Der erste Schritt dieser kombinierten Vorgehensweise kann in der oben im Zusammenhang mit den in-vivo-Ansätzen beschriebenen Weise durchgeführt werden, während der zweite Schritt dieser Vorgehensweise in der oben in Zusammenhang mit den in-Vitro-Ansätzen beschriebenen Weise durchgeführt werden kann. Zu bevorzugten Methoden für die Hyperimmunisierung von nicht-humanen Tieren mit anschließendem in-vitro-Screening von Phage-Display-Bänken, die aus den stimulierten Lymphozyten hergestellt wurden, gehören diejenigen, die von BioSite Inc., siehe z.B. WO 98/47343, WO 91/17271, US-Patent Nr. 5,427,908 und US-Patent Nr. 5,580,717 beschrieben sind.

Einer weiteren Ausführungsform zufolge beinhaltet ein kombiniertes Verfahren, dass man zunächst ein nicht-humanes Tier (z.B. eine Maus, Ratte, Kaninchen, Huhn, Kamelide, Ziege oder eine Knockout- und/oder transgene Version davon, oder eine chimäre Maus) mit einem erfindungsgemäßen RGM-Protein oder Derivat/Äquivalent davon immunisiert, um eine Antikörperantwort gegen das RGM-Protein oder Derivat/Äquivalent davon zu stimulieren, und die Lymphozyten, welche die Antikörper mit der gewünschten Spezifität produzieren, selektiert, indem man (z.B. aus den immunisierten Tieren hergestellte) Hybridome screent. Die Gene für die Antikörper oder Einzeldomän-Antikörper werden aus den selektierten Klonen isoliert (mittels standardisierter Klonierungsverfahren, wie der Reversen Transkriptase-Polymerasekettenreaktion) und einer in-vitro-Affinitätsreifung unterzogen, um dadurch die Bindungseigenschaften des selektierten Antikörpers oder der selektierten Antikörper zu verbessern. Der erste Schritt dieser Vorgehensweise kann in der oben in Zusammenhang mit den in-vivo-Ansätzen beschriebenen Weise vollzogen werden, während der zweite Schritt dieser Vorgehensweise in der oben in Zusammenhang mit den in-vitro-Ansätzen beschriebenen Weise vollzogen werden kann, insbesondere indem man Verfahren zur in-vitro-Affinitätsreifung verwendet, wie diejenigen, die in WO 97/29131 und WO 00/56772 beschrieben sind.

In einem weiteren kombinierten Verfahren werden die rekombinanten Antikörper aus einzelnen isolierten Lymphozyten erzeugt, indem man eine Vorgehensweise verwendet, die dem Fachmann als Lymphozyten-Antikörper-Selektionsverfahren (SLAM) bekannt und in US-Patent Nr. 5,627,052, WO 92/02551 und Babcock, J.S. et al. (1996) Proc. Natl. Acad. Sci. USA 93:7843-7848 beschrieben ist. In diesem Verfahren wird ein nicht-humanes Tier (z.B. eine Maus, Ratte, Kaninchen, Huhn, Kamelide, Ziege, oder eine transgene Version davon, oder eine chimäre Maus) zunächst in vivo mit erfindungsgemäßem RGM-Protein oder einem Derivat/Äquivalent davon immunisiert, um eine Immunantwort gegen das RGM-Protein oder Derivat/Äquivalent zu stimulieren, und dann werden einzelne, interessierende Antikörper sekretierende Zellen selektiert, indem man einen antigenspezifischen hämolytischen Plaque-Assay verwendet. Hierzu können das RGM-Protein oder Derivat/Äquivalent davon, oder interessierende strukturell verwandte Moleküle an Schaf-Erythrozyten gekoppelt werden, wobei man einen Linker wie Biotin verwendet, wodurch sich einzelne Zellen, die Antikörper mit geeigneter Spezifität sekretieren, unter Verwendung des hämolytischen Plaque-Assays identifizieren lassen. In Anschluss an die Identifizierung von Zellen, die interessierende Antikörper sekretieren, werden cDNAs für die variablen Regionen der leichten und schweren Ketten aus den Zellen durch Reverse Transkriptase-PCR gewonnen und diese variablen Regionen können dann in Zusammenhang mit geeigneten konstanten Immunglobulinregionen (z.B. humanen konstanten Regionen) in Säugerwirtszellen wie COS- oder CHO-Zellen exprimiert werden. Die mit den aus in vivo selektierten Lymphozyten abstammenden, amplifizierten Immungrobulinsequenzen transfizierten Wirtszellen können dann einer weiteren in-vitro-Analyse und -Selektion unterzogen werden, indem man beispielsweise die transfizierten Zellen ausbreitet, um Zellen zu isolieren, die Antikörper mit der gewünschten Spezifität exprimieren. Die amplifizierten Immunglobulinsequenzen können des weiteren in vitro manipuliert werden.

### 6. Pharmazeutische Mittel

### 6.1 Allgemein

Gegenstand der vorliegenden Erfindung sind auch pharmazeutische Mittel (Zusammensetzungen), die als Wirkstoff ein erfindungsgemäßes Protein (RGM-Protein; RGM-Protein-bindende Liganden, wie anti-RGM-Protein-Antikörper,) oder eine kodierende RGM-Protein-Nukleinsäuresequenz und gegebenenfalls einen pharmazeutisch verträglichen Träger beinhalten. Erfindungsgemäße pharmazeutische Zusammensetzungen können des weiteren wenigstens ein zusätzliches therapeutisches Agens, z. B. ein oder mehrere zusätzliche therapeutische Agenzien zur Behandlung einer der hierin beschriebenen Erkrankungen beinhalten.

Zu pharmazeutisch verträglichen Trägern gehören sämtliche Lösungsmittel, Dispersionsmedien, Überzüge, antimikrobielle Agenzien, isotonisierende und die Absorption verzögernde Agenzien, und dergleichen, sofern sie physiologisch kompatibel sind.

Zu pharmazeutisch akzeptablen Trägern gehören beispielsweise Wasser, Kochsalzlösung, Phosphat-gepufferte Kochsalzlösung, Lactose, Dextrose, Succrose, Sorbitol, Manitol, Stärken, Akaziengummi, Calciumphosphat, Alginate, Traganth, Gelantine, Calciumsilikat, mikrokristalline Cellulose, Polyvinylpyrrolidon, Cellulose, Wasser, Sirup und Methylcellulose. Ferner können die Formulierungen pharmazeutisch akzeptable Träger oder übliche Hilfsstoffe, wie Gleitmittel, beispielsweise Talg, Magnesiumstearat und Mineralöl; Netzmittel; emulgierende und suspendierende Mittel; konservierende Mittel, wie Methyl- und Propylhydroxybenzoate; Antioxidantien; Antireizstoffe; Chelatbildner; Dragierhilfsmittel; Emulsionsstabilisatoren Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme-oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle umfassen. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, dargestellt ist. Vgl. auch Hager's Handbuch der Pharmazeutischen Praxis, Springer Verlag, Heidelberg.

Die pharmazeutischen Zusammensetzungen können beispielsweise zur parenteralen Verabreichung geeignet sein. Hierzu wird der Wirkstoff, wie z.B. der Antikörper, vorzugsweise als injizierbare Lösungen mit einem Wirkstoffgehalt von 0,1 - 250 mg/ml zubereitet. Die injizierbaren Lösungen können in flüssiger oder lyophilisierter Form in einem Flintglas oder Vial, einer Ampulle oder einer gefüllten Spritze als Dosierungsform zubereitet sein.

Der Puffer kann L-Histidin (1 - 50 mM, vorzugsweise 5 - 10 mM) enthalten und einen pH-Wert von 5,0 - 7,0, vorzugsweise von 6,0, aufweisen. Zu weiteren geeigneten Puffern gehören, ohne darauf beschränkt zu sein, Natriumsuccinat-, Natriumcitrat-, Natriumphosphat- oder Kaliumphosphat-Puffer.

Man kann Natriumchlorid verwenden, um die Tonizität der Lösung auf eine Konzentration von 0 - 300 mM (vorzugsweise 150 mM für eine flüssige Dosierungsform) einzustellen. Cryoschutzmittel können für eine lyophilisierte Dosierungsform mit einbezogen werden, wie z.B. Sucrose (z.B. 0 - 10 %, vorzugsweise 0,5 - 1,0 % (w/w)). Zu weiteren geeigneten Cryoschutzmitteln gehören Trehalose und Laktose. Füllstoffe können für eine lyophilisierte Dosierungsform mit einbezogen werden, z.B. Mannitol (z.B. 1 - 10 %, vorzugsweise 2 - 4 %(w/w)). Stabilisatoren können sowohl in flüssigen als auch lyophilisierten Dosierungsformen verwendet werden, z.B. L-Methionin (z.B. 51 - 50 mM, vorzugsweise 5 - 10 mM). Zu weiteren geeigneten Füllstoffen gehören Glycin und Arginin. Ebenso können Tenside verwendet werden, beispielsweise Polysorbat-80 (z.B. 0 - 0,05 %, vorzugsweise 0,005 - 0,01 %(w/w)). Zu weiteren Tensiden gehören Polysorbat-20 und BRIJ-Tenside.

Die erfindungsgemäßen Zusammensetzungen können eine Vielzahl von Formen annehmen. Zu diesen gehören flüssige, halbfeste und feste Dosierungsformen, wie flüssige Lösungen (z. B. injizierbare und infundierbare Lösungen, Lotionen, Augen- und Ohrentropfen), Liposome, Dispersionen oder Suspensionen und feste Formen, wie Pulver, Puder, Granulate, Tabletten, Pastillen, Sachets, Cachets, Dragees, Kapseln wie Hart- und Weichgelatinekapseln, Suppositorien oder vaginale Arzneiformen, halbfeste Arzneiformen, wie Salben, Cremes, Hydrogele, Pasten oder Pflaster. Auch implantierte Abgabevorrichtungen können zur Verabreichung erfindungsgemäßer Wirkstoffe verwendet werden. Die bevorzugte Form hängt von der beabsichtigten Verabreichungsart und der therapeutischen Anwendung ab. Typischerweise werden Zusammensetzungen in Form von injizierbaren oder infundierbaren Lösungen bevorzugt. Ein geeigneter Verabreichungsweg ist z.B. parenteral (z. B. intravenös, subkutan, intraperitoneal, intramuskulär). Gemäß einer bevorzugten Ausführungsform wird der Wirkstoff durch intravenöse Infusion oder Injektion verabreicht. Einer weiteren bevorzugten Ausführungsform zufolge wird der Wirkstoff durch intramuskuläre oder subkutane Injektion verabreicht.

Therapeutische Zusammensetzungen müssen typischerweise steril und unter den Herstellungs- und Lagerungsbedingungen stabil sein. Die Zusammensetzungen können als Lösung, Mikroemulsion, Dispersion, liposomal oder einer weiteren für hohe Wirkstoffkonzentrationen geeigneten, geordneten Struktur formuliert werden. Sterile injizierbare Lösungen können hergestellt werden, indem man die aktive Verbindung (wie z.B. den Antikörper) in der benötigten Menge in ein geeignetes Lösungsmittel gegebenenfalls mit einem oder einer Kombination der vorstehend genannten Inhaltsstoffe, je nach Bedarf, einbringt und anschließend steril filtriert. Dispersionen werden in der Regel zubereitet, indem man die aktive Verbindung in ein steriles Vehikel einbringt, welches ein Grunddispersionsmedium und gegebenenfalls weitere benötigte Inhaltsstoffe enthält. Im Falle eines sterilen lyophilisierten Pulvers zur Herstellung steriler injizierbarer Lösungen stellen die Vakuumtrocknung und Sprühtrocknung bevorzugte Herstellungsverfahren dar, mit denen ein Pulver des aktiven Inhaltsstoffes und gegebenenfalls weiterer erwünschter Inhaltsstoffe aus einer zuvor steril filtrierten Lösung erhalten wird. Die richtige Fliessfähigkeit einer Lösung kann beibehalten werden, indem man beispielsweise einen Überzug wie Lecithin verwendet, im Fall von Dispersionen die benötigte Partikelgröße beibehält oder Tenside verwendet. Eine verlängerte Absorption injizierbarer Zusammensetzungen kann erreicht werden, indem man ein Agens, das die Absorption verzögert, beispielsweise Monostearatsalze und Gelatine, in die Zusammensetzung mit einbringt.

Die erfindungsgemäßen Wirkstoffe können mit einer Vielzahl von Verfahren, die dem Fachmann bekannt sind, verabreicht werden, wenngleich für viele therapeutische Anwendungen die subkutane Injektion, intravenöse Injektion oder Infusion die bevorzugte Verabreichungsart darstellt. Der Fachmann weiß, dass Weg und/oder Art der Verabreichung vom gewünschten Resultat abhängen. Bestimmten Ausführungsformen zufolge kann die aktive Verbindung mit einem Träger zubereitet werden, der die Verbindung gegen rasche Freisetzung schützt, so zum Beispiel eine Formulierung mit kontrollierter Freisetzung, wozu Implantate, transdermale Pflaster und mikroverkapselte Abgabesysteme gehören. Es können biologisch abbaubare biokompatible Polymere verwendet werden, wie Ethylenvinylacetat, Polyanhydride, Polyglykolsäure, Collagen, Polyorthoester und Polymilchsäure. Die Verfahren zur Zubereitung solcher Formulierungen sind dem Fachmann allgemein bekannt, siehe zum Beispiel Sustained und Controlled Release Drug Delivery Systems, J.R. Robinson, ed., Marcel Dekker, Inc., New York, 1978.

Bestimmten Ausführungsformen zufolge kann ein erfindungsgemäßer Wirkstoff oral verabreicht werden, beispielsweise in einem inerten Verdünnungsmittel oder einem assimilierbaren essbaren Träger. Der Wirkstoff (und gewünschtenfalls weitere Inhaltsstoffe) können auch in einer Hart- oder Weichgelatinekapsel eingeschlossen, zu Tabletten verpresst oder direkt der Nahrung zugesetzt werden. Für die orale therapeutische Verabreichung können die Wirkstoffe mit Exzipienten vermischt und in Form von schluckbaren Tabletten, Buccaltabletten, Kapseln, Elixieren, Suspensionen, Sirups und dergleichen verwendet werden. Soll ein erfindungsgemäßer Wirkstoffe über einen anderen als den parenteralen Weg verabreicht werden, kann es erforderlich sein, eine Beschichtung aus einem Material zu wählen, das seine Inaktivierung verhindert.

Die erfindungsgemäßen Wirkstoffe können zusammen mit einem oder mehreren zusätzlichen therapeutischen Agenzien verabreicht werden, die bei der Behandlung der zuvor beschriebenen Erkrankungen brauchbar sind.

Die pharmazeutischen Zusammensetzungen der vorliegenden Erfindung enthalten in der Regel eine therapeutisch wirksame Menge oder eine prophylaktisch wirksame Menge wenigstens eines erfindungsgemäßen Wirkstoffs. In Abhängigkeit von der gewünschten Behandlung, ob beispielsweise eine therapeutische oder prophylaktische Behandlung gewünscht ist, können Dosierungspläne gewählt und angepasst werden. Beispielsweise kann man eine Einzeldosis, mehrere getrennte Dosen über die Zeit verteilt oder eine ansteigende bzw. sinkende Dosierung je nach den Anforderungen der therapeutischen Situation verabreichen. Es ist insbesondere von Vorteil, parentale Zusammensetzungen in Einheitsdosierungsform zu formulieren, um die Verabreichung zu erleichtern und eine Gleichförmigkeit der Dosierung zu gewährleisten.

Der behandelnde Arzt kann die für die jeweilige Therapie und den jeweiligen Wirkstoff am meisten geeignete Darreichungsform, Art der Verabreichung und Dosierung ohne weiteres bestimmen.

Eine therapeutisch oder prophylaktisch wirksame Menge eines erfindungsgemäßen Wirkstoffs kann beispielsweise im Bereich von 0,1 - 20 mg/kg und vorzugsweise 1 - 10 mg/kg liegen, ohne darauf beschränkt zu sein. Natürlich können diese Mengen je nach Art und Schwere des zu lindernden Zustandes variieren.

### 6.2 Vakzine

Die erfindungsgemäßen RGM-Proteine und Derivate/Äquivalente davon sind als Immunogen zur Vakzinierung eines zu behandelnden Patienten brauchbar.

Zu diesem Zweck brauchbare Vakzine stellen in der Regel eine pharmazeutische Zusammensetzung dar, die wenigstens ein erfindungsgemäßes RGM-Protein und/oder wenigstens ein erfindungsgemäßes Derivat/Äquivalent davon enthält. Ferner kann die Zusammensetzung einen physiologisch verträglichen Träger und gegebenenfalls weitere Hilfsstoffe, beispielsweise Immunstimulantien, enthalten.

Während geeignete Träger im Prinzip beliebig gewählt werden können, richtet sich die Art des Trägers in der Regel nach dem Verabreichungsweg. So können die erfindungsgemäßen Vakzine insbesondere in einer zur parenteralen, beispielsweise intravenösen, intramuskulären und subkutanen Verabreichung geeigneten Form formuliert werden. In diesen Fällen beinhaltet der Träger vorzugsweise Wasser, Kochsalzlösung, Alkohol, ein Fett, ein Wachs und/oder einen Puffer.

Es können beliebige einer Vielzahl von Immunstimulantien in den erfindungsgemäßen Vakzinen verwendet werden. Beispielsweise kann ein Adjuvans mit einbezogen werden. Die meisten Adjuvantien enthalten eine Substanz, die das Antigen vor einem raschen Abbau schützen sollen, wie Aluminiumhydroxid oder ein Mineralöl, sowie ein von Lipid A, aus Bortadella pertussis oder Mycobacterium tuberculosis abgeleitetes Protein. Geeignete Adjuvantien sind in der Regel kommerziell erhältlich, beispielsweise komplettes oder inkomplettes Freund-Adjuvans; AS-2; Aluminiumsalze, wie Aluminiumhydroxid (gegebenenfalls alas Gel) oder Aluminiumphosphat; Calcium-, Eisen- oder Zinksalze; eine unlösliche Suspension acylierten Tyrosins; acylierte Zucker; kationisch oder anionisch derivatisierte Polysaccharide; Polyphosphazene; biologisch abbaubare Mikrosphären; Monophosphoryl-Lipid A. Cytokine, wie GM-CSF oder Interleukin-2, -7 oder -12 können ebenfalls als Adjuvantien verwendet werden.

### 7. Therapieverfahren

### 7.1. Behandlung von neuronalen Erkrankungen

Aus dem Stand der Technik ist bereits bekannt, dass bei Verletzungen des Zentralen Nervensystems eine Akkumulation von RGM-Protein an der Läsionsstelle zu beobachten ist (vgl. Schwab et al. a.a.O.). Gleichzeitig wird dadurch das erneute Auswachsen der verletzten Nervenfasern verhindert. Neutralisierung von RGM A im Rückenmarkverletzungsmodell der Ratte durch einen polyklonalen RGM A-spezifischen Antikörper, führte zu Regeneration und funktioneller Erholung (Hata K. et al. J. Cell Biol. 173: 47 - 58, 2006). Die schädigende oder inhibitorische Wirkung auf das Nervenfaserwachstum wird durch die Bindung von RGM A an das Rezeptormolekül Neogenin vermittelt (Conrad S. et al. J. Biol. Chem. 282: 16423 - 16433, 2007). Eine Modulation, insbesondere Inhibition, der Wechselwirkung zwischen RGM und dem Rezeptormolekül Neogenin wäre deshalb geeignet, um die inhibitorische Aktivität von RGM auf das Nervenfaserwachstum zu unterbinden.

### 7.2. Behandlung von Tumorerkrankungen

Es gibt seit längerem Hinweise, die vermuten lassen, dass Neogenin mit der Entstehung und/oder der Progression von Tumorerkrankungen in ursächlichem Zusammenhang stehen könnte. So berichteten beispielsweise Meyerhardt et al., in Oncogene (1997) 14, 1129-1136, dass Neogenin in mehr als 50 untersuchten Krebszelllinien, darunter Glioblastom-, Medulloblastom-, Neuroblastom-Zelllinien sowie Zelllinien von Colorektal-, Brust-, Pankreas- und Zervixkarzinomen nachweisbar war. Eine Überexpression von Neogenin wurde außerdem in Oesophaguskrebszelllinien beobachtet (Hue et al., Clinical Cancer Research (2001) 7, 2213-2221). Eine systematische Untersuchung der Expressionsprofile von 3588 Genen in 211 Lungenadenocarzinom-Patienten lieferte kürzlich einen weiteren Hinweis auf die Beteiligung von Neogenin an der Entstehung und dem Verlauf der Tumorerkrankung (Berrar et al., J. Comput. Biol. (2005) 12 (5), 534-544).

Da außerdem bekannt ist, dass RGM eine potentielle tumorfördernde Wirkung aufweist, in dem es Zelltod durch Bindung an den tumorzell-assoziierten Neogeninrezeptor verhindern kann (Matsunaga et al. Nature Cell Biol. 6, 749 - 755, 2004), könnte durch Modulation der RGM-Neogenin-Wechselwirkung, insbesondere durch Unterbrechung dieser Wechselwirkung mit Hilfe spezifischer Anti-RGM-Antikörper ein neuer Therapieansatz zur Behandlung von Tumorerkrankungen geschaffen werden.

Neogenin-Rezeptor aktivierende Fragmente des humanen RGM A Proteins könnten andererseits die Tumorzellwanderung oder die Metastasierung von Neogenin-positiven Tumorzellen hemmen. Diese Hemmung der Tumorzellwanderung könnte in analoger Weise zur Hemmung des Nervenfaserwachstums erfolgen. Auch Nervenfasern wachsen invasiv, allerdings im Gegensatz zu Tumorzellen in der Regel kontrolliert invasiv. Ein Argument dafür ist die vor kurzem erfolgte Identifikation des hRGM A als möglicher Tumorsuppressorkandidat bem klassischen Hodgkin-Lymphom (Feys et al. Haematologica 2007, Band 92, 913 - 920).

### 7.3. Behandlung von Eisenstoffwechselerkrankungen

RGM C, auch Hemojuvelin genannt, ist von essentieller Bedeutung für den Eisenstoffwechsel des menschlichen und tierischen Körpers. Juvenile Hemochromatose ist eine vererbte, relativ seltene Eisenstoffwechselerkrankung, welche sich in einer Eisenüberladung des Organismus äußert. Für die Entstehung dieser Erkrankung sind Mutationen im Hemojuvelin-Molekül ursächlich (vgl. Huang et al., The Journal of Clinical Investigation (2005), 115, 2087-2091). Die Verabreichung von funktionalem erfindungsgemäßen RGM-Proteinen oder deren aktiven Domänen stellt daher einen brauchbaren Therapieansatz zur Linderung derartiger Eisenstoffwechselerkrankungen dar. Bei der Anämie der chronischen Erkrankungen kommt es durch entzündliche oder maligne Prozesse zu einer massiven Hochregulation bestimmter Cytokine wie z.B. Tumor Nekrose Faktor alpha (Weiss MD, Goodnough LT New Engl. J. Med. 352: 1011 - 1022, 2005). Diese Cytokine sind potente Induuktoren des wichtigsten Regulators des Eisenstoffwechsels, des Peptidhormons Hepcidin und Hepcidin-Überproduktion oder Hepcidin-Akkumulation wird als wesentliche Ursache für die Pathogeneses der Anämie der chronischen Erkrankung angesehen. Neuere Maus in vivo Daten belegen, dass Fc-konjugiertes RGM C (Fc- Hemojuvelin), Hepcidin-Expression hemmt und Serumeisenspiegel erhöht (Babitt, J. L. et al., The Journal of Clinical Investigation, 2007, Bd. 117, 7, 1933-1939). Bei dieser Regulation ist die Interaktion der RGM Proteine mit den BMP-Proteinen ein wichtiger Faktor (Babitt, J. L. et al., Nature Genetics, 2006, Bd. 48, 5, 531-539). Fc-konjugiertes RGM C oder Fc-konjugierte Fragmente von RGM C, oder RGM A, oder B, die mit BMP-Proteinen interagieren, können daher als therapeutische Mittel zur Behandlung der Anämie der chronischen Erkrankung eingesetzt werden.

### 7.4. Förderung der Bildung von Knochengewebe

Im Stand der Technik gibt es Hinweise darauf, dass ein Mitglied der RGM-Familie von Proteinen, nämlich RGM B, auch bekannt unter der Bezeichnung DRAGON, an der Knochenmorphogenese beteiligt ist. So beschreiben beispielsweise Samad et al. in JBC Papers 2005, Band 280, 14122 - 14129*,* die Wechselwirkung zwischen DRAGON und den Rezeptoren vom Typ I und Typ II des Bone Morphogenetic Protein (BMP). Durch Verabreichung erfindungsgemäßer RGM-Polypeptide ist daher eine das Knochenwachstum fördernde Wirkung und damit ein neuer Therapieansatz zur Behandlung von Erkrankungen mit gestörtem Knochenwachstum oder von Knochenverletzungen denkbar. Alle drei RGM Proteine (RGM A, B, C) interagieren mit verschiedenen Mitgliedern der BMP-Familie und steigern die Aktivierung des BMP-Signalweges (Babitt, J. L. et al., Nature Genetics, 2006, Bd. 48, 5, 531-539; Babitt, J. L. et al., J. Biol. Chem., 2005, Bd. 280, 33, 29820-29827; Babitt, J. L. et al., The Journal of Clinical Investigation, 2007, Bd. 117, 7, 1933-1939; Samad, T. A. et al., J. Biol. Chem., 2005, Bd. 280, 14, 14122-14129; Halbrooks et al. J. Molecular Signaling 2, 4: 2007: publiziert in elektronischer Form).

### 7.5 Behandlung von Autoimmunerkrankungen

Anhaltspunkte dafür, dass erfindungsgemäße Wirkstoffe für die Behandlung von Autoimmunerkrankungen brauchbar sein könnten, finden sich in folgenden Publikationen: Urist et al. Prog. Clin. Biol. Res. 1985, Band 187: 77 - 96; Lories and Luyten, Cytokine & Growth Factor Reviews 2005, Band 16, 287 - 298.

### 8. Diagnostizierverfahren

Als diagnostische Agenzien sind erfindungsgemäß insbesondere RGM-Protein und Derivate/Äquivalente gemäß obiger Definition sowie dagegen gerichtete Antikörper zu nennen.

Die vorliegende Erfindung ermöglicht daher insbesondere die verbesserte qualitative oder quantitative Bestimmung oben definierter Krankheitszustände durch Nachweis krankheitstypischer Antigene oder Antikörper.

Die Bestimmung erfolgt vorzugsweise mit immunologischen Methoden. Prinzipiell kann dies mit jedem analytischen bzw. diagnostischen Testverfahren erfolgen, bei dem Antikörper eingesetzt werden. Hierzu gehören Agglutinations- und Präzipitations-Techniken, Immunoassays, immunhistochemische Verfahren und Immunoblot-Techniken, z.B. Western-Blotting oder Dot Blot-Verfahren. Auch in vivo-Verfahren gehören dazu, beispielsweise bildgebende Verfahren.

Von Vorteil ist der Einsatz in Immunoassays. Geeignet sind sowohl kompetitive Immunoassays, d.h. Antigen und markiertes Antigen (Tracer) konkurrieren um die Antikörperbindung, als auch Sandwich-Immunoassays, d.h. die Bindung spezifischer Antikörper an das Antigen wird mit einem zweiten, meist markierten Antikörper nachgewiesen. Diese Assays können sowohl homogen, d.h. ohne eine Trennung in feste und flüssige Phase, als auch heterogen sein, d.h. gebundene Markierungen werden von ungebundenen getrennt, beispielsweise über Festphasen-gebundene Antikörper. Die verschiedenen heterogenen und homogenen Immunoassay-Formate können je nach Markierung und Meßmethode bestimmten Klassen zugeordnet werden, beispielsweise RIAs (Radioimmunoassays), ELISA (Enzyme linked immunosorbent assay), FIA (Fluoreszenz-Immunoassay), LIA (Lumineszenz-Immunoassay), TRFIA (zeitlich aufgelöster FIA), IMAC (Immunaktivierung), EMIT (Enzyme multiplied immune test), TIA (Turbodimetrischer Immunoassay), I-PCR (Immuno-PCR).

Zur erfindungsgemäßen Antigen-Bestimmung sind kompetitive Immunoassays bevorzugt. Dabei konkurriert markiertes Antigen (Tracer) mit dem zu quantifizierenden Antigen der Probe um die Bindung an den verwendeten Antikörper. Aus der Menge des verdrängten Tracers lässt sich mit Hilfe einer Standardkurve die Antigenmenge, sprich die Antigenmenge, in der Probe bestimmen.

Von den für diese Zwecke zur Verfügung stehenden Markierungen haben sich Enzyme als vorteilhaft erwiesen. Beispielsweise können Systeme auf Basis von Peroxidasen, insbesondere der Meerrettich-Peroxidase, der alkalischen Phosphatase und der β-D-Galactosidase, verwendet werden. Für diese Enzyme stehen spezifische Substrate zur Verfügung, deren Umsetzung sich z.B. photometrisch verfolgen lässt. Geeignete Substratsysteme basieren auf p-Nitrophenylphosphat (p-NPP), 5-Brom-4-chlor-3-indolylphosphat/Nitroblau-Tetrazolium (BCIP/NPT), Fast-Red/Naphthol-AS-TS-Phosphat für die alkalische Phosphatase; 2,2-Azino-bis-(3-ethylbenzthiazolin-6-sulfonsäure) (ABTS), o-Phenylendiamin (OPT), 3,3',5,5'-Tetramethylbenzidin (TMB), o-Dianisidin, 5-Aminosalicylsäure, 3-Dimethylaminobenzoesäure (DMAB) und 3-Methyl-2-benzothiazolinhydrazon (MBTH) für Peroxidasen; o-Nitrophenyl-β-D-galactosid (o-NPG), p-Nitrophenyl-β-D-Galactosid und 4-Methylumbelliphenyl-β-D-galactosid (MUG) für die β-D-Galactosidase. Diese Substratsysteme sind in vielen Fällen in gebrauchsfertiger Form kommerziell erhältlich, beispielsweise in Form von Tabletten, die auch weitere Reagenzien, wie zweckmäßige Puffer und ähnliches enthalten können.

Die Kopplung von Markierungen an Peptide oder Antikörper zur Herstellung von Tracern kann in an sich bekannter Weise erfolgen. Darüber hinaus stehen eine Reihe zur Konjugation an Proteine zweckmäßig modifizierte Markierungen zur Verfügung, beispielsweise Biotin-, Avidin-, Extravidin- oder Streptavidin-konjugierte Enzyme, Maleimid-aktivierte Enzyme und ähnliches. Diese Markierungen können direkt mit dem erfindungsgemäß zu verwendenden Molekül umgesetzt werden.

Wird ein heterogenes Immunoassay-Format gewählt, so kann der Antigen-Antikörper-Komplex zwecks Trennung beispielsweise über einen an den Träger gekoppelten antiidiotypischen Antikörper, z.B. einen gegen Kaninchen-IgG gerichteten Antikörper, an den Träger gebunden werden. Träger, insbesondere Mikrotiterplatten, die mit entsprechenden Antikörpern beschichtet sind, sind bekannt und kommerziell erhältlich.

Ein weiterer Gegenstand der vorliegenden Erfindung sind Immunoassay-Sets mit wenigstens einem vorstehend beschriebenen Antikörper und weiteren Komponenten. Es handelt sich hierbei um eine Zusammenstellung, in der Regel als Verpackungseinheit, von Mitteln zur Durchführung einer erfindungsgemäßen Bestimmung. Zwecks möglichst einfacher Handhabung werden diese Mittel vorzugsweise im wesentlichen gebrauchsfertig bereitgestellt. Eine vorteilhafte Anordnung bietet den Immunoassay in Kit-Form an. Ein Kit umfasst in der Regel mehrere Behältnisse zur getrennten Anordnung von Komponenten. Alle Komponenten können in gebrauchsfertiger Verdünnung, als Konzentrat zum Verdünnen oder als Trockensubstanz oder Lyophilisat zum Lösen oder Suspendieren bereitgestellt werden; einzelne oder sämtliche Komponenten können eingefroren sein oder bei Umgebungstemperatur bis zum Gebrauch aufbewahrt werden. Seren sind vorzugsweise schockgefroren, beispielsweise bei -20°C, so dass in diesen Fällen ein Immunoassay vor Gebrauch vorzugsweise bei Gefriertemperaturen zu halten ist.

Weitere, dem Immunoassay beigefügte Komponenten können sein: Standardprotein, Tracer; Kontrollserum, Mikrotiterplatten, vorzugsweise mit Antikörper beschichtet, Puffer, beispielsweise zum Testen, zum Waschen oder zur Umsetzung des Substrats, und das Enzymsubstrat selbst.

Allgemeine Prinzipien von Immunoassays und die Erzeugung und Verwendung von Antikörpern als Hilfsmittel in Labor und Klinik finden sich beispielsweise in Antibodies, A Laboratory Manual (Harlow, E., and Lane, D., Ed., Cold Spring Harbor Laboratory, Cold Spring Harbor, NY, 1988).

### 9. Screening-Verfahren

Gegenstand der Erfindung sind auch Verfahren zum Nachweis von Effektoren des RGM-Rezeptors (Neogenin und/oder BMP), wobei man eine Probe, in der man einen Effektor vermutet, mit einem RGM-Protein oder -Polypeptid inkubiert und den Ansatz auf die Bildung eines Effektor- RGM-Protein -Komplexes untersucht.

Derartige Effektoren können eine agonistische, partiell agonistische, antagonistische oder invers agonistische Wirkung besitzen. Es können dies z.B. synthetische niedermolekulare Supstanzen, synthetische Peptide, natürliche oder synthetische Antikörpermoleküle oder Naturstoffe sein.

Solche erfindungsgemäßen Verfahren werden in der Regel als *in vitro*-Screening-Verfahren durchgeführt, mit denen man aus einer Vielzahl verschiedener Substanzen diejenigen auslesen kann, die im Hinblick auf eine künftige Anwendung am aussichtsreichsten zu sein scheinen.

Beispielsweise können mittels kombinatorischer Chemie umfangreiche Stoffbanken angelegt werden, die eine Vielzahl potentieller Wirkstoffe umfassen. Das Durchmustern kombinatorischer Substanzbibliotheken nach Stoffen mit gewünschter Aktivität ist automatisierbar. Screening-Roboter dienen der effizienten Auswertung der vorzugsweise auf Mikrotiterplatten angeordneten Einzelassays. So betrifft die vorliegende Erfindung auch Screening-Verfahren, d.h. sowohl Primär- als auch Sekundärscreening-Verfahren, bei denen vorzugsweise wenigstens eines der nachfolgend beschriebenen Verfahren zur Anwendung kommt. Kommen mehrere Verfahren zur Anwendung, so kann das zeitlich versetzt oder gleichzeitig an ein und derselben Probe oder an verschiedenen Proben einer zu untersuchenden Substanz geschehen.

Eine effektive Technologie zur Durchführung derartiger Verfahren ist der im Bereich des Wirkstoffscreenings bekannte Scintillation Proximity Assay, kurz SPA genannt. Kits und Komponenten zur Durchführung dieses Assays können kommerziell bezogen werden, beispielweise bei Amersham Pharmacia Biotech. Im Prinzip werden solubilisierte oder membrangebundene Rezeptoren auf Szintillationssubstanz enthaltenden, kleinen Fluoromikrosphären immobilisert. Bindet beispielsweise ein Radioligand an die immobilisierten Rezeptoren, so wird die Szintillationssubstanz zur Lichtemission angeregt, da die räumliche Nähe zwischen Szintillationssubstanz und Radioligand gegeben ist.

Eine weitere effektive Technologie zur Durchführung derartiger Verfahren ist die im Bereich des Wirkstoffscreenings bekannte FlashPlateR-Technologie. Kits und Komponenten zur Durchführung dieses Assays können kommerziell bezogen werden, beispielweise bei NEN Life Science Products. Dieses Prinzip basiert ebenfalls auf Mikrotiterplatten (96er oder 384er), die mit Scintillationssubstanz beschichtet sind.

Die nach diesen Verfahren identifizierbaren Substanzen oder Teile aus Substanzgemischen sind ebenfalls Gegenstand der vorliegenden Erfindung.

Die Erfindung wird nun unter Bezugnahme auf folgende nichtlimitierende Herstellungsund Anwendungsbeispiele näher erläutert

### Experimenteller Teil

### 1. Allgemeine Arbeitsweisen

### SDS-Polyacrylamid-Gelelektrophorese

Proteine wurden aufgrund ihres Molekulargewichts in SDS-Polyacrylamidgelen (4-20 % Tris-Glycin-Gel: Invitrogen # EC6025BOX; 10-20 % Tricin-Gel: Invitrogen #EC6625BOX) getrennt. Die Proben wurden mit NuPage SDS-Probenpuffer (4 x) unter Verwendung von Reduktionsmittel gemischt. Nach 10-minütiger Inkubation im Thermomixer bei 95 °C wurden die Proben bei 125 V mit Tris/Glycin oder Tricin-SDS-Laufpuffer (Invitrogen) entwickelt. Als Molekulargewichtstandard-Proteine wurden SeeBlue oder SeeBlue Plus 2 (Invitrogen) verwendet. Die Gele wurden mit Coomassie gefärbt oder auf Nitrocellulose (Nitrocellulose Membrane Filter Paper Sandwich (Invitrogen #LC2001) transferiert.

### Coomassie-Färbung

Zur Detektion von Proteinen in Polyacrylamidgelen wurden die Proteine mit Coomassie nach dem Gellauf gefärbt. Die Gele wurden 1 Stunde in Färbelösung SimplyBlue Membran (0,2 µm pore) Safestain (Invitrogen) alternativ in kolloidalem Coomassie (0,25 % Coomassie Blue R250/L, 45 % Methanol, 10 % Essigsäure) gefärbt. Das Entfärben erfolgte unter Verwendung von entsalztem Wasser oder Entfärbelösung (40 % Methanol, 10 % Essigsäure) bis Proteinbanden klar sichtbar waren.

### Western Blot

Filterpapier und Nitrocellulose wurden 10 Minuten mit Novex-Transferpuffer mit 20 % Methanol getränkt. Das Blotting erfolgte in einer Novex-Kammer bei konstantem Strom (100 mA) über einen Zeitraum von 2 Stunden bei Zimmertemperatur.

### Dot Blot

2 µl Protein wurden in verschiedenen Konzentrationen in TTBS-Puffer auf trockene Nitrocellulosemembranen getupft. Folgende Verdünnungen wurden verwendet:
Verdünnungen:
a) 100 µg/ml ≈ 200 ng/Spot
b) 50 µg/ml ≈ 100 ng/Spot
c) 10 µg/ml ≈ 20 ng/Spot
d) 5 µg/ml ≈ 10 ng/Spot
e) 1 µg/ml ≈ 2 ng/Spot
f) 500 µg/ml ≈ 1 ng/Spot

Nach dem Auftupfen der Proben wurde die Membran 10 Minuten bei Zimmertemperatur getrocknet, bevor das Immundetektions-Protokoll gestartet wurde.

### Transfektion und Expression von RGM A-Fragmenten in HEK 293F-Zellen

Hierzu wurde das von Invitrogen bereitgestellte Protokoll für die Tansfektion von HEK 293F-Zellen verwendet. Die Zellen wurden in Kultur über einen Zeitraum von 2-3 Tagen in Free Style 293 Expressionsmedium gezüchtet und anschließend bei 400 x g zentrifugiert und der Überstand wurde verworfen. Das Zell-Pellet wurde in Medium resuspendiert und auf 3 x 10⁷ Zellen in 28 ml frischem Medium eingestellt. Dieses wurde in einen 125-ml-Erlenmeyer-Kolben transferiert und in einem Inkubator bei 37 °C und 8 % CO₂ auf einem Orbitalschüttler bei 150 Upm inkubiert, bis das Transfektionsgemisch hergestellt war.

Transfektionsgemische mit 293fectin-DNA-Komplex wurden wie folgt hergestellt:
(1) 30 µg DNA wurden mit Opti-MEM I auf ein Gesamtvolumen von 1000 µl verdünnt und gemischt (als Kontrolle wurden 1000 µl Opti-MEM I verwendet).
(2) 35 µl 293fectin (Invitrogen # 12347-019; 1 ml) wurden mit Opti-MEM I auf ein Gesamtvolumen von 1.000 µl verdünnt, gemischt und 5 Minuten bei Zimmertemperatur inkubiert.
(3) Das DNA-Gemisch aus Schritt 1 und die 293fectin-Lösung wurden auf ein neues Röhrchen übertragen, vorsichtig gemischt und nach 25-minütiger Inkubation bei Zimmertemperatur den Zellen im Erlenmeyer-Kolben zugesetzt.
   Die Zellen wurden mit diesem Transfektionsgemisch 40 bis 48 Stunden wie oben beschrieben in einem Inkubator bei 37 °C, 8 % CO₂ auf einem Orbitalschüttler bei 150 Upm inkubiert. Die Zellüberstände wurden durch 10-minütige Zentrifugation bei 400 x g geerntet.

### Reinigung von Proteinen unter Verwendung von Ni-Chelat-Affinität (Ni-NTA)

Ni-NTA Superflow Beads (Qiagen #1018611) wurden verwendet. Die Beads wurden 3 Minuten in PBS (phosphatgepufferter Kochsalzlösung, Invitrogen) durch Zentrifugation der Bead-Suspension bei 13500 Upm gewaschen. Die Überstände wurden verworfen und die Beads wurden in frischem PBS resuspendiert. 200 µl der Bead-Suspension wurden für 30 ml Zellkulturüberstand verwendet. Die Beads wurden mit den Zellkulturüberständen über Nacht bei 4 °C auf einem Schüttler (60 Upm) inkubiert und nach der Inkubation zentrifugiert (10 Min, 3000 Upm), um die Beads zu pelletieren. Die Überstände wurden verworfen und die Beads wurden 3-fach mit PBS gewaschen. Gebundene Proteine wurden von den Beads unter Verwendung von 250 µl Elutionspuffer (PBS, 160mM NaCl, 150 mM Imidazol) eluiert. Nach 30-minütiger Inkubation auf einem Schüttler bei Zimmertemperatur wurden die Beads durch Zentrifugation (3 Min, 13500 Upm) pelletiert. Die Überstände wurden gesammelt. Das eluierte Protein wurde zur weiteren Analyse bei -20 °C eingefroren.

### Immundetektion

Für die Immundetektion von immobilisierten Proteinen auf Nitrocellulose wurde eine unspezifische Bindung von Proteinen durch 1-stündige Inkubation der Blots in TTBS (0,1 % Tween 20, Tris-gepufferte Kochsalzlösung (TBS)) bei Zimmertemperatur oder 4 °C über Nacht blockiert. Der Primärantikörper wurde in einer Konzentration von 10 µg/ml in TTBS für eine Dauer von 2 Stunden bei Zimmertemperatur verwendet. Die Blots wurden 3-fach in TTBS gewaschen und mit Sekundärantikörper (Alkalische Phosphatase-konjugierter anti-Maus-IgG-Antikörper; Sigma), 1 : 5000 verdünnt in TTBS, 1 Stunde bei Zimmertemperatur inkubiert. Die Blots wurden 3-fach in TTBS gewaschen und mit dem AP-Substrat NBT/BCIP (Roche, 1 Tablette gelöst in 10 ml Reinwasser) durch Bedecken des Blots mit der Färbelösung über einen Zeitraum von 3 Minuten entwickelt. Die Farbreaktion wurde durch Zusatz von reinem Wasser zu dem Blot gestoppt.

### Testmethode 1: Darstellung der Wirkung von RGM Peptiden im Neuritenwachstumsassay mit SH-SY5Y

SH-SY5Y-Zellen sind menschliche Neuroblastomzellen. Blastome sind embryonale Tumore, die während der Gewebe- und Organbildung entstehen. Häufig ist die Herkunft der Blastomzellen nicht bekannt, da in frühen Embryonalstadien die Differenzierung vieler Zellen noch sehr jung ist, das heißt sie sind eine heterogene Zellpopulation. Die Zellen eines Neuroblastoms, Neuroblasten, stammen aus der Neuralleiste (Struktur des Embryonalstadiums) aus dem autonomen Nervengewebe und sind praktisch in einem unreifen Stadium stehen geblieben. Die heutigen Zellen stammen von einer clonalen Subkultur von der neuroepitheliomen Zelllinie SK-N-SH ab, die 1970 durch eine Knochenmarksbiopsie eines 4Jahre alten Mädchens mit Metastasen von Neuroblastomen isoliert wurden. [http://www.dsmz.de/human_and_animal_cell_lines]

**Kulturmedium für SH-SY5Y-Zellen (ECACC Nr. 94030304)**

| | |
|---|---|
| 250 ml | EBSS (Invitrogen)50% |
| 250 ml | F12 (Ham) NUT MIX + Glutamax I (Invitrogen) 50% |
| 50 ml | FBS 10% (JRH Biosciences) (hitzeinaktiviert) |
| 5 ml | NEAA (Sigma) (100x) 1% |
| 5 ml | Penicillin / Streptomycin 1 % (Invitrogen) |

Das Medium wurde mit einer 500 ml Filtereinheit von NUNC sterilfiltriert und bis zum Gebrauch im Kühlschrank bei 4°C aufbewahrt. Vor der Verwendung wurde es auf 37°C in einem Wasserbad erwärmt.

SH-SY5Y-Zellen sind epithelial-neuronal-ähnliche Zellen, die langsam in einer Monolayer adhärent wachsen und niemals eine Konfluenz von 100% erreichen (maximal 80%). Die Zellkultur wurde zweimal pro Woche 1: 3 gesplittet. Um die Zellen zu vereinzeln ist eine Inkubation mit Trypsin von 1-3 Minuten im Brutschrank notwendig.

Da es sich um eine Population von noch unreife Vorläuferzellen handelt besteht die Möglichkeit die neuronal-ähnlichen Zellen mit Retinsäure zu differenzieren, so dass ein bestimmter Prozentsatz der Zellen neuritenähnliche Ausläufer bekommen. Dazu wurde die Kultur 3 Tage mit 10 µM Retinsäure im Medium je nach Versuch direkt in der Kulturschale oder der Flasche inkubiert.

Mikrotiterplatten (96 well) (mit Kollagen I - beschichtete Platten) werden zusätzlich mit den hRGM A Fragmenten beschichtet. Nach zweimaligem Waschen mit PBS werden die Zellen ausgesät. 18 - 24 h später erfolgt die Fixierung der Kulturen und ihre Färbung. Bei der quantitativen Auswertung werden die SH-SY5Y Zellen, die auf hRGM A Fragmenten wachsen mit SH-SY5Y Zellen verglichen, die auf Kollagen I allein wachsen. Die Neuritenlänge der Zellen wird automatisch gemessen und dient der Auswertung.

### Testmethode 2: Darstellung der Wirkung von RGM Peptiden im Neuritenwachstumstest mit Ntera-2 Zellen

**Die humane, pluripotente Karzinom-Zellinie Ntera2 (DSMZ ACC527) ist als Zellkulturmodell etabliert. Neuriten wachsen dabei aus Zell-Aggregaten aus und bilden eine Korona aus Neuriten um das jeweilige Aggregat.**

NTera-2 Zellen sind menschliche, embryonale Teratokarzinomzellen. Ein Karzinom ist eine Krebsgeschwulst, während ein Teratom oder Teratokarzinom eine Mischgeschwulst der Keimzellen ist, die aus verschieden differenzierten und undifferenzierten Gewebe und somit wie die SH-SY5Y Kultur aus einer heterogenen Population besteht. Meist liegt die Geschwulst in einer gekapselten Form vor in der sich verschiedene Gewebetypen, wie Haare, Haut, Zähne, Muskel- und Nervengewebe, befinden. Sie entstehen typischer Weise in den Eierstöcken, Hoden oder Bauchraum und Gehirn. Die Zellinie wurde aus der Linie TERA-2 geklont, die aus einem metastatischen Teratokarzinom eines 22 Jährigen Kaukasiers erhalten wurden. [http://de.wikipedia.org/wiki/] [http://www.dsmz.de/human_and_animal_cell_lines]

Ntera-2sind epitheliale, adhärent wachsende Zellen, die eine Monolayerschicht bilden. Sie sind sehr gut von den anderen Zellen zu unterscheiden, weil sie viele granuläre Partikel in sich tragen. Zur Kultivierung wurden sie zwei mal wöchentlich 1:5 gesplittet. Es ist möglich die Zellen dieser gemischten Kultur mit Retinsäure in neuronale Zellen zu differenzieren.

**Kulturmedium für Wachstum**

| | |
|---|---|
| 500 ml | DMEM (Dulbecco's Modified Eagle Medium) |
| 50 ml | FBS 10% (hitzeinaktiviert) |
| 10 ml | Pferdeserum (HS) 5% (hitzeinaktiviert) |

Das Medium wurde mit einer 500 ml Filtereinheit von NUNC sterilfiltriert und bis zum Gebrauch im Kühlschrank bei 4°C aufbewahrt. Vor der Verwendung wurde es auf 37°C in einem Wasserbad erwärmt.

### Differenzierung von NTera-2

Zur Differenzierung der Zellen war es notwendig in das Kulturmedium der Zelllinie Antibiotikum hinzuzufügen, da die Zellen drei Wochen in der selben Kulturflasche verblieben. Zuvor wurde die undifferenzierte Kultur abtrypsiniert und mit der Neubauer-Zählkammer die Zellzahl bestimmt. Es wurden 2,5 Millionen Zellen in eine neue Kulturflasche mit 25 ml Medium überführt. 25 µl Retinsäure (10 µM) wurde frisch und unter abgedunkelten Bedingungen zum Medium hinzugegeben. Sie wurde in Aliquots (10 M) im Kühlschrank aufbewahrt und vor Gebrauch im Thermomixer bei 22°C resuspendiert,

**Kulturmedium für Differenzierung**

| | |
|---|---|
| 500 ml | DMEM (Dulbecco's Modified Eagle Medium) |
| 50 ml | FBS 10% (hitzeinaktiviert) |
| 10 ml | Pferdeserum (HS) 5% (hitzeinaktiviert) |
| 5 ml | Penicillin / Streptomycin 1 % |

Die Kultur wurde im Brutschrank bei 37°C und 5% CO₂-Begasung aufbewahrt und jeweils zu Beginn und zum Ende der Woche wurde ein Mediumswechsel vorgenommen. Mit der Zeit wachsen die Zellen nicht mehr als Monolayer, sondern bilden kleinere Häufchen von Zellansammlungen, die schon ohne Lichtmikroskop als helle Punkte auf dem Zellrasen zu erkennen sind. Zur weiteren Kultivierung erfolgte ein Mediumswechsel indem das alte Medium abgesaugt, dann mit 10 ml PBS gewaschen und anschließend 25 ml frisches Kulturmedium dem Retinsäure zugesetzt wurde. Nach drei Wochen sind die Zellen ausdifferenziert und bereit zum Einsatz in Experimenten.

### Plattenbeschichtung für NTera-2

Zum Wachstum der NTera-2 Zellen auf Kulturschalenböden war eine Beschichtung mit Poly-L-Lysin / Laminin förderlich.

Für die Versuche kamen unterschiedliche Platten-Formate zum Einsatz. Für die Isolierung von Protein waren 6-well-Platten notwendig um eine ausreichende Menge zu gewinnen. Zur RNA Isolierung und für die Immunfluoreszenz genügten die 24-well-Platten, während der Assay hauptsächlich in den 96-well-Platten etabliert wurde. Entsprechend dem Fassungsvermögen der wells wurden unterschiedliche Volumina zum Beschichten und Waschen eingesetzt. Sie sind der Tabelle zu entnehmen.

Zunächst wurde eine Poly-L-Lysin Lösung (100 µg/ml) in die wells gegeben und für 15 Minuten bei Raumtemperatur inkubiert. Danach wird die Lösung abgesaugt und die wells 3 x für 5-10 Minuten mit PBS gewaschen. Anschliessend erfolgte noch ein Waschschritt mit sterilen Milipore-Wasser.

Nach dem Waschen wurde eine Laminin Lösung (20 µg/ml) in die wells pipettiert und die Platte für 2 Stunden bei 37°C und 5% CO₂-Begasung im Brutschrank inkubiert. Anschliessend wurde wiederum 3 x 5-10 Minuten mit PBS gewaschen und zuletzt das PBS durch Neurobasalmedium mit oder ohne Pen/Strep ersetzt.

| Plattentyp | Poly-L-Lysin oder Laminin | PBS |
|---|---|---|
| 6-well | 1 ml | 2 ml |
| 24-well | 250 µl | 500 µl |
| 24-well + Glasplättchen | 350 µl | 500 µl |
| 96-well | 50 µl | 100 µl |

### Neurite Outgrowth mit NTera-2

Nach 3 Wochen der Differenzierung erfolgte eine 1:6 Splittung auf 6 Kulturflaschen um die neuronalen Zellen zu selektieren. Das Medium wurde zu diesem Zeitpunkt nicht mehr mit Retinsäure versetzt. Innerhalb der nächsten zwei Tage, am dritten Tag wurden die Zellen geerntet, setzen sich die neuronalen Zellen bevorzugt auf anderen nicht-neuronalen Zellen ab, wachsen aber nicht zu fest an, so dass sie als oberste Schicht einfach abgeschlagen werden konnten. Dazu wurde das Kulturmedium abgenommen, mit ca. 10 ml PBS gewaschen und erneut 10 ml PBS in die Flasche gegeben. Während des seitlichen Schlagens der Flasche lösten sich die Zellen almählich vom Untergrund. Da allerdings die nicht-neuronalen Zellen haften bleiben sollten, wurde ab und an unter dem Lichtmikroskop eine optische Kontrolle durchgeführt. Die neuronalen Zellen sind dadurch zu erkennen, dass sie einen hell-leuchtenden Rand besitzen und sehr abgekugelt sind. Sind sie schon zu fest gewachsen, dann behalten sie eine Weile die Neuriten nach dem Abschlagen bei. Die PBS-Zell-Lösung von jeweils 3 Flaschen wurde in einem 50 ml Zentrifugenröhrchen vereint und die Zellen 5 Minuten bei 1000 rpm und Raumtemperatur abzentrifugiert. Danach wurde der Überstand in den 2 Röhrchen abgesaugt und die Pellets zusammen in 10 ml Neurobasal Medium, das heißt in 10 ml vereinigt, gut resuspendiert. Die Bestimmung der Zellzahl erfolgte mit der Neubauer-Zählkammer. Das Neurobasal Medium ist ein Spezialmedium für die weitere Kultivierung der bereits ausdifferenzierten NTera-2 Zellen.

**Kulturmedium**

| | |
|---|---|
| 100 ml | Neurobasal Medium¹⁾ |
| 2 ml | B27-Supplement 2% |
| 1 ml | L-Glutamin 2 mM 1% |
| 1 ml | Penicillin / Streptomycin 1 % |

| | |
|---|---|
| ¹⁾(Neurobasal Medium (Gibco/Invitrogen 21103-049) | |

### Bildung von Aggregaten mit differenzierten NTera-2

Zur Bildung von Zell-Aggregaten wurden die abgeschlagenen, differenzierten Zellen (siehe vorheriges Kapitel), die in Neurobasal Medium gelöst waren, nach der Zellzahlbestimmung auf eine Konzentration von ca. 1 Millionen Zellen / ml mit Neurobasal Medium verdünnt. 20 ml dieser Zellsuspension wurden in einen 100 ml sterilen Einweg-Schüttelkolben überführt und im Brutschrank bei 37°C und 5% CO₂-Begasung und unter ständigen Schütteln über Nacht inkubiert. Wichtig dabei war, dass das Volumen von 20 ml nicht überschritten wurde, da sonst keine Rotation der Flüssigkeit stattfindet und die Zellen keine schönen Aggregate bilden.

Die mit Polylysin/Laminin beschichteten 96 well Platten wurden zusätzlich mit den hRGM A Fragmenten beschichtet. Nach 2x maligem Waschen mit PBS werden die NTera Aggregate ausgesät. 18 - 24 h später erfolgt die Fixierung der Kulturen und ihre Färbung. Bei der quantitativen Auswertung werden die NTera Aggregate, die auf hRGM A Fragmenten wuchsen mit NTera Aggregaten verglichen, die auf Polylysin/Laminin allein wuchsen. Die Neuritenlänge der NTera Aggregate wurde automatisch gemessen nach der Methode wie von Lingor et al beschrieben (J. Neurochem. 2007, publiziert in elektronischer Form).

Die inhibitorische Wirkung von RGM Peptiden und Fragmenten wurde durch Zugabe unterschiedlicher Konzentrationen der zu testenden Substanzen analysiert. Alternativ wurden RGM Fragmente als Substrat angeboten.

### Testmethode 3: RGM A- Neogenin Bindungstest:

a) Materialen:
   - Immuno-Platte: Cert. Maxi Sorp F96 (NUNC, 439454)
   - Rekombinantes humanes RGM A, R&D Systems; Prod.#2495-RM (260µg/ml)
   - Rekombinantes humanes Neogenin-Fc, Abbott; Ludwigshafen (ALU 1514/122; 425µg/ml)
   - Peroxidase-conjugierter, affinitätsgereinigter Maus-anti human IgG-Fc Fragment AK (Jackson Immuno Research, Code:209-035-098 (0,8mg/ml))
   - Entwicklersubstrate: Immuno Pure TMB Substrate Kit (Pierce,#34021)
   - Schwefelsäure (Merck,#4.80354.1000)
b) Methode:
   1. RGM A Bindung an Immuno-Platte:
      - 2,5µg/ml RGM A (R&D) in 50 mM Na2CO3 (50 µl/ well)
      - Inkubation 1h bei 37°C
   2. Waschschritt:
      - 3 x mit PBS/ 0,02% Tween20 (100µl/ well) waschen
   3. Blockierung unspezifischer Bindungsstellen
      - Blockierung mit 3% BSA in PBS/0,02% Tween (200µl/ well)
      - Inkubation 1h bei 37°C
   4. Neogenin Bindung:
      - Zugabe von Neogenin in Verdünnungen (Start Konz. 1µg/ml) in 1 %BSA PBS/0,02% Tween
      - Inkubation 1h bei 37°C
   5. Waschschritt:
      - 3 x mit PBS/ 0,02% Tween20 (100µl/ well) waschen
   6. Antikörper Detektion des gebundenen Neogenins:
      - Zugabe von HRP gekoppeltem Maus anti human IgG-Fc Fragment AK (1:2500 in PBS/1%BSA verdünnt) (50µl /well)
      - Inkubation 1h bei 37°C
   7. Waschschritt:
      - 3 x mit PBS/ 0,02% Tween20 (100µl/ well) waschen
   8. Entwicklung
      - Zugabe von 50µl/ well des Entwicklungssubstrates (Immuno Pure TMB substrate, Pierce)
      - Inkubation 1-30 min, Raumtemperatur,
      - Reaktion stoppen mit 50µl 2,5M H2SO4 / well

### Testmethode 4: In-vitro-Interaktionstest zur Bestimmung der Wechselwirkung zwischen RGMA und BMP-2 und -4

Die Interaktionstests wurden wie unten beschrieben durchgeführt:

### Variante A: Immobilisierung des BMP-2/ oder -4 Proteins und Nachweis der Bindung verschiedener RGMA-Fc Fusionsproteine

1. Platte:
   - Immuno Plate Cert. Maxi Sorp F96 (Fa.NUNC, 439454)
2. Coating:
   Recombinant Human BMP-2, Kat. Nummer:355-BM, Fa.: R&D Systems; Recombinant Human RGM A R&D Systems; Prod.#2495-RM oder
   Recombinant Human BMP-4, Kat. Nummer:314-BM, Fa.: R&D Systems;
      - Konzentration: 10µg/ml
      - Eingesetztes Volumen: 2,5µg/ml in Na₂CO₃; Zugabe: 50µl pro well
      - 1 h bei 37°C in feuchter Kammer
3. Waschschritt:
   - 3 x waschen mit PBS/0,02%Tween20
4. Blockieren:
   - 3% BSA in PBS/0,02%Tween, 1h bei 37°C in feuchter Kammer;Zugabe: 200µl pro well
5. RGMA-Peptide:
   RGMA-Fc Fragmente,
      - 1µg/ml Anfangskonzentration, dann 1:2 herunter verdünnt mit PBS/0,02%Tween20
      - 1h h RT inkubieren
      - 1 h bei 37°C in feuchter Kammer
6. Waschschritt:
   - 3 x waschen mit PBS/0,02%Tween20
7. Antikörper:
   Biotin anti-human Fc (R&D-Systems), Kat. Nummer:709065; 1mg/ml;
      - 1:200 in 0,6% BAS/PBS-T (0,02%Tween)
      - Zugabe: 50µl pro well
      - 1 h bei 37°C in feuchter Kammer
8. Sekundär-AK: Strep. POD (Roche);Kat. Nummer:11089153001
   - 500U; 1:5000 in 0,6% BAS/PBS-T (0,02%Tween)
   - Zugabe: 50µl pro well
   - 1 h bei 37°C in feuchter Kammer
9. Waschschritt:
   - 3 x waschen mit PBS/0,02%Tween20
10. Substrat:
   Immuno Pure TMB Substrat Kit; Fa.Pierce,#34021
      - Entwicklungsdauer: ca.1-30Min.
      - 1:1 Gemisch PBS/0,02%Tween20 ; Zugabe: 50µl pro well
11. Stop:
   - 2,5M H₂SO₄; Zugabe: 50µl pro well

### Variante B: Immobilisierung verschiedener RGMA-Fc Fusionsproteine und Nachweis der Bindung des BMP-2/ oder -4 Proteins

1. Platte:
   Immuno Plate Cert. Maxi Sorp F96 (Fa.NUNC, 439454)
2. Coating:
   RGMA-Fc Fragmente
      - Eingesetztes Volumen: 2,5µg/ml in Na₂CO_{3;} Zugabe: 50µl pro well
      - 1 h bei 37°C in feuchter Kammer
3. Waschschritt:
   - 3 x waschen mit PBS/0,02%Tween20
4. Blockieren:
   - 3% BSA in PBS/0,02%Tween, 1 h bei 37°C in feuchter Kammer
   - Zugabe: 200µl pro well, Inkubation: 1 h, bei 37°C in feuchter Kammer
5. BMP-Peptide:
   Recombinant Human BMP-2, Kat. Nummer:355-BM, Fa.: R&D Systems; oder
   Recombinant Human RGM A R&D Systems; Prod.#2495-RM, recombinant human BMP-4, Kat. Nummer:314-BM, Fa.: R&D Systems;
   Konzentration jeweils: 10µg/ml
   Verdünnungsschritte jeweils 1:2 mit PBS/0,02%Tween20
6. Waschschritt:
   - 3 x waschen mit PBS/0,02%Tween20
7. Antikörper:
   Anti-human BMP-4 AK-Biotin; Catno.:BAM7572 1:200 in 1 %BSA-PBS-T
8. Waschschritt:
   - 3 x waschen mit PBS/0,02%Tween20
9. Sekundär-AK:
   Strep. POD (Roche)
   Catno.:11089153001; 500U; 1:5000 in 0,6% BAS/PBS-T (0,02%Tween)
      - Zugabe: 50µl pro well
      - 1 h bei 37°C in feuchter Kammer
10. Waschschritt:
   - 3 x waschen mit PBS/0,02%Tween20
11. Substrat:
   Immuno Pure TMB Substrat Kit; (Fa.Pierce,#34021)
      - Entwicklungsdauer: ca.1-30Min.
      - 1:1 Gemisch PBS/0,02%Tween20; Zugabe: 50µl pro well
12. Stop:
   - 2,5M H₂SO₄; Zugabe: 50µl pro well

### 2. Herstellungsbeispiele

### Herstellungsbeispiel 1: Herstellung von RGM A-Protein-Fragmenten in Säugetierzellen

Zur Charakterisierung der aktiven RGM A wurden folgende RGM A Moleküle in Säugetierzellen (HEK293) als Fc-Fusionsproteine exprimiert:
- 41-168/Xa
- 47-90
- 47-168
- 316-386
- 1-450

Dazu wurde die für das jeweilige Molekül kodierende DNA in den Vektor: **pcDNA3.1(+)Zeo IgK/XaihIgG lambda hc 257-Stop)** x Hindlll/EcoRI/Phosphatase, (Invitrogen) kloniert. Die für den jeweiligen Fragmentbereich kodierende DNA wurde zu diesem Zweck mittels PCR aus dem RZPD Klon (Klon AL136826 (DKFZp434D0727); publizierte RZPD Sequenz: BC015886, AL136826) amplifiziert. Dazu wurden unten aufgelistetetn Oligonukleotid-Primer eingesetzt, die von der publizierten RGMA Sequenz (publizierte Sequenz: NM_020211) abgeleitet wurden.

Die PCR wurde jeweils mit diesen Primern und AccuPrime Polymerase auf oben genannten RZPD Klon pSport-1 DKFZp434D0727 durchgeführt. Nach Aufreinigung der PCR-Produkte, Verdau mit HindIII/EcoRI und Elution der daraus resultierenden Bande erfolgte die Ligation des gewünschten Fragments in **pcDNA3.1(+)Zeo IgK/Xa/hIgG lambda hc 257-Stop)** x HindIII/EcoRI/Phosphatase. Mit dem erhaltenen Produkt wurden NEBTurbo Zellen (Invitrogen) bzw TOP10 Zellen (Invitrogen) transformiert. Die erhaltenen Klone wurden durch Sequenzierung auf die Richtigkeit der enthaltenen Sequenz überprüft.

### 41-168/Xa:

- AM 131:: GGGGAAGCTT***TT*C**CCCGCAGCCACCTCC (SEQ ID NO: 17) (hRGMA sense Primer ab Aminosäure); ***F41*** mit HindIII Schnitt
- AM 132:: GGGGGAATTCAAACGACCTTCGAT***GTC***CCCGAAGAGGCCACAGTG (SEQ ID NO: 18)
hRGMA antisense Primer bis Aminosäure ***D168*** mit Factor Xa und EcoRI Schnittstelle

Transformation in NEBTurbo Zellen;
Plasmidname: **pcDNA3.1(+)Zeo IgK/hRGMA 41-168/Xa/Xa/hIgG lambda hc 257-Stop (no att)**

### 47-90:

- AM 169:: GGGGAAGCTTCCGTGCAAGATCCTCAAGTGCAAC (SEQ ID NO: 19) hRGMA sense Primer ab Aminosäure **P47** mit HindIII Schnitt
- AM 171:: CCCCGAATTCAA***GGC***CGTCCGCCGCG (SEQ ID NO: 20) hRGMA antisense Primer bis Aminosäure **A90** mit EcoRI Schnittstelle

Transformation in TOP10 Zellen, Laborjournal ALU2163/5
Plasmidname : **pcDNA3.1(+)Zeo IgK/hRGMA 47-90/Xa/hlgG lambda hc 257-Stop (no att)**

### 47-168:

- AM 169:: GGGGAAGCTT***CCG***TGCAAGATCCTCAAGTGCAAC (SEQ ID NO: 19) hRGMA sense Primer ab Aminosäure **P47** mit HindIII Schnitt
- AM 175:: CCCCGAATTCAA***GTC***CCCGAAGAGGCCACAGTG (SEQ ID NO: 21) hRGMA antisense Primer bis Aminosäure ***D168*** mit EcoRI Schnittstelle

Transformation in TOP10 Zellen,
Plasmidname : **pcDNA3.1(+)Zeo IgK/hRGMA 47-168/Xa/hIgG lambda hc 257-Stop (no att)**

### 316-386

- AM 181:: GGGGAAGCTTCTGCGGGGCTGCCCCC (SEQ ID NO: 22) hRGMA sense Primer ab Aminosäure **L316** mit HindIII Schnitt
- AM 182:: CCCCGAATTCAAGCCCGTGGTGAGGAGGTCG (SEQ ID NO: 23) hRGMA antisense Primer bis Aminosäure **G386** mit EcoRI Schnittstelle

Transformation in TOP10 Zellen,
Plasmidname : **pcDNA3.1(+)Zeo IgK/hRGMA 166-386/Xa/hIgG lambda hc 257-Stop (no att)**

### 1-450

- Mey 744 :: ***ATG***CAGCCGCCAAGGGAGAG (SEQ ID NO: 24) hRGMA Primer mit Start **ATG**
- Mey 745 :: ***GCA***GAACACAGGGAGCAGGGC (SEQ ID NO: 25) hRGMA antisense Primer bis Aminosäure **C*450*** ohne Stop

Zu Vergleichszwecken wurden in analoger Weise die folgenden Fc-Fusionsproteine mit weiteren RGMA-Fragmenten hergestellt, Deren Herstellung ist aber nicht gesondert beschrieben wird:
- 266-284
- 70-120
- 110-169
- 169-422
- 266-335
- 47-422 Myc His

HEK293F-Zellen (DSMZ Nr. ACC 305) wurden mit den Plasmiden transfiziert und das exprimierte Fusionsprotein wurde wie oben beschrieben isoliert.

Die Analyse der Proteine und die Konzentrationsbestimmung erfolgte mit Hilfe der oben angegebenen Methoden.

### 3. Ausführungsbeispiele

### Ausführungsbeispiel 1: In vitro Interaktionsassay mit BMP-4; Vergleich verschiedener RGMA-Fragmente mit RGMA

Getestete Fusionsproteine:
RGMA-Fc
47-168-Fc ("Fragment 0")
218-284-Fc ("Fragment 2")
266-335-Fc ("Fragment 3")
169-422-Fc ("Fragment 6")

Die Testdurchführung erfolgte nach obiger Testmethode 4, Variante B, wobei die Fusionsproteine (1mg/ml) immobilisiert wurden. Die Bindung vom BMP-4 wurde mittels anti-BMP-4- Biotin Antikörpern nachgewiesen.

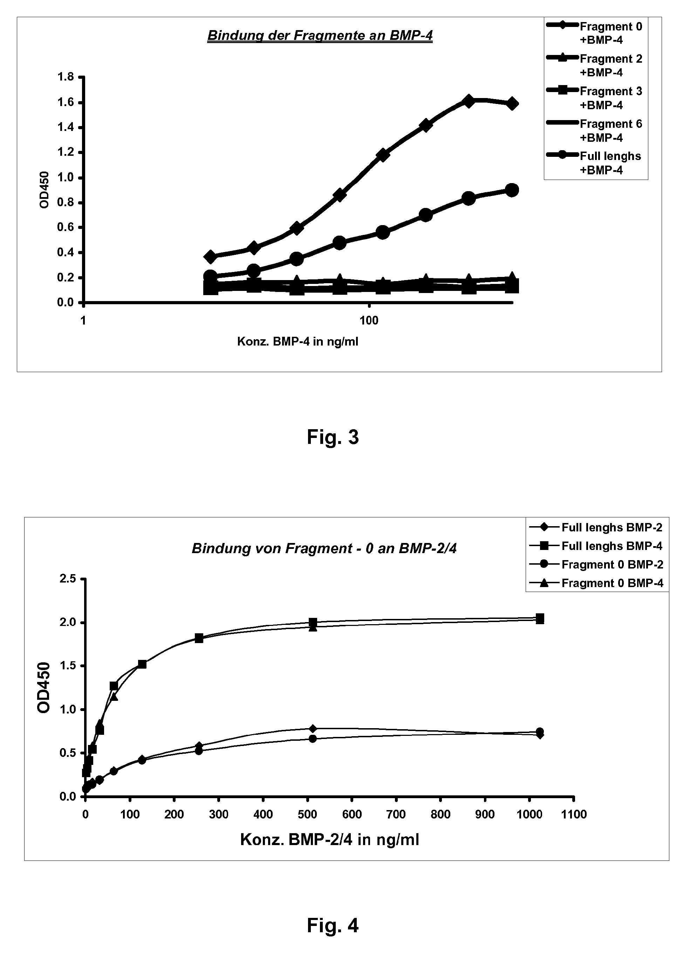

Die Ergebnisse sind in Figur 3 grafisch dargestellt. Man beobachtet eine signifikante Binding von BMP-4 mit RGMA und eine überraschend signifikantere Bindung mit dem 47-168 Fragment.

### Ausführungsbeispiel 2: In vitro Interaktionsassay mit BMP-4 und BMP-2; Vergleich des RGMA-Fragments 47-168 mit RGMA

Getestete Fusionsproteine:
RGMA-Fc
47-168-Fc ("Fragment 0")

Die Testdurchführung erfolgte nach obiger Testmethode 4, Variante B, wobei die Fusionsproteine (1mg/ml) immobilisiert wurden. Die Bindung vom BMP-4 und -2 wurde mittels anti-BMP-4 bzw. anti-BMP-2-Biotin Antikörpern nachgewiesen.

Die Ergebnisse sind in Figur 4 grafisch dargestellt. Man beobachtet eine signifikante Binding von BMP-4 und 2 mit RGMA und eine überraschend signifikantere Bindung mit dem 47-168 Fragment. BMP-2 und 4 werden jeweils in etwa gleich stark gebunden.

### Ausführungsbeispiel 3: In vitro Interaktionsassay mit BMP-4; Vergleich verschiedener RGMA-Fragmente

Getestete Fusionsproteine:
47-90-Fc (#785)
47-168-Fc (#786)
316-386-Fc (#790)
169-422-Fc (#769)
70-120-Fc (#779)
110-169-Fc (#780)
266-335-Fc (#789)
47-422Myc-HIS (#801)

Die Testdurchführung erfolgte nach obiger Testmethode 4, Variante A, wobei BMP-4 (1mg/ml) immobilisiert wurden. Die Bindung der Fusionsproteine wurde mittels anti-human-Fc bzw. anti-Myc-anti-Rabbit (Invitrogen) Antikörpern nachgewiesen.

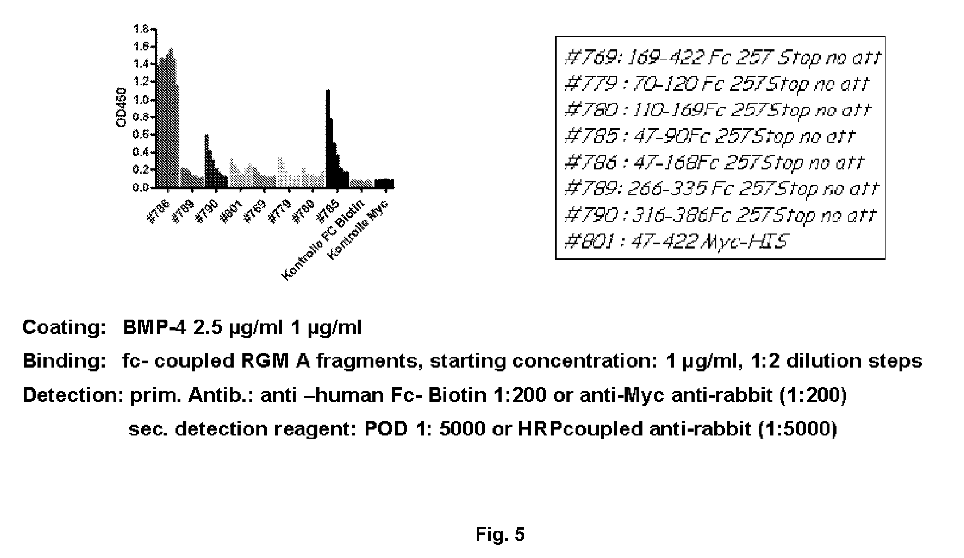

Die Ergebnisse sind in beiliegender Figur 5 dargestellt.

Man beobachtet eine signifikante, konzentrationsabhängige Bindung der erfindungsgemäßen Fragmente #785, #786 und #790, wobei #786 am stärksten bindet.

### Ausführungsbeispiel 4: In vitro Interaktionsassay mit BMP-4; Vergleich verschiedener bindender RGMA-Fragmente

Getestete Fusionsproteine:
47-90-Fc (#785)
47-168-Fc (#786)
316-386-Fc (#790)

Die Testdurchführung erfolgte nach obiger Testmethode 4, Variante A, wobei BMP-4 (1mg/ml) immobilisiert wurden. Die Bindung der Fusionsproteine wurde mittels anti-human-Fc Antikörpern nachgewiesen.

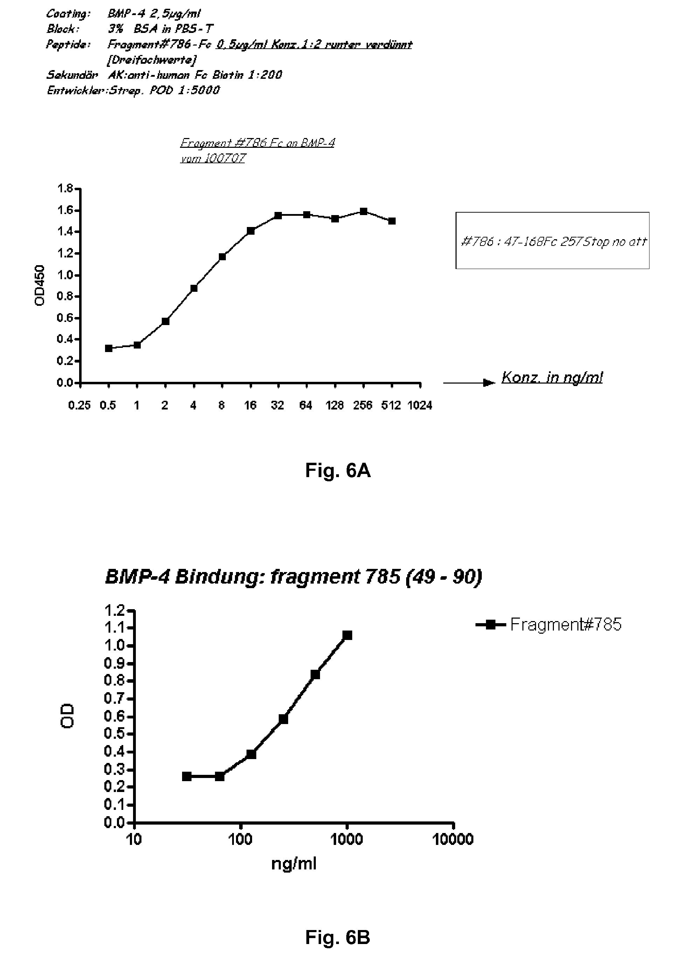

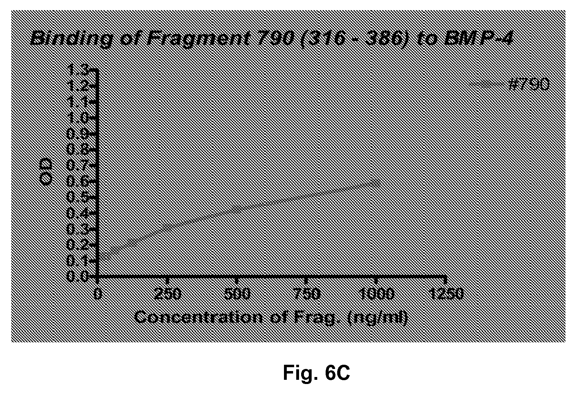

Die Ergebnisse sind in den Figuren 6A, B und C dargestellt. Die Ergebnisse bestätigen die Befunde Ausführungsbeispiel 3.

### Ausführungsbeispiel 5: Untersuchung synthetischer RGM A-Fragmenten im Nervenfaserwachstumstest

Die anschließend aufgelisteten RGMA Fragmente,
47-168-Fc (#786)
316-386-Fc (#790)

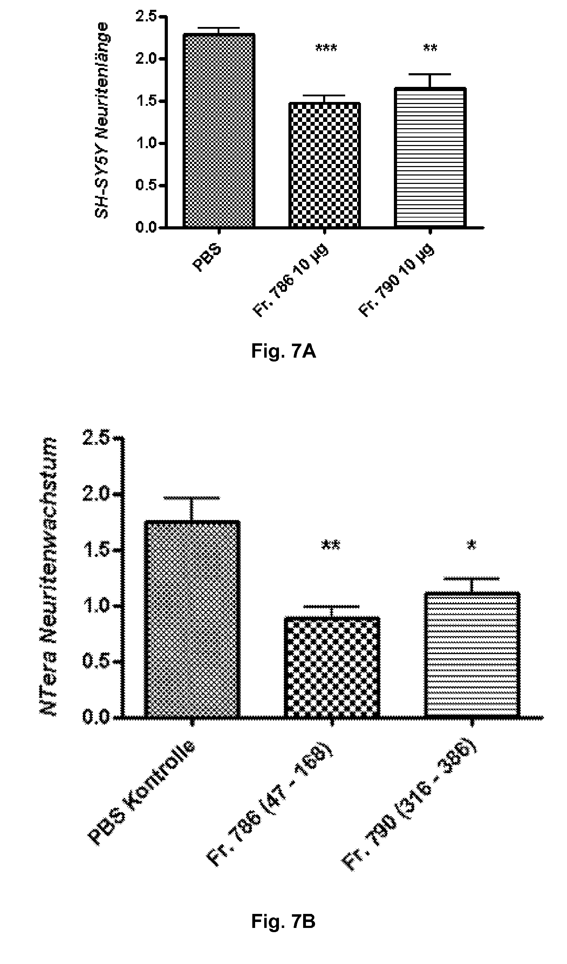

hergestellt gemäß Herstellungsbeispiel 1, wurden im Neuritenwachstumstest (vgl. obige Testmethoden 1 und 2) mit humanen NTera Nervenzellen oder mit humanen SH-SY5Y Zellen auf inhibitorische Aktivität getestet. Die Ergebnisse sind in den Figuren 7A (für SH-SY5Y) und 7B (für Ntera) dargestellt.

Dabei zeigt 47-168-Fc (#786) eine signifikant höhere Aktivität.

Auf die Offenbarung der in vorliegender Beschreibung zitierten Druckschriften wird ausdrücklich Bezug genommen.

## Patentansprüche

1. Bone Morphogenetic Protein (BMP)-bindende Domänen des Repulsive Guidance Molecule (RGM).

2. BMP-bindende Domänen nach Anspruch 1, abgeleitet von RGM von Säugern.

3. BMP-bindende Domänen nach Anspruch 1 oder 2, abgeleitet von einem humanen RGM A gemäß SEQ ID NO: 2, humanen RGM B gemäß SEQ ID NO:4 oder humanen RGM C gemäß SEQ ID NO: 6.

4. BMP-bindende Domänen nach einem der vorhergehenden Ansprüche, lokalisiert in einem Aminosäuresequenzbereich mit einer Länge von bis zu etwa 170 N-terminal zur von-Willebrand-Faktor-Domäne von RGM A.

5. BMP-bindende Domänen nach Anspruch 4, mit einer Länge von etwa 30 bis 150 Aminosäureresten, funktionale Derivate davon und Fusionsproteine, enthaltend wenigstens eine BMP-bindende Domäne in funktionaler Verknüpfung mit wenigstens einer weiteren, davon verschiedenen, Aminosäuresequenz.

6. BMP-bindende Domänen nach einem der vorhergehenden Ansprüche, **gekennzeichnet durch** wenigstens eine der folgenden Teilsequenzen gemäß SEQ ID NO: 7 und 8:
X₁C(K/R)IX₂(K/R)CX₃(S/T/A)(E/D)(FN)X₄SX₅T (SEQ ID NO:7)
worin X₁ bis X₅ beliebige Aminosäurereste bedeuten; oder
X₆CX₇ALRX₈YAX₉CTX₁₀RTX₁₁ (SEO ID NO:8)
worin X₆ bis X₁₁ beliebige Aminosäurereste bedeuten;
oder eine Teilsequenz der Formel
(SEQ ID NO:7) -Link1- (SEQ ID NO:8)
worin Link1 für eine SEQ ID NO: 7 und 8 verbrückende Aminosäuresequenz steht, umfassend 13 bis 28 zusammenhängende beliebige Aminosäurereste.

7. BMP-bindende Domänen nach einem der vorhergehenden Ansprüche, umfassend eine der folgenden Aminosäuresequenzen von SEQ ID NO:2:
Aminosäureposition von etwa 47 bis etwa168
Aminosäureposition von etwa 47 bis etwa 90 oder
Aminosäureposition von etwa 75 bis etwa 121;
oder eine der folgenden Aminosäuresequenzen von SEQ ID NO:4:
Aminosäureposition von etwa 94 bis etwa 209
Aminosäureposition von etwa 94 bis etwa 137 oder
Aminosäureposition von etwa 122 bis etwa 168;
eine der folgenden Aminosäuresequenzen von SEQ ID NO:6:
Aminosäureposition von etwa 36 bis etwa172
Aminosäureposition von etwa 36 bis etwa 94 oder
Aminosäureposition von etwa 80 bis etwa 125;
oder ein funktionales, BMP-bindendes Fragment davon.

8. BMP-bindende Domänen oder bindende Fragmente davon nach einem der Ansprüche 1 bis 3, welche wenigstens 10 zusammenhängende Aminosäurereste aus dem Sequenzbereich etwa von Position 316 bis etwa 386 gemäß SEQ ID NO: 2, aus dem Sequenzbereich etwa von Position 350 bis etwa 421 gemäß SEQ ID NO: 4 oder aus dem Sequenzbereich etwa von Position 314 bis 369 gemäß SEQ ID NO: 6 umfassen.

9. BMP-bindende Domänen oder bindende Fragmente davon nach Anspruch 7, welche wenigstens 10 zusammenhängende Aminosäurereste aus dem Sequenzbereich etwa von Position 47 bis etwa 168 gemäß SEQ ID NO: 2, aus dem Sequenzbereich etwa von Position 94 bis etwa 209 gemäß SEQ ID NO: 4 oder aus dem Sequenzbereich etwa von Position 36 bis 172 gemäß SEQ ID NO: 6 umfassen.

10. BMP-bindende Domäne nach einem der vorhergehenden Ansprüche, welche an wenigstens ein BMP ausgewählt unter BMP-2, BMP-4, BMP-5, BMP-6 und BMP-12, insbesondere an BMP-2 und/oder BMP-4 bindet.

11. BMP-bindende Domäne nach Anspruch 10, welche auch an Neogenin bindet.

12. Antigenes Polypeptid-Fragment einer BMP-bindenden Domäne nach einem der vorhergehenden Ansprüche.

13. Antigenes Polypeptid-Fragment nach Anspruch 12, welches zur Herstellung von Immunglobulin-Molekülen brauchbar ist, welche die Bindung von RGM an BMP und/oder Neogenin modulieren.

14. Antigenes Polypeptid-Fragment nach Anspruch 12 oder 13, welches wenigstens 10 zusammenhängende Aminosäurereste eines Peptids mit einer der Sequenzen gemäß SEQ ID NO: 2, 4 oder 6 umfasst.

15. Fusionsprotein umfassend wenigstens ein erstes biologisch aktives Polypeptid, ausgewählt unter BMP-bindenden Domänen nach einem der Ansprüche 1 bis 11, operativ verknüpft mit einem zweiten Polypeptid, ausgewählt unter einem mono-oder polyvalenten Trägerpolypeptid oder einem zweiten biologisch aktiven Polypeptid.

16. Fusionsprotein nach Anspruch 15, wobei der polyvalente Träger wenigstens ein Fc oder Fc" umfasst, wobei jede der beiden Polypetidketten davon mit gleichen oder verschiedenen BMP-bindenden Domänen nach einem der Ansprüche 1 bis 10 operativ verknüpft ist.

17. Verwendung einer BMP-bindenden Domäne nach einem der Ansprüche 1 bis 11 oder eines Polypeptid-Fragments nach einem der Ansprüche 12 bis 14 zur Herstellung eines polyklonalen Antiserums oder eines monoklonalen Antikörpers gegen RGM.

18. Verwendung nach Anspruch 17, wobei das Antiserum oder der Antikörper die Bindung von RGM an Neogenin (den Neogenin-Rezeptor) moduliert.

19. Polyklonales Antiserum oder monoklonaler Antikörper gegen RGM gemäß der Definition in einem der Ansprüche 17 und 18 zur Verwendung in der Diagnose oder Therapie.

20. Verwendung eines polyklonalen Antiserums oder eines monoklonalen Antikörpers nach Anspruch 19 zur Herstellung eines pharmazeutischen Mittels zur Diagnose oder Therapie von Krankheiten oder Krankheitsstadien welche durch eine Wechselwirkung des Neogenin-Rezeptors (Neogenins) mit RGM oder einem RGM-Fragment mediiert werden.

21. Verwendung nach Anspruch 20, wobei die Krankheiten oder Krankheitsstadien ausgewählt sind unter
a) mechanischen Verletzungen von Schädel, Gehirn und Rückenmark,
b) chronische Erkrankungen, ausgewählt unter neurodegenerativen, inflammatorischen oder Autoimmun-Erkrankungen,
c) Störungen der neuronalen Regeneration, des axonalen Sproutings, der Neuritenextension und der neuronalen Plastizität,
d) Tumorerkrankungen und Tumormetastasierung.

22. Verwendung einer BMP-bindenden Domäne nach einem der Ansprüche 1 bis 11 oder eines Fusionsproteins nach einem der Ansprüche 15 und 16 zur Herstellung eines Mittels zur Diagnose oder Therapie von Krankheiten oder Krankheitsstadien welche durch eine gestörte oder beeinträchtigte Wechselwirkung von RGM oder einem RGM-Fragment mit dem zugeordneten Rezeptor (Neogenin oder BMP) mediiert werden.

23. Verwendung nach Anspruch 22, wobei die Krankheiten oder Krankheitsstadien ausgewählt sind unter
a) veränderten Neuritogeneseprozessen bei psychotischen Erkrankungen und chronischen Schmerzzuständen die durch exzessive Neuritensprossung und/oder pathologische Synaptogenese hervorgerufen werden.
b) Erkrankungen, assoziiert mit einem gestörten Eisenstoffwechsel;
c) Erkrankungen, assoziiert mit einem gestörten Knochenwachstum
d) Erkrankungen, assoziiert mit degenerativen Knorpelveränderungen
e) Erkrankungen, assoziiert mit Bandscheiben - und Wirbelkörperschäden
f) Erkrankungen, assoziiert mit deregulierten, unkontrollierten Zellmigrationsvorgängen.

24. Verwendung einer BMP-bindenden Domäne oder eines bindenden Fragments davon nach einem der Ansprüche 1 bis 11 oder eines Fusionsproteins nach einem der Ansprüche 15 und 16 zur Herstellung eines Mittels zur Diagnose oder Therapie von Krankheiten oder Krankheitsstadien welche durch Stimulation oder Verstärkung des BMP-Signalwegs (durch Bindung an BMP-2, BMP-4, BMP-5, BMP-6, und/oder BMP-12, insbesondere an BMP-2 und /oder 4) behandelbar sind.

25. Verwendung nach Anspruch 24, zur Behandlung von Erkrankungen mit gestörtem Knochenwachstum oder zur Behandlung von Knochenfrakturen.

26. Verwendung einer BMP-bindenden Domäne oder eines bindenden Fragments davon nach einem der Ansprüche 1 bis 11 oder eines Fusionsproteins nach einem der Ansprüche 15 und 16 zur Herstellung eines Mittels zur Diagnose oder Therapie von Autoimmunerkrankungen, insbesondere ausgewählt unter Spondylitis ankylosans, Antiphospholipid syndrome, Addison Krankheit, autoimmuner hämolytischer Anämie, Autoimmun-Hepatitis, autoimmunes lymphoproliferatives Syndrom (ALPS), autoimmunes thrombocytopenische Purpura (ATP), Behcet Krankheit, bullöses Pemphigoid, Kardiomyopathie, Zöliakie, Dermatitis herpetiformis, Chronic Fatigue Immune Dysfunction Syndrom (CFIDS), Chronic Inflammatory Demyelinating Polyneuropathie (CIDP), vernarbendes Pemphigoid, systemische Sclerose (CREST Syndrom), Kälteagglutinationskrankheit, Crohn's Krankheit, cutane Vasculitis, Degos Krankheit, Dermatomyositis, juvenile Dermatomyositis, Lupus erythematosus discoides, essentielle gemischte Kryoglubulinämie, Fibromyalgie, Goodpasture Syndrom, Graves Krankheit, Guillain-Barre Syndrom, Hashimoto Thyroiditis, idiopathische pulmonäre Fibrose, idiopathische Thrombocytopenia purpura (ITP), Immunoglobulin A Nephropathie, Insulinabhängige Diabetes mellitus, juvenile Arthritis, Kawasaki Krankheit, Lichen planus, membranöse Glomerulonephritis, Meniere Krankheit, Bindegewebskrankheit vom gemischten Typus, multifocale motorische Neuropathy, Multiple Sklerose, Myasthenia gravis, Pemphigus vulgaris, perniciöse Anämie, Polyarteritis nodosa, Polychondritis, Polyglanduläres Syndrom, Polymyalgia rheumatica, Polymyositis, Dermatomyositis, primäre Agammaglobulinemia, primäre Cirrhosis biliaris, Psoriasis, Raynaud Phänomen, Reiter Syndrome, rheumatisches Fieber, rheumatoide Arthritis, Sarcoidose, Scleroderma, Sjogren Syndrom, Stiff-Man Syndrom, systemischer Lupus erythematosus, Takayasu Arthritis, temporäre Arthritis/Riesenzellenartritis, ulcerative Colitis, Uveitis, Vasculitis, Vitiligo, und Wegener Granulomatose; oder
Haarausfallerkrankungen, insbesondere ausgewählt unter Alopecia areata, Alopecia totalis, Alopecia universalis, androgener Alopecie, telogenem Effluvium, anagenem Effluvium, und Chemotherapie-induzierter Alopecie.

27. Verwendung einer BMP-bindenden Domäne nach einem der Ansprüche 1 bis 11 als Target zum Nachweis oder zur Identifizierung von RGM-bindenden Liganden.

28. Verwendung einer BMP-bindenden Domäne nach einem der Ansprüche 1 bis 11 oder eines Fragments nach einem der Ansprüche 12 bis 14 als Immunogen zur aktiven oder passiven Immunisierung.

29. Polyklonales Antiserum, erhältlich durch Immunisierung eines Säugers mit einer antigenen Menge einer BMP-bindenden Domäne nach einem der Ansprüche 1 bis 11 oder eines Polypeptid-Fragments nach einem der Ansprüche 12 bis 14.

30. Monoklonaler Antikörper gegen eine BMP-bindende Domäne nach einem der Ansprüche 1 bis 11 oder gegen ein Polypeptid-Fragment nach einem der Ansprüche 12 bis 14, oder ein Antigen-bindendes Fragment davon, gegebenenfalls in humanisierter Form.

31. Pharmazeutisches Mittel, umfassend in einem pharmazeutisch verträglichen Träger wenigstens einen aktiven Bestandteil ausgewählt unter:
a) einer BMP-bindenden Domäne nach einem der Ansprüche 1 bis 11 oder einem Polypeptid-Fragment nach einem der Ansprüche 12 bis 14 oder ein Fusionsprotein nach einem der Ansprüche 15 und 16,
b) monoklonale oder polyklonale Antikörpern nach einem der Ansprüche 26 und 27.

32. Pharmazeutisches Mittel nach Anspruch 31, zur intrathecalen, intravenösen, subkutanen, oralen oder parenteralen, perkutanen, subdermalen, intraossealen, nasalen, extrakorporalen und Inhalations-Verabreichung.

33. Pharmazeutisches Mittel, zur Behandlung von Knochenfrakturen, umfassend wenigstens eine BMP-bindende Domäne nach einem der Ansprüche 1 bis 11 oder ein Fusionsprotein nach einem der Ansprüche 15 und 16, in einem flüssigen, halbfesten, oder festen Träger.

34. Expressionsvektor, umfassend wenigstens eine kodierende Nukleinsäuresequenz für eine BMP-bindende Domäne nach einem der Ansprüche 1 bis 11, ein Fusionsprotein nach einem der Ansprüche 15 und 16 oder ein Polypeptid-Fragment nach einem der Ansprüche 12 bis 14, operativ verknüpft mit wenigstens einer regulativen Nukleinsäuresequenz.

35. Rekombinanter Mikroorganismus, welcher wenigstes einen Vektor nach Anspruch 34 trägt.

36. Hybridomzelllinie, welche einen monoklonalen Antikörper nach Anspruch 30 produziert.

37. Verfahren zur Herstellung einer BMP-bindenden Domäne nach einem der Ansprüche 1 bis 11, eines Fusionsproteins nach einem der Ansprüche 15 und 16 oder eines Polypeptid-Fragment nach einem der Ansprüche 12 bis 14, wobei man einen rekombinanten Miroorganismus nach Anspruch 35 kultiviert und das produzierte Proteinprodukt aus der Kultur isoliert.

38. Verfahren zur Herstellung eines monoklonalen Antikörpers nach Anspruch 30, wobei man eine Hybridomzelllinie nach Anspruch 36 kultiviert und das produzierte Proteinprodukt aus der Kultur isoliert.

39. Verwendung einer BMP-bindenden Domäne nach einem der Ansprüche 1 bis 16 oder eines Fusionsproteins nach einem der Ansprüche 15 und 16 zur Herstellung eines pharmazeutischen Mittels zur Stimulierung eines RGM-Rezeptors, insbesondere von Neogenin oder wenigstens eines BMP insbesondere ausgewählt unter BMP-2, BMP-4, BMP-5, BMP-6 und BMP-12.

40. Verwendung eines monoklonalen Antikörpers nach Anspruch 30, zur Herstellung eines pharmazeutischen Mittels zur Blockierung der Aktivierung eines RGM-Rezeptors, wie insbesondere Neogenin.


SEQUENCE LISTING

<110>  Abbott GmbH & Co. KG

<120>  RGM Bindungsdomänen

<130>  M/48272

<160>  27

<170>  PatentIn version 3.3

<210>  1
<211>  1353
<212>  DNA
<213>  Homo sapiens


<220>
<221>  CDS
<222>  (1)..(1353)

<400>  1
atg cag ccg cca agg gag agg cta gtg gta aca ggc cga gct gga tgg      48
Met Gln Pro Pro Arg Glu Arg Leu Val Val Thr Gly Arg Ala Gly Trp
1               5                   10                  15

atg ggt atg ggg aga ggg gca gga cgt tca gcc ctg gga ttc tgg ccg      96
Met Gly Met Gly Arg Gly Ala Gly Arg Ser Ala Leu Gly Phe Trp Pro
            20                  25                  30

acc ctc gcc ttc ctt ctc tgc agc ttc ccc gca gcc acc tcc ccg tgc      144
Thr Leu Ala Phe Leu Leu Cys Ser Phe Pro Ala Ala Thr Ser Pro Cys
        35                  40                  45

aag atc ctc aag tgc aac tct gag ttc tgg agc gcc acg tcg ggc agc      192
Lys Ile Leu Lys Cys Asn Ser Glu Phe Trp Ser Ala Thr Ser Gly Ser
    50                  55                  60

cac gcc cca gcc tca gac gac acc ccc gag ttc tgt gca gcc ttg cgc      240
His Ala Pro Ala Ser Asp Asp Thr Pro Glu Phe Cys Ala Ala Leu Arg
65                  70                  75                  80

agc tac gcc ctg tgc acg cgg cgg acg gcc cgc acc tgc cgg ggt gac      288
Ser Tyr Ala Leu Cys Thr Arg Arg Thr Ala Arg Thr Cys Arg Gly Asp
                85                  90                  95

ctg gcc tac cac tcg gcc gtc cat ggc ata gag gac ctc atg agc cag      336
Leu Ala Tyr His Ser Ala Val His Gly Ile Glu Asp Leu Met Ser Gln
            100                 105                 110

cac aac tgc tcc aag gat ggc ccc acc tcg cag cca cgc ctg cgc acg      384
His Asn Cys Ser Lys Asp Gly Pro Thr Ser Gln Pro Arg Leu Arg Thr
        115                 120                 125

ctc cca ccg gcc gga gac agc cag gag cgc tcg gac agc ccc gag atc      432
Leu Pro Pro Ala Gly Asp Ser Gln Glu Arg Ser Asp Ser Pro Glu Ile
    130                 135                 140

tgc cat tac gag aag agc ttt cac aag cac tcg gcc acc ccc aac tac      480
Cys His Tyr Glu Lys Ser Phe His Lys His Ser Ala Thr Pro Asn Tyr
145                 150                 155                 160

acg cac tgt ggc ctc ttc ggg gac cca cac ctc agg act ttc acc gac      528
Thr His Cys Gly Leu Phe Gly Asp Pro His Leu Arg Thr Phe Thr Asp
                165                 170                 175

cgc ttc cag acc tgc aag gtg cag ggc gcc tgg ccg ctc atc gac aat      576
Arg Phe Gln Thr Cys Lys Val Gln Gly Ala Trp Pro Leu Ile Asp Asn

            180                 185                 190

aat tac ctg aac gtg cag gcc acc aac acg cct gtg ctg ccc ggc tca 624
Asn Tyr Leu Asn Val Gln Ala Thr Asn Thr Pro Val Leu Pro Gly Ser
        195                 200                 205

gcg gcc act gcc acc agc aag ctc acc atc atc ttc aag aac ttc cag 672
Ala Ala Thr Ala Thr Ser Lys Leu Thr Ile Ile Phe Lys Asn Phe Gln
    210                 215                 220

gag tgt gtg gac cag aag gtg tac cag gct gag atg gac gag ctc ccg 720
Glu Cys Val Asp Gln Lys Val Tyr Gln Ala Glu Met Asp Glu Leu Pro
225                 230                 235                 240

gcc gcc ttc gtg gat ggc tct aag aac ggt ggg gac aag cac ggg gcc 768
Ala Ala Phe Val Asp Gly Ser Lys Asn Gly Gly Asp Lys His Gly Ala
                245                 250                 255

aac agc ctg aag atc act gag aag gtg tca ggc cag cac gtg gag atc 816
Asn Ser Leu Lys Ile Thr Glu Lys Val Ser Gly Gln His Val Glu Ile
            260                 265                 270

cag gcc aag tac atc ggc acc acc atc gtg gtg cgc cag gtg ggc cgc 864
Gln Ala Lys Tyr Ile Gly Thr Thr Ile Val Val Arg Gln Val Gly Arg
        275                 280                 285

tac ctg acc ttt gcc gtc cgc atg cca gag gaa gtg gtc aat gct gtg 912
Tyr Leu Thr Phe Ala Val Arg Met Pro Glu Glu Val Val Asn Ala Val
    290                 295                 300

gag gac tgg gac agc cag ggt ctc tac ctc tgc ctg cgg ggc tgc ccc 960
Glu Asp Trp Asp Ser Gln Gly Leu Tyr Leu Cys Leu Arg Gly Cys Pro
305                 310                 315                 320

ctc aac cag cag atc gac ttc cag gcc ttc cac acc aat gct gag ggc 1008
Leu Asn Gln Gln Ile Asp Phe Gln Ala Phe His Thr Asn Ala Glu Gly
                325                 330                 335

acc ggt gcc cgc agg ctg gca gcc gcc agc cct gca ccc aca gcc ccc 1056
Thr Gly Ala Arg Arg Leu Ala Ala Ala Ser Pro Ala Pro Thr Ala Pro
            340                 345                 350

gag acc ttc cca tac gag aca gcc gtg gcc aag tgc aag gag aag ctg 1104
Glu Thr Phe Pro Tyr Glu Thr Ala Val Ala Lys Cys Lys Glu Lys Leu
        355                 360                 365

ccg gtg gag gac ctg tac tac cag gcc tgc gtc ttc gac ctc ctc acc 1152
Pro Val Glu Asp Leu Tyr Tyr Gln Ala Cys Val Phe Asp Leu Leu Thr
    370                 375                 380

acg ggc gac gtg aac ttc aca ctg gcc gcc tac tac gcg ttg gag gat 1200
Thr Gly Asp Val Asn Phe Thr Leu Ala Ala Tyr Tyr Ala Leu Glu Asp
385                 390                 395                 400

gtc aag atg ctc cac tcc aac aaa gac aaa ctg cac ctg tat gag agg 1248
Val Lys Met Leu His Ser Asn Lys Asp Lys Leu His Leu Tyr Glu Arg
                405                 410                 415

act cgg gac ctg cca ggc agg gcg gct gcg ggg ctg ccc ctg gcc ccc 1296
Thr Arg Asp Leu Pro Gly Arg Ala Ala Ala Gly Leu Pro Leu Ala Pro
            420                 425                 430

cgg ccc ctc ctg ggc gcc ctc gtc ccg ctc ctg gcc ctg ctc cct gtg 1344
Arg Pro Leu Leu Gly Ala Leu Val Pro Leu Leu Ala Leu Leu Pro Val
        435                 440                 445

ttc tgc tag 1353
Phe Cys
    450

<210>  2
<211>  450
<212>  PRT
<213>  Homo sapiens

<400>  2

Met Gln Pro Pro Arg Glu Arg Leu Val Val Thr Gly Arg Ala Gly Trp
1               5                   10                  15

Met Gly Met Gly Arg Gly Ala Gly Arg Ser Ala Leu Gly Phe Trp Pro
            20                  25                  30

Thr Leu Ala Phe Leu Leu Cys Ser Phe Pro Ala Ala Thr Ser Pro Cys
        35                  40                  45

Lys Ile Leu Lys Cys Asn Ser Glu Phe Trp Ser Ala Thr Ser Gly Ser
    50                  55                  60

His Ala Pro Ala Ser Asp Asp Thr Pro Glu Phe Cys Ala Ala Leu Arg
65                  70                  75                  80

Ser Tyr Ala Leu Cys Thr Arg Arg Thr Ala Arg Thr Cys Arg Gly Asp
                85                  90                  95

Leu Ala Tyr His Ser Ala Val His Gly Ile Glu Asp Leu Met Ser Gln
            100                 105                 110

His Asn Cys Ser Lys Asp Gly Pro Thr Ser Gln Pro Arg Leu Arg Thr
        115                 120                 125

Leu Pro Pro Ala Gly Asp Ser Gln Glu Arg Ser Asp Ser Pro Glu Ile
    130                 135                 140

Cys His Tyr Glu Lys Ser Phe His Lys His Ser Ala Thr Pro Asn Tyr
145                 150                 155                 160

Thr His Cys Gly Leu Phe Gly Asp Pro His Leu Arg Thr Phe Thr Asp
                165                 170                 175

Arg Phe Gln Thr Cys Lys Val Gln Gly Ala Trp Pro Leu Ile Asp Asn
            180                 185                 190

Asn Tyr Leu Asn Val Gln Ala Thr Asn Thr Pro Val Leu Pro Gly Ser
        195                 200                 205

Ala Ala Thr Ala Thr Ser Lys Leu Thr Ile Ile Phe Lys Asn Phe Gln
    210                 215                 220

Glu Cys Val Asp Gln Lys Val Tyr Gln Ala Glu Met Asp Glu Leu Pro
225                 230                 235                 240

Ala Ala Phe Val Asp Gly Ser Lys Asn Gly Gly Asp Lys His Gly Ala
                245                 250                 255

Asn Ser Leu Lys Ile Thr Glu Lys Val Ser Gly Gln His Val Glu Ile
            260                 265                 270

Gln Ala Lys Tyr Ile Gly Thr Thr Ile Val Val Arg Gln Val Gly Arg
        275                 280                 285

Tyr Leu Thr Phe Ala Val Arg Met Pro Glu Glu Val Val Asn Ala Val
    290                 295                 300

Glu Asp Trp Asp Ser Gln Gly Leu Tyr Leu Cys Leu Arg Gly Cys Pro
305                 310                 315                 320

Leu Asn Gln Gln Ile Asp Phe Gln Ala Phe His Thr Asn Ala Glu Gly
                325                 330                 335

Thr Gly Ala Arg Arg Leu Ala Ala Ala Ser Pro Ala Pro Thr Ala Pro
            340                 345                 350

Glu Thr Phe Pro Tyr Glu Thr Ala Val Ala Lys Cys Lys Glu Lys Leu
        355                 360                 365

Pro Val Glu Asp Leu Tyr Tyr Gln Ala Cys Val Phe Asp Leu Leu Thr
    370                 375                 380

Thr Gly Asp Val Asn Phe Thr Leu Ala Ala Tyr Tyr Ala Leu Glu Asp
385                 390                 395                 400

Val Lys Met Leu His Ser Asn Lys Asp Lys Leu His Leu Tyr Glu Arg
                405                 410                 415

Thr Arg Asp Leu Pro Gly Arg Ala Ala Ala Gly Leu Pro Leu Ala Pro
            420                 425                 430

Arg Pro Leu Leu Gly Ala Leu Val Pro Leu Leu Ala Leu Leu Pro Val
        435                 440                 445

Phe Cys
    450

<210>  3
<211>  1437
<212>  DNA
<213>  Homo sapiens

<220>
<221>  CDS
<222>  (1)..(1437)

<400>  3
atg ata agg aag aag agg aag cga agc gcg ccc ccc ggc cca tgc cgc          48
Met Ile Arg Lys Lys Arg Lys Arg Ser Ala Pro Pro Gly Pro Cys Arg

1                   5                   10                  15

agc cac ggg ccc aga ccc gcc acg gcg ccc gcg ccg ccg ccc tcg ccg        96
Ser His Gly Pro Arg Pro Ala Thr Ala Pro Ala Pro Pro Pro Ser Pro
            20                  25                  30

gag ccc acg aga cct gca tgg acg ggc atg ggc ttg aga gca gca cct       144
Glu Pro Thr Arg Pro Ala Trp Thr Gly Met Gly Leu Arg Ala Ala Pro
        35                  40                  45

tcc agc gcc gcc gct gcc gcc gcc gag gtt gag cag cgc cgc cgc ccc       192
Ser Ser Ala Ala Ala Ala Ala Ala Glu Val Glu Gln Arg Arg Arg Pro
    50                  55                  60

ggg ctc tgc ccc ccg ccg ctg gag ctg ctg ctg ctg ctg ctg ttc agc       240
Gly Leu Cys Pro Pro Pro Leu Glu Leu Leu Leu Leu Leu Leu Phe Ser
65                  70                  75                  80

ctc ggg ctg ctc cac gca ggt gac tgc caa cag cca gcc caa tgt cga       288
Leu Gly Leu Leu His Ala Gly Asp Cys Gln Gln Pro Ala Gln Cys Arg
                85                  90                  95

atc cag aaa tgc acc acg gac ttc gtg tcc ctg act tct cac ctg aac       336
Ile Gln Lys Cys Thr Thr Asp Phe Val Ser Leu Thr Ser His Leu Asn
            100                 105                 110

tct gcc gtt gac ggc ttt gac tct gag ttt tgc aag gcc ttg cgt gcc       384
Ser Ala Val Asp Gly Phe Asp Ser Glu Phe Cys Lys Ala Leu Arg Ala
        115                 120                 125

tat gct ggc tgc acc cag cga act tca aaa gcc tgc cgt ggc aac ctg       432
Tyr Ala Gly Cys Thr Gln Arg Thr Ser Lys Ala Cys Arg Gly Asn Leu
    130                 135                 140

gta tac cat tct gcc gtg ttg ggt atc agt gac ctc atg agc cag agg       480
Val Tyr His Ser Ala Val Leu Gly Ile Ser Asp Leu Met Ser Gln Arg
145                 150                 155                 160

aat tgt tcc aag gat gga ccc aca tcc tct acc aac ccc gaa gtg acc       528
Asn Cys Ser Lys Asp Gly Pro Thr Ser Ser Thr Asn Pro Glu Val Thr
                165                 170                 175

cat gat cct tgc aac tat cac agc cac gct gga gcc agg gaa cac agg       576
His Asp Pro Cys Asn Tyr His Ser His Ala Gly Ala Arg Glu His Arg
            180                 185                 190

aga ggg gac cag aac cct ccc agt tac ctt ttt tgt ggc ttg ttt gga       624
Arg Gly Asp Gln Asn Pro Pro Ser Tyr Leu Phe Cys Gly Leu Phe Gly
        195                 200                 205

gat cct cac ctc aga act ttc aag gat aac ttc caa aca tgc aaa gta       672
Asp Pro His Leu Arg Thr Phe Lys Asp Asn Phe Gln Thr Cys Lys Val
    210                 215                 220

gaa ggg gcc tgg cca ctc ata gat aat aat tat ctt tca gtt caa gtg       720
Glu Gly Ala Trp Pro Leu Ile Asp Asn Asn Tyr Leu Ser Val Gln Val
225                 230                 235                 240

aca aac gta cct gtg gtc cct gga tcc agt gct act gct aca aat aag       768
Thr Asn Val Pro Val Val Pro Gly Ser Ser Ala Thr Ala Thr Asn Lys
                245                 250                 255

atc act att atc ttc aaa gcc cac cat gag tgt aca gat cag aaa gtc       816
Ile Thr Ile Ile Phe Lys Ala His His Glu Cys Thr Asp Gln Lys Val
            260                 265                 270

tac caa gct gtg aca gat gac ctg ccg gcc gcc ttt gtg gat ggc acc       864
Tyr Gln Ala Val Thr Asp Asp Leu Pro Ala Ala Phe Val Asp Gly Thr
        275                 280                 285

acc agt ggt ggg gac agc gat gcc aag agc ctg cgt atc gtg gaa agg 912
Thr Ser Gly Gly Asp Ser Asp Ala Lys Ser Leu Arg Ile Val Glu Arg
    290                 295                 300

gag agt ggc cac tat gtg gag atg cac gcc cgc tat ata ggg acc aca 960
Glu Ser Gly His Tyr Val Glu Met His Ala Arg Tyr Ile Gly Thr Thr
305                 310                 315                 320

gtg ttt gtg cgg cag gtg ggt cgc tac ctg acc ctt gcc atc cgt atg 1008
Val Phe Val Arg Gln Val Gly Arg Tyr Leu Thr Leu Ala Ile Arg Met
                325                 330                 335

cct gaa gac ctg gcc atg tcc tac gag gag agc cag gac ctg cag ctg 1056
Pro Glu Asp Leu Ala Met Ser Tyr Glu Glu Ser Gln Asp Leu Gln Leu
            340                 345                 350

tgc gtg aac ggc tgc ccc ctg agt gaa cgc atc gat gac ggg cag ggc 1104
Cys Val Asn Gly Cys Pro Leu Ser Glu Arg Ile Asp Asp Gly Gln Gly
        355                 360                 365

cag gtg tct gcc atc ctg gga cac agc ctg cct cgc acc tcc ttg gtg 1152
Gln Val Ser Ala Ile Leu Gly His Ser Leu Pro Arg Thr Ser Leu Val
    370                 375                 380

cag gcc tgg cct ggc tac aca ctg gag act gcc aac act caa tgc cat 1200
Gln Ala Trp Pro Gly Tyr Thr Leu Glu Thr Ala Asn Thr Gln Cys His
385                 390                 395                 400

gag aag atg cca gtg aag gac atc tat ttc cag tcc tgt gtc ttc gac 1248
Glu Lys Met Pro Val Lys Asp Ile Tyr Phe Gln Ser Cys Val Phe Asp
                405                 410                 415

ctg ctc acc act ggt gat gcc aac ttt act gcc gca gcc cac agt gcc 1296
Leu Leu Thr Thr Gly Asp Ala Asn Phe Thr Ala Ala Ala His Ser Ala
            420                 425                 430

ttg gag gat gtg gag gcc ctg cac cca agg aag gaa cgc tgg cac att 1344
Leu Glu Asp Val Glu Ala Leu His Pro Arg Lys Glu Arg Trp His Ile
        435                 440                 445

ttc ccc agc agt ggc aat ggg act ccc cgt gga ggc agt gat ttg tct 1392
Phe Pro Ser Ser Gly Asn Gly Thr Pro Arg Gly Gly Ser Asp Leu Ser
    450                 455                 460

gtc agt cta gga ctc acc tgc ttg atc ctt atc gtg ttt ttg tag 1437
Val Ser Leu Gly Leu Thr Cys Leu Ile Leu Ile Val Phe Leu
465                 470                 475

<210> 4
<211> 478
<212> PRT
<213> Homo sapiens

<400> 4

Met Ile Arg Lys Lys Arg Lys Arg Ser Ala Pro Pro Gly Pro Cys Arg
1               5                   10                  15

Ser His Gly Pro Arg Pro Ala Thr Ala Pro Ala Pro Pro Pro Ser Pro
            20                  25                  30

Glu Pro Thr Arg Pro Ala Trp Thr Gly Met Gly Leu Arg Ala Ala Pro
        35                  40                  45

Ser Ser Ala Ala Ala Ala Ala Ala Glu Val Glu Gln Arg Arg Arg Pro
    50                  55                  60

Gly Leu Cys Pro Pro Pro Leu Glu Leu Leu Leu Leu Leu Leu Phe Ser
65                  70                  75                  80

Leu Gly Leu Leu His Ala Gly Asp Cys Gln Gln Pro Ala Gln Cys Arg
                85                  90                  95

Ile Gln Lys Cys Thr Thr Asp Phe Val Ser Leu Thr Ser His Leu Asn
            100                 105                 110

Ser Ala Val Asp Gly Phe Asp Ser Glu Phe Cys Lys Ala Leu Arg Ala
        115                 120                 125

Tyr Ala Gly Cys Thr Gln Arg Thr Ser Lys Ala Cys Arg Gly Asn Leu
    130                 135                 140

Val Tyr His Ser Ala Val Leu Gly Ile Ser Asp Leu Met Ser Gln Arg
145                 150                 155                 160

Asn Cys Ser Lys Asp Gly Pro Thr Ser Ser Thr Asn Pro Glu Val Thr
                165                 170                 175

His Asp Pro Cys Asn Tyr His Ser His Ala Gly Ala Arg Glu His Arg
            180                 185                 190

Arg Gly Asp Gln Asn Pro Pro Ser Tyr Leu Phe Cys Gly Leu Phe Gly
        195                 200                 205

Asp Pro His Leu Arg Thr Phe Lys Asp Asn Phe Gln Thr Cys Lys Val
    210                 215                 220

Glu Gly Ala Trp Pro Leu Ile Asp Asn Asn Tyr Leu Ser Val Gln Val
225                 230                 235                 240

Thr Asn Val Pro Val Val Pro Gly Ser Ser Ala Thr Ala Thr Asn Lys
                245                 250                 255

Ile Thr Ile Ile Phe Lys Ala His His Glu Cys Thr Asp Gln Lys Val
            260                 265                 270

Tyr Gln Ala Val Thr Asp Asp Leu Pro Ala Ala Phe Val Asp Gly Thr
        275                 280                 285

Thr Ser Gly Gly Asp Ser Asp Ala Lys Ser Leu Arg Ile Val Glu Arg
    290                 295                 300

Glu Ser Gly His Tyr Val Glu Met His Ala Arg Tyr Ile Gly Thr Thr
305                 310                 315                 320

Val Phe Val Arg Gln Val Gly Arg Tyr Leu Thr Leu Ala Ile Arg Met

325                 330                 335

Pro Glu Asp Leu Ala Met Ser Tyr Glu Glu Ser Gln Asp Leu Gln Leu
            340                 345                 350

Cys Val Asn Gly Cys Pro Leu Ser Glu Arg Ile Asp Asp Gly Gln Gly
        355                 360                 365

Gln Val Ser Ala Ile Leu Gly His Ser Leu Pro Arg Thr Ser Leu Val
    370                 375                 380

Gln Ala Trp Pro Gly Tyr Thr Leu Glu Thr Ala Asn Thr Gln Cys His
385                 390                 395                 400

Glu Lys Met Pro Val Lys Asp Ile Tyr Phe Gln Ser Cys Val Phe Asp
                405                 410                 415

Leu Leu Thr Thr Gly Asp Ala Asn Phe Thr Ala Ala Ala His Ser Ala
            420                 425                 430

Leu Glu Asp Val Glu Ala Leu His Pro Arg Lys Glu Arg Trp His Ile
        435                 440                 445

Phe Pro Ser Ser Gly Asn Gly Thr Pro Arg Gly Gly Ser Asp Leu Ser
    450                 455                 460

Val Ser Leu Gly Leu Thr Cys Leu Ile Leu Ile Val Phe Leu
465                 470                 475

<210>  5
<211>  1281
<212>  DNA
<213>  Homo sapiens

<220>
<221>  CDS
<222>  (1)..(1281)

<400>  5
atg ggg gag cca ggc cag tcc cct agt ccc agg tcc tcc cat ggc agt      48
ccc cca act cta agc act ctc act ctc ctg ctg ctc ctc tgt gga cat      96
gct cat tct caa tgc aag atc ctc cgc tgc aat gct gag tac gta tcg     144
tcc act ctg agc ctt aga ggt ggg ggt tca tca gga gca ctt cga gga     192
gga gga gga gga ggc cgg ggt gga ggg gtg ggc tct ggc ggc ctc tgt     240
cga gcc ctc cgc tcc tat gcg ctc tgc act cgg cgc acc gcc cgc acc 288
tgc cgc ggg gac ctc gcc ttc cat tcg gcg gta cat ggc atc gaa gac 336
ctg atg atc cag cac aac tgc tcc cgc cag ggc cct aca gcc cct ccc 384
ccg ccc cgg ggc ccc gcc ctt cca ggc gcg ggc tcc ggc ctc cct gcc 432
ccg gac cct tgt gac tat gaa ggc cgg ttt tcc cgg ctg cat ggt cgt 480
ccc ccg ggg ttc ttg cat tgc gct tcc ttc ggg gac ccc cat gtg cgc 528
agc ttc cac cat cac ttt cac aca tgc cgt gtc caa gga gct tgg cct 576
cta ctg gat aat gac ttc ctc ttt gtc caa gcc acc agc tcc ccc atg 624
gcg ttg ggg gcc aac gct acc gcc acc cgg aag ctc acc atc ata ttt 672
aag aac atg cag gaa tgc att gat cag aag gtg tat cag gct gag gtg 720
gat aat ctt cct gta gcc ttt gaa gat ggt tct atc aat gga ggt gac 768
cga cct ggg gga tcc agt ttg tcg att caa act gct aac cct ggg aac 816
cat gtg gag atc caa gct gcc tac att ggc aca act ata atc att cgg 864
cag aca gct ggg cag ctc tcc ttc tcc atc aag gta gca gag gat gtg 912
gcc atg gcc ttc tca gct gaa cag gac ctg cag ctc tgt gtt ggg ggg 960
tgc cct cca agt cag cga ctc tct cga tca gag cgc aat cgt cgg gga 1008
gct ata acc att gat act gcc aga cgg ctg tgc aag gaa ggg ctt cca 1056
gtg gaa gat gct tac ttc cat tcc tgt gtc ttt gat gtt tta att tct      1104
ggt gat ccc aac ttt acc gtg gca gct cag gca gca ctg gag gat gcc      1152
cga gcc ttc ctg cca gac tta gag aag ctg cat ctc ttc ccc tca gat      1200
gct ggg gtt cct ctt tcc tca gca acc ctc tta gct cca ctc ctt tct      1248
ggg ctc ttt gtt ctg tgg ctt tgc att cag taa                          1281

<210>  6
<211>  426
<212>  PRT
<213>  Homo sapiens

<400>  6

<210> 7
<211> 15

<212>   PRT
<213>   Artificial

<220>
<223>   Consensus sequence


<220>
<221>   MISC_FEATURE
<222>   (1)..(1)
<223>   Xaa is any amino acid

<220>
<221>   MISC_FEATURE
<222>   (3)..(3)
<223>   Xaa is K or R

<220>
<221>   MISC_FEATURE
<222>   (3)..(3)
<223>   Xaa is Lys (K) or Arg (R)

<220>
<221>   MISC_FEATURE
<222>   (5)..(5)
<223>   Xaa is any amino acid

<220>
<221>   MISC_FEATURE
<222>   (6)..(6)
<223>   Xaa is K or R

<220>
<221>   MISC_FEATURE
<222>   (8)..(8)
<223>   Xaa is any amino acid

<220>
<221>   MISC_FEATURE
<222>   (9)..(9)
<223>   Xaa is Ser, Thr or Ala

<220>
<221>   MISC_FEATURE
<222>   (10)..(10)
<223>   Xaa is E or D

<220>
<221>   MISC_FEATURE
<222>   (11)..(11)
<223>   Xaa is F or Y

<220>
<221>   MISC_FEATURE
<222>   (12)..(12)
<223>   Xaa is any amino acid

<220>
<221>   MISC_FEATURE
<222>   (14)..(14)
<223>   Xaa is any amino acid

<400>   7

Xaa Cys Xaa Ile Xaa Xaa Cys Xaa Xaa Xaa Xaa Xaa Ser Xaa Thr
1               5                   10                  15


<210>   8

<211>  16
<212>  PRT
<213>  Artificial

<220>
<223>  Consensus 2


<220>
<221>  MISC_FEATURE
<222>  (1)..(1)
<223>  Xaa is any amino acid

<220>
<221>  MISC_FEATURE
<222>  (3)..(3)
<223>  Xaa is any amino acid

<220>
<221>  MISC_FEATURE
<222>  (7)..(7)
<223>  Xaa is any amino acid

<220>
<221>  MISC_FEATURE
<222>  (10)..(10)
<223>  Xaa is any amino acid

<220>
<221>  MISC_FEATURE
<222>  (13)..(13)
<223>  Xaa is any amino acid

<220>
<221>  MISC_FEATURE
<222>  (16)..(16)
<223>  Xaa is any amino acid

<400>  8

Xaa Cys Xaa Ala Leu Arg Xaa Tyr Ala Xaa Cys Thr Xaa Arg Thr Xaa
1               5                   10                  15


<210>  9
<211>  933
<212>  DNA
<213>  Artificial

<220>
<223>  hRGMA 47-90 Fc Plasmid


<220>
<221>  CDS
<222>  (1)..(933)

<220>
<221>  misc_feature
<222>  (1)..(55)
<223>  Murine Ig k-chain V-J2-Csignal peptide

<220>
<221>  misc_feature
<222>  (103)..(234)
<223>  RGMA 47-90

<220>
<221>  misc_feature

<222>  (250)..(260)
<223>  Factor Xa

<220>
<221>  misc_feature
<222>  (261)..(933)
<223>  Fc

<400>  9
atg gag aca gac aca ctc ctg cta tgg gta ctg ctg ctc tgg gtt cca      48
Met Glu Thr Asp Thr Leu Leu Leu Trp Val Leu Leu Leu Trp Val Pro
1               5                   10                  15

ggt tcc act ggt gac gcg gcc cag ccg gcc agg cgc gcg cgc cgt acg      96
Gly Ser Thr Gly Asp Ala Ala Gln Pro Ala Arg Arg Ala Arg Arg Thr
            20                  25                  30

aag ctt ccg tgc aag atc ctc aag tgc aac tct gag ttc tgg agc gcc      144
Lys Leu Pro Cys Lys Ile Leu Lys Cys Asn Ser Glu Phe Trp Ser Ala
        35                  40                  45

acg tcg ggc agc cac gcc cca gcc tca gac gac acc ccc gag ttc tgt      192
Thr Ser Gly Ser His Ala Pro Ala Ser Asp Asp Thr Pro Glu Phe Cys
    50                  55                  60

gca gcc ttg cgc agc tac gcc ctg tgc acg cgg cgg acg gcc ttg aat      240
Ala Ala Leu Arg Ser Tyr Ala Leu Cys Thr Arg Arg Thr Ala Leu Asn
65                  70                  75                  80

tct gca gat atc gag gga cga atg gat cca ccg tgc cca gca cct gaa      288
Ser Ala Asp Ile Glu Gly Arg Met Asp Pro Pro Cys Pro Ala Pro Glu
                85                  90                  95

ctc ctg ggg gga ccg tca gtc ttc ctc ttc ccc cca aaa ccc aag gac      336
Leu Leu Gly Gly Pro Ser Val Phe Leu Phe Pro Pro Lys Pro Lys Asp
            100                 105                 110

acc ctc atg atc tcc cgg acc cct gag gtc aca tgc gtg gtg gtg gac      384
Thr Leu Met Ile Ser Arg Thr Pro Glu Val Thr Cys Val Val Val Asp
        115                 120                 125

gtg agc cac gaa gac cct gag gtc aag ttc aac tgg tac gtg gac ggc      432
Val Ser His Glu Asp Pro Glu Val Lys Phe Asn Trp Tyr Val Asp Gly
    130                 135                 140

gtg gag gtg cat aat gcc aag aca aag ccg cgg gag gag cag tac aac      480
Val Glu Val His Asn Ala Lys Thr Lys Pro Arg Glu Glu Gln Tyr Asn
145                 150                 155                 160

agc acg tac cgt gtg gtc agc gtc ctc acc gtc ctg cac cag gac tgg      528
Ser Thr Tyr Arg Val Val Ser Val Leu Thr Val Leu His Gln Asp Trp
                165                 170                 175

ctg aat ggc aag gag tac aag tgc aag gtc tcc aac aaa gcc ctc cca      576
Leu Asn Gly Lys Glu Tyr Lys Cys Lys Val Ser Asn Lys Ala Leu Pro
            180                 185                 190

gcc ccc atc gag aaa acc atc tcc aaa gcc aaa ggg cag ccc cga gaa      624
Ala Pro Ile Glu Lys Thr Ile Ser Lys Ala Lys Gly Gln Pro Arg Glu
        195                 200                 205

cca cag gtg tac acc ctg ccc cca tcc cgg gag gag atg acc aag aac      672
Pro Gln Val Tyr Thr Leu Pro Pro Ser Arg Glu Glu Met Thr Lys Asn
    210                 215                 220

cag gtc agc ctg acc tgc ctg gtc aaa ggc ttc tat ccc agc gac atc      720
Gln Val Ser Leu Thr Cys Leu Val Lys Gly Phe Tyr Pro Ser Asp Ile
225                 230                 235                 240

gcc gtg gag tgg gag agc aat ggg cag ccg gag aac aac tac aag acc        768
Ala Val Glu Trp Glu Ser Asn Gly Gln Pro Glu Asn Asn Tyr Lys Thr
                245                 250                 255

acg cct ccc gtg ctg gac tcc gac ggc tcc ttc ttc ctc tat agc aag        816
Thr Pro Pro Val Leu Asp Ser Asp Gly Ser Phe Phe Leu Tyr Ser Lys
            260                 265                 270

ctc acc gtg gac aag agc agg tgg cag cag ggg aac gtc ttc tca tgc        864
Leu Thr Val Asp Lys Ser Arg Trp Gln Gln Gly Asn Val Phe Ser Cys
        275                 280                 285

tcc gtg atg cat gag gct ctg cac aac cac tac acg cag aag agc ctc        912
Ser Val Met His Glu Ala Leu His Asn His Tyr Thr Gln Lys Ser Leu
    290                 295                 300

tcc ctg tct ccg ggt aaa tga                                            933
Ser Leu Ser Pro Gly Lys
305                 310


<210>  10
<211>  310
<212>  PRT
<213>  Artificial

<220>
<223>  Synthetic Construct

<400>  10
   Met Glu Thr Asp Thr Leu Leu Leu Trp Val Leu Leu Leu Trp Val Pro
   1               5                   10                  15
   Gly Ser Thr Gly Asp Ala Ala Gln Pro Ala Arg Arg Ala Arg Arg Thr
               20                  25                  30
   Lys Leu Pro Cys Lys Ile Leu Lys Cys Asn Ser Glu Phe Trp Ser Ala
           35                  40                  45
   Thr Ser Gly Ser His Ala Pro Ala Ser Asp Asp Thr Pro Glu Phe Cys
       50                  55                  60
   Ala Ala Leu Arg Ser Tyr Ala Leu Cys Thr Arg Arg Thr Ala Leu Asn
   65                  70                  75                  80
   Ser Ala Asp Ile Glu Gly Arg Met Asp Pro Pro Cys Pro Ala Pro Glu
                   85                  90                  95
   Leu Leu Gly Gly Pro Ser Val Phe Leu Phe Pro Pro Lys Pro Lys Asp
               100                 105                 110
   Thr Leu Met Ile Ser Arg Thr Pro Glu Val Thr Cys Val Val Val Asp
           115                 120                 125
   Val Ser His Glu Asp Pro Glu Val Lys Phe Asn Trp Tyr Val Asp Gly
       130                 135                 140
   Val Glu Val His Asn Ala Lys Thr Lys Pro Arg Glu Glu Gln Tyr Asn
   145                 150                 155                 160
   Ser Thr Tyr Arg Val Val Ser Val Leu Thr Val Leu His Gln Asp Trp
                   165                 170                 175
   Leu Asn Gly Lys Glu Tyr Lys Cys Lys Val Ser Asn Lys Ala Leu Pro
               180                 185                 190
   Ala Pro Ile Glu Lys Thr Ile Ser Lys Ala Lys Gly Gln Pro Arg Glu
           195                 200                 205
   Pro Gln Val Tyr Thr Leu Pro Pro Ser Arg Glu Glu Met Thr Lys Asn
       210                 215                 220
   Gln Val Ser Leu Thr Cys Leu Val Lys Gly Phe Tyr Pro Ser Asp Ile
   225                 230                 235                 240
   Ala Val Glu Trp Glu Ser Asn Gly Gln Pro Glu Asn Asn Tyr Lys Thr
                   245                 250                 255
   Thr Pro Pro Val Leu Asp Ser Asp Gly Ser Phe Phe Leu Tyr Ser Lys
               260                 265                 270
   Leu Thr Val Asp Lys Ser Arg Trp Gln Gln Gly Asn Val Phe Ser Cys
           275                 280                 285
   Ser Val Met His Glu Ala Leu His Asn His Tyr Thr Gln Lys Ser Leu
       290                 295                 300
   Ser Leu Ser Pro Gly Lys
   305                 310

<210>  11
<211>  1167
<212>  DNA
<213>  Artificial

<220>
<223>  hRGMA 47-168 Fc Plasmid

<220>
<221>  CDS
<222>  (1)..(1167)

<220>
<221>  misc_feature
<222>  (103)..(468)
<223>  hRGMA 47-168

<220>
<221>  misc_feature
<222>  (496)..(1167)
<223>  Fc

<400>  11
atg gag aca gac aca ctc ctg cta tgg gta ctg ctg ctc tgg gtt cca        48
Met Glu Thr Asp Thr Leu Leu Leu Trp Val Leu Leu Leu Trp Val Pro
1               5                   10                  15

ggt tcc act ggt gac gcg gcc cag ccg gcc agg cgc gcg cgc cgt acg 96
Gly Ser Thr Gly Asp Ala Ala Gln Pro Ala Arg Arg Ala Arg Arg Thr
            20                  25                  30

aag ctt ccg tgc aag atc ctc aag tgc aac tct gag ttc tgg agc gcc 144
Lys Leu Pro Cys Lys Ile Leu Lys Cys Asn Ser Glu Phe Trp Ser Ala
        35                  40                  45

acg tcg ggc agc cac gcc cca gcc tca gac gac acc ccc gag ttc tgt 192
Thr Ser Gly Ser His Ala Pro Ala Ser Asp Asp Thr Pro Glu Phe Cys
    50                  55                  60

gca gcc ttg cgc agc tac gcc ctg tgc acg cgg cgg acg gcc cgc acc 240
Ala Ala Leu Arg Ser Tyr Ala Leu Cys Thr Arg Arg Thr Ala Arg Thr
65                  70                  75                  80

tgc cgg ggt gac ctg gcc tac cac tcg gcc gtc cat ggc ata gag gac 288
Cys Arg Gly Asp Leu Ala Tyr His Ser Ala Val His Gly Ile Glu Asp
                85                  90                  95

ctc atg agc cag cac aac tgc tcc aag gat ggc ccc acc tcg cag cca 336
Leu Met Ser Gln His Asn Cys Ser Lys Asp Gly Pro Thr Ser Gln Pro
            100                 105                 110

cgc ctg cgc acg ctc cca ccg gcc gga gac agc cag gag cgc tcg gac 384
Arg Leu Arg Thr Leu Pro Pro Ala Gly Asp Ser Gln Glu Arg Ser Asp
        115                 120                 125

agc ccc gag atc tgc cat tac gag aag agc ttt cac aag cac tcg gcc 432
Ser Pro Glu Ile Cys His Tyr Glu Lys Ser Phe His Lys His Ser Ala
    130                 135                 140

acc ccc aac tac acg cac tgt ggc ctc ttc ggg gac ttg aat tct gca 480
Thr Pro Asn Tyr Thr His Cys Gly Leu Phe Gly Asp Leu Asn Ser Ala
145                 150                 155                 160

gat atc gag gga cga atg gat cca ccg tgc cca gca cct gaa ctc ctg 528
Asp Ile Glu Gly Arg Met Asp Pro Pro Cys Pro Ala Pro Glu Leu Leu
                165                 170                 175

ggg gga ccg tca gtc ttc ctc ttc ccc cca aaa ccc aag gac acc ctc 576
Gly Gly Pro Ser Val Phe Leu Phe Pro Pro Lys Pro Lys Asp Thr Leu
            180                 185                 190

atg atc tcc cgg acc cct gag gtc aca tgc gtg gtg gtg gac gtg agc 624
Met Ile Ser Arg Thr Pro Glu Val Thr Cys Val Val Val Asp Val Ser
        195                 200                 205

cac gaa gac cct gag gtc aag ttc aac tgg tac gtg gac ggc gtg gag 672
His Glu Asp Pro Glu Val Lys Phe Asn Trp Tyr Val Asp Gly Val Glu
    210                 215                 220

gtg cat aat gcc aag aca aag ccg cgg gag gag cag tac aac agc acg 720
Val His Asn Ala Lys Thr Lys Pro Arg Glu Glu Gln Tyr Asn Ser Thr
225                 230                 235                 240

tac cgt gtg gtc agc gtc ctc acc gtc ctg cac cag gac tgg ctg aat 768
Tyr Arg Val Val Ser Val Leu Thr Val Leu His Gln Asp Trp Leu Asn
                245                 250                 255

ggc aag gag tac aag tgc aag gtc tcc aac aaa gcc ctc cca gcc ccc 816
Gly Lys Glu Tyr Lys Cys Lys Val Ser Asn Lys Ala Leu Pro Ala Pro
            260                 265                 270

atc gag aaa acc atc tcc aaa gcc aaa ggg cag ccc cga gaa cca cag 864
Ile Glu Lys Thr Ile Ser Lys Ala Lys Gly Gln Pro Arg Glu Pro Gln
        275                 280                 285

gtg tac acc ctg ccc cca tcc cgg gag gag atg acc aag aac cag gtc        912
Val Tyr Thr Leu Pro Pro Ser Arg Glu Glu Met Thr Lys Asn Gln Val
    290                 295                 300

agc ctg acc tgc ctg gtc aaa ggc ttc tat ccc agc gac atc gcc gtg        960
Ser Leu Thr Cys Leu Val Lys Gly Phe Tyr Pro Ser Asp Ile Ala Val
305                 310                 315                 320

gag tgg gag agc aat ggg cag ccg gag aac aac tac aag acc acg cct       1008
Glu Trp Glu Ser Asn Gly Gln Pro Glu Asn Asn Tyr Lys Thr Thr Pro
                325                 330                 335

ccc gtg ctg gac tcc gac ggc tcc ttc ttc ctc tat agc aag ctc acc       1056
Pro Val Leu Asp Ser Asp Gly Ser Phe Phe Leu Tyr Ser Lys Leu Thr
            340                 345                 350

gtg gac aag agc agg tgg cag cag ggg aac gtc ttc tca tgc tcc gtg       1104
Val Asp Lys Ser Arg Trp Gln Gln Gly Asn Val Phe Ser Cys Ser Val
        355                 360                 365

atg cat gag gct ctg cac aac cac tac acg cag aag agc ctc tcc ctg       1152
Met His Glu Ala Leu His Asn His Tyr Thr Gln Lys Ser Leu Ser Leu
    370                 375                 380

tct ccg ggt aaa tga                                                   1167
Ser Pro Gly Lys
385


<210>  12
<211>  388
<212>  PRT
<213>  Artificial

<220>
<223>  Synthetic Construct

<400>  12

Met Glu Thr Asp Thr Leu Leu Leu Trp Val Leu Leu Leu Trp Val Pro
1               5                   10                  15


Gly Ser Thr Gly Asp Ala Ala Gln Pro Ala Arg Arg Ala Arg Arg Thr
            20                  25                  30


Lys Leu Pro Cys Lys Ile Leu Lys Cys Asn Ser Glu Phe Trp Ser Ala
        35                  40                  45


Thr Ser Gly Ser His Ala Pro Ala Ser Asp Asp Thr Pro Glu Phe Cys
    50                  55                  60


Ala Ala Leu Arg Ser Tyr Ala Leu Cys Thr Arg Arg Thr Ala Arg Thr
65                  70                  75                  80


Cys Arg Gly Asp Leu Ala Tyr His Ser Ala Val His Gly Ile Glu Asp
                85                  90                  95


Leu Met Ser Gln His Asn Cys Ser Lys Asp Gly Pro Thr Ser Gln Pro
            100                 105                 110


Arg Leu Arg Thr Leu Pro Pro Ala Gly Asp Ser Gln Glu Arg Ser Asp
        115                 120                 125

Ser Pro Glu Ile Cys His Tyr Glu Lys Ser Phe His Lys His Ser Ala
    130                 135                 140

Thr Pro Asn Tyr Thr His Cys Gly Leu Phe Gly Asp Leu Asn Ser Ala
145                 150                 155                 160

Asp Ile Glu Gly Arg Met Asp Pro Pro Cys Pro Ala Pro Glu Leu Leu
                165                 170                 175

Gly Gly Pro Ser Val Phe Leu Phe Pro Pro Lys Pro Lys Asp Thr Leu
            180                 185                 190

Met Ile Ser Arg Thr Pro Glu Val Thr Cys Val Val Val Asp Val Ser
        195                 200                 205

His Glu Asp Pro Glu Val Lys Phe Asn Trp Tyr Val Asp Gly Val Glu
    210                 215                 220

Val His Asn Ala Lys Thr Lys Pro Arg Glu Glu Gln Tyr Asn Ser Thr
225                 230                 235                 240

Tyr Arg Val Val Ser Val Leu Thr Val Leu His Gln Asp Trp Leu Asn
                245                 250                 255

Gly Lys Glu Tyr Lys Cys Lys Val Ser Asn Lys Ala Leu Pro Ala Pro
            260                 265                 270

Ile Glu Lys Thr Ile Ser Lys Ala Lys Gly Gln Pro Arg Glu Pro Gln
        275                 280                 285

Val Tyr Thr Leu Pro Pro Ser Arg Glu Glu Met Thr Lys Asn Gln Val
    290                 295                 300

Ser Leu Thr Cys Leu Val Lys Gly Phe Tyr Pro Ser Asp Ile Ala Val
305                 310                 315                 320

Glu Trp Glu Ser Asn Gly Gln Pro Glu Asn Asn Tyr Lys Thr Thr Pro
                325                 330                 335

Pro Val Leu Asp Ser Asp Gly Ser Phe Phe Leu Tyr Ser Lys Leu Thr
            340                 345                 350

Val Asp Lys Ser Arg Trp Gln Gln Gly Asn Val Phe Ser Cys Ser Val
        355                 360                 365

Met His Glu Ala Leu His Asn His Tyr Thr Gln Lys Ser Leu Ser Leu
    370                 375                 380

Ser Pro Gly Lys
385

<210>  13
<211>  1197
<212>  DNA
<213>  Artificial

<220>
<223>  hRGMa 41-168 Fc Plasmid


<220>
<221>  CDS
<222>  (1)..(1197)

<400>  13
atg gag aca gac aca ctc ctg cta tgg gta ctg ctg ctc tgg gtt cca      48
Met Glu Thr Asp Thr Leu Leu Leu Trp Val Leu Leu Leu Trp Val Pro
1               5                   10                  15

ggt tcc act ggt gac gcg gcc cag ccg gcc agg cgc gcg cgc cgt acg      96
Gly Ser Thr Gly Asp Ala Ala Gln Pro Ala Arg Arg Ala Arg Arg Thr
            20                  25                  30

aag ctt ttc ccc gca gcc acc tcc ccg tgc aag atc ctc aag tgc aac      144
Lys Leu Phe Pro Ala Ala Thr Ser Pro Cys Lys Ile Leu Lys Cys Asn
        35                  40                  45

tct gag ttc tgg agc gcc acg tcg ggc agc cac gcc cca gcc tca gac      192
Ser Glu Phe Trp Ser Ala Thr Ser Gly Ser His Ala Pro Ala Ser Asp
    50                  55                  60

gac acc ccc gag ttc tgt gca gcc ttg cgc agc tac gcc ctg tgc acg      240
Asp Thr Pro Glu Phe Cys Ala Ala Leu Arg Ser Tyr Ala Leu Cys Thr
65                  70                  75                  80

cgg cgg acg gcc cgc acc tgc cgg ggt gac ctg gcc tac cac tcg gcc      288
Arg Arg Thr Ala Arg Thr Cys Arg Gly Asp Leu Ala Tyr His Ser Ala
                85                  90                  95

gtc cat ggc ata gag gac ctc atg agc cag cac aac tgc tcc aag gat      336
Val His Gly Ile Glu Asp Leu Met Ser Gln His Asn Cys Ser Lys Asp
            100                 105                 110

ggc ccc acc tcg cag cca cgc ctg cgc acg ctc cca ccg gcc gga gac      384
Gly Pro Thr Ser Gln Pro Arg Leu Arg Thr Leu Pro Pro Ala Gly Asp
        115                 120                 125

agc cag gag cgc tcg gac agc ccc gag atc tgc cat tac gag aag agc      432
Ser Gln Glu Arg Ser Asp Ser Pro Glu Ile Cys His Tyr Glu Lys Ser
    130                 135                 140

ttt cac aag cac tcg gcc acc ccc aac tac acg cac tgt ggc ctc ttc      480
Phe His Lys His Ser Ala Thr Pro Asn Tyr Thr His Cys Gly Leu Phe
145                 150                 155                 160

ggg gac atc gaa ggt cgt ttg aat tct gca gat atc gag gga cga atg      528
Gly Asp Ile Glu Gly Arg Leu Asn Ser Ala Asp Ile Glu Gly Arg Met
                165                 170                 175

gat cca ccg tgc cca gca cct gaa ctc ctg ggg gga ccg tca gtc ttc      576
Asp Pro Pro Cys Pro Ala Pro Glu Leu Leu Gly Gly Pro Ser Val Phe
            180                 185                 190

ctc ttc ccc cca aaa ccc aag gac acc ctc atg atc tcc cgg acc cct      624
Leu Phe Pro Pro Lys Pro Lys Asp Thr Leu Met Ile Ser Arg Thr Pro
        195                 200                 205

gag gtc aca tgc gtg gtg gtg gac gtg agc cac gaa gac cct gag gtc      672
Glu Val Thr Cys Val Val Val Asp Val Ser His Glu Asp Pro Glu Val

210                 215                 220

aag ttc aac tgg tac gtg gac ggc gtg gag gtg cat aat gcc aag aca        720
Lys Phe Asn Trp Tyr Val Asp Gly Val Glu Val His Asn Ala Lys Thr
225                 230                 235                 240

aag ccg cgg gag gag cag tac aac agc acg tac cgt gtg gtc agc gtc        768
Lys Pro Arg Glu Glu Gln Tyr Asn Ser Thr Tyr Arg Val Val Ser Val
                245                 250                 255

ctc acc gtc ctg cac cag gac tgg ctg aat ggc aag gag tac aag tgc        816
Leu Thr Val Leu His Gln Asp Trp Leu Asn Gly Lys Glu Tyr Lys Cys
            260                 265                 270

aag gtc tcc aac aaa gcc ctc cca gcc ccc atc gag aaa acc atc tcc        864
Lys Val Ser Asn Lys Ala Leu Pro Ala Pro Ile Glu Lys Thr Ile Ser
        275                 280                 285

aaa gcc aaa ggg cag ccc cga gaa cca cag gtg tac acc ctg ccc cca        912
Lys Ala Lys Gly Gln Pro Arg Glu Pro Gln Val Tyr Thr Leu Pro Pro
    290                 295                 300

tcc cgg gag gag atg acc aag aac cag gtc agc ctg acc tgc ctg gtc        960
Ser Arg Glu Glu Met Thr Lys Asn Gln Val Ser Leu Thr Cys Leu Val
305                 310                 315                 320

aaa ggc ttc tat ccc agc gac atc gcc gtg gag tgg gag agc aat ggg        1008
Lys Gly Phe Tyr Pro Ser Asp Ile Ala Val Glu Trp Glu Ser Asn Gly
                325                 330                 335

cag ccg gag aac aac tac aag acc acg cct ccc gtg ctg gac tcc gac        1056
Gln Pro Glu Asn Asn Tyr Lys Thr Thr Pro Pro Val Leu Asp Ser Asp
            340                 345                 350

ggc tcc ttc ttc ctc tat agc aag ctc acc gtg gac aag agc agg tgg        1104
Gly Ser Phe Phe Leu Tyr Ser Lys Leu Thr Val Asp Lys Ser Arg Trp
        355                 360                 365

cag cag ggg aac gtc ttc tca tgc tcc gtg atg cat gag gct ctg cac        1152
Gln Gln Gly Asn Val Phe Ser Cys Ser Val Met His Glu Ala Leu His
    370                 375                 380

aac cac tac acg cag aag agc ctc tcc ctg tct ccg ggt aaa tga            1197
Asn His Tyr Thr Gln Lys Ser Leu Ser Leu Ser Pro Gly Lys
385                 390                 395


<210>  14
<211>  398
<212>  PRT
<213>  Artificial

<220>
<223>  Synthetic Construct

<400>  14

Met Glu Thr Asp Thr Leu Leu Leu Trp Val Leu Leu Leu Trp Val Pro
1               5                   10                  15


Gly Ser Thr Gly Asp Ala Ala Gln Pro Ala Arg Arg Ala Arg Arg Thr
            20                  25                  30


Lys Leu Phe Pro Ala Ala Thr Ser Pro Cys Lys Ile Leu Lys Cys Asn
        35                  40                  45

Ser Glu Phe Trp Ser Ala Thr Ser Gly Ser His Ala Pro Ala Ser Asp
    50                  55                  60

Asp Thr Pro Glu Phe Cys Ala Ala Leu Arg Ser Tyr Ala Leu Cys Thr
65                  70                  75                  80

Arg Arg Thr Ala Arg Thr Cys Arg Gly Asp Leu Ala Tyr His Ser Ala
                85                  90                  95

Val His Gly Ile Glu Asp Leu Met Ser Gln His Asn Cys Ser Lys Asp
            100                 105                 110

Gly Pro Thr Ser Gln Pro Arg Leu Arg Thr Leu Pro Pro Ala Gly Asp
        115                 120                 125

Ser Gln Glu Arg Ser Asp Ser Pro Glu Ile Cys His Tyr Glu Lys Ser
    130                 135                 140

Phe His Lys His Ser Ala Thr Pro Asn Tyr Thr His Cys Gly Leu Phe
145                 150                 155                 160

Gly Asp Ile Glu Gly Arg Leu Asn Ser Ala Asp Ile Glu Gly Arg Met
                165                 170                 175

Asp Pro Pro Cys Pro Ala Pro Glu Leu Leu Gly Gly Pro Ser Val Phe
            180                 185                 190

Leu Phe Pro Pro Lys Pro Lys Asp Thr Leu Met Ile Ser Arg Thr Pro
        195                 200                 205

Glu Val Thr Cys Val Val Val Asp Val Ser His Glu Asp Pro Glu Val
    210                 215                 220

Lys Phe Asn Trp Tyr Val Asp Gly Val Glu Val His Asn Ala Lys Thr
225                 230                 235                 240

Lys Pro Arg Glu Glu Gln Tyr Asn Ser Thr Tyr Arg Val Val Ser Val
                245                 250                 255

Leu Thr Val Leu His Gln Asp Trp Leu Asn Gly Lys Glu Tyr Lys Cys
            260                 265                 270

Lys Val Ser Asn Lys Ala Leu Pro Ala Pro Ile Glu Lys Thr Ile Ser
        275                 280                 285

Lys Ala Lys Gly Gln Pro Arg Glu Pro Gln Val Tyr Thr Leu Pro Pro
    290                 295                 300

Ser Arg Glu Glu Met Thr Lys Asn Gln Val Ser Leu Thr Cys Leu Val
305                 310                 315                 320

Lys Gly Phe Tyr Pro Ser Asp Ile Ala Val Glu Trp Glu Ser Asn Gly

                325                 330                 335

Gln Pro Glu Asn Asn Tyr Lys Thr Thr Pro Pro Val Leu Asp Ser Asp
            340                 345                 350

Gly Ser Phe Phe Leu Tyr Ser Lys Leu Thr Val Asp Lys Ser Arg Trp
        355                 360                 365

Gln Gln Gly Asn Val Phe Ser Cys Ser Val Met His Glu Ala Leu His
    370                 375                 380

Asn His Tyr Thr Gln Lys Ser Leu Ser Leu Ser Pro Gly Lys
385                 390                 395

<210> 15
<211> 1014
<212> DNA
<213> Artificial

<220>
<223> hRGMA 316-386 Fc Plasmid

<220>
<221> CDS
<222> (1)..(1014)

<400> 15
atg gag aca gac aca ctc ctg cta tgg gta ctg ctg ctc tgg gtt cca 48
Met Glu Thr Asp Thr Leu Leu Leu Trp Val Leu Leu Leu Trp Val Pro
1               5                   10                  15

ggt tcc act ggt gac gcg gcc cag ccg gcc agg cgc gcg cgc cgt acg 96
Gly Ser Thr Gly Asp Ala Ala Gln Pro Ala Arg Arg Ala Arg Arg Thr
            20                  25                  30

aag ctt ctg cgg ggc tgc ccc ctc aac cag cag atc gac ttc cag gcc 144
Lys Leu Leu Arg Gly Cys Pro Leu Asn Gln Gln Ile Asp Phe Gln Ala
        35                  40                  45

ttc cac acc aat gct gag ggc acc ggt gcc cgc agg ctg gca gcc gcc 192
Phe His Thr Asn Ala Glu Gly Thr Gly Ala Arg Arg Leu Ala Ala Ala
    50                  55                  60

agc cct gca ccc aca gcc ccc gag acc ttc cca tac gag aca gcc gtg 240
Ser Pro Ala Pro Thr Ala Pro Glu Thr Phe Pro Tyr Glu Thr Ala Val
65                  70                  75                  80

gcc aag tgc aag gag aag ctg ccg gtg gag gac ctg tac tac cag gcc 288
Ala Lys Cys Lys Glu Lys Leu Pro Val Glu Asp Leu Tyr Tyr Gln Ala
                85                  90                  95

tgc gtc ttc gac ctc ctc acc acg ggc ttg aat tct gca gat atc gag 336
Cys Val Phe Asp Leu Leu Thr Thr Gly Leu Asn Ser Ala Asp Ile Glu
            100                 105                 110

gga cga atg gat cca ccg tgc cca gca cct gaa ctc ctg ggg gga ccg 384
Gly Arg Met Asp Pro Pro Cys Pro Ala Pro Glu Leu Leu Gly Gly Pro
        115                 120                 125

tca gtc ttc ctc ttc ccc cca aaa ccc aag gac acc ctc atg atc tcc 432
Ser Val Phe Leu Phe Pro Pro Lys Pro Lys Asp Thr Leu Met Ile Ser
    130                 135                 140

cgg acc cct gag gtc aca tgc gtg gtg gtg gac gtg agc cac gaa gac 480
Arg Thr Pro Glu Val Thr Cys Val Val Val Asp Val Ser His Glu Asp
145                 150                 155                 160

cct gag gtc aag ttc aac tgg tac gtg gac ggc gtg gag gtg cat aat 528
Pro Glu Val Lys Phe Asn Trp Tyr Val Asp Gly Val Glu Val His Asn
                165                 170                 175

gcc aag aca aag ccg cgg gag gag cag tac aac agc acg tac cgt gtg 576
Ala Lys Thr Lys Pro Arg Glu Glu Gln Tyr Asn Ser Thr Tyr Arg Val
            180                 185                 190

gtc agc gtc ctc acc gtc ctg cac cag gac tgg ctg aat ggc aag gag 624
Val Ser Val Leu Thr Val Leu His Gln Asp Trp Leu Asn Gly Lys Glu
        195                 200                 205

tac aag tgc aag gtc tcc aac aaa gcc ctc cca gcc ccc atc gag aaa 672
Tyr Lys Cys Lys Val Ser Asn Lys Ala Leu Pro Ala Pro Ile Glu Lys
    210                 215                 220

acc atc tcc aaa gcc aaa ggg cag ccc cga gaa cca cag gtg tac acc 720
Thr Ile Ser Lys Ala Lys Gly Gln Pro Arg Glu Pro Gln Val Tyr Thr
225                 230                 235                 240

ctg ccc cca tcc cgg gag gag atg acc aag aac cag gtc agc ctg acc 768
Leu Pro Pro Ser Arg Glu Glu Met Thr Lys Asn Gln Val Ser Leu Thr
                245                 250                 255

tgc ctg gtc aaa ggc ttc tat ccc agc gac atc gcc gtg gag tgg gag 816
Cys Leu Val Lys Gly Phe Tyr Pro Ser Asp Ile Ala Val Glu Trp Glu
            260                 265                 270

agc aat ggg cag ccg gag aac aac tac aag acc acg cct ccc gtg ctg 864
Ser Asn Gly Gln Pro Glu Asn Asn Tyr Lys Thr Thr Pro Pro Val Leu
        275                 280                 285

gac tcc gac ggc tcc ttc ttc ctc tat agc aag ctc acc gtg gac aag 912
Asp Ser Asp Gly Ser Phe Phe Leu Tyr Ser Lys Leu Thr Val Asp Lys
    290                 295                 300

agc agg tgg cag cag ggg aac gtc ttc tca tgc tcc gtg atg cat gag 960
Ser Arg Trp Gln Gln Gly Asn Val Phe Ser Cys Ser Val Met His Glu
305                 310                 315                 320

gct ctg cac aac cac tac acg cag aag agc ctc tcc ctg tct ccg ggt 1008
Ala Leu His Asn His Tyr Thr Gln Lys Ser Leu Ser Leu Ser Pro Gly
                325                 330                 335

aaa tga 1014
Lys

<210> 16
<211> 337
<212> PRT
<213> Artificial

<220>
<223> Synthetic Construct

<400> 16

Met Glu Thr Asp Thr Leu Leu Leu Trp Val Leu Leu Leu Trp Val Pro
1               5                   10                  15

Gly Ser Thr Gly Asp Ala Ala Gln Pro Ala Arg Arg Ala Arg Arg Thr
            20                  25                  30

Lys Leu Leu Arg Gly Cys Pro Leu Asn Gln Gln Ile Asp Phe Gln Ala
        35                  40                  45

Phe His Thr Asn Ala Glu Gly Thr Gly Ala Arg Arg Leu Ala Ala Ala
    50                  55                  60

Ser Pro Ala Pro Thr Ala Pro Glu Thr Phe Pro Tyr Glu Thr Ala Val
65                  70                  75                  80

Ala Lys Cys Lys Glu Lys Leu Pro Val Glu Asp Leu Tyr Tyr Gln Ala
                85                  90                  95

Cys Val Phe Asp Leu Leu Thr Thr Gly Leu Asn Ser Ala Asp Ile Glu
            100                 105                 110

Gly Arg Met Asp Pro Pro Cys Pro Ala Pro Glu Leu Leu Gly Gly Pro
        115                 120                 125

Ser Val Phe Leu Phe Pro Pro Lys Pro Lys Asp Thr Leu Met Ile Ser
    130                 135                 140

Arg Thr Pro Glu Val Thr Cys Val Val Val Asp Val Ser His Glu Asp
145                 150                 155                 160

Pro Glu Val Lys Phe Asn Trp Tyr Val Asp Gly Val Glu Val His Asn
                165                 170                 175

Ala Lys Thr Lys Pro Arg Glu Glu Gln Tyr Asn Ser Thr Tyr Arg Val
            180                 185                 190

Val Ser Val Leu Thr Val Leu His Gln Asp Trp Leu Asn Gly Lys Glu
        195                 200                 205

Tyr Lys Cys Lys Val Ser Asn Lys Ala Leu Pro Ala Pro Ile Glu Lys
    210                 215                 220

Thr Ile Ser Lys Ala Lys Gly Gln Pro Arg Glu Pro Gln Val Tyr Thr
225                 230                 235                 240

Leu Pro Pro Ser Arg Glu Glu Met Thr Lys Asn Gln Val Ser Leu Thr
                245                 250                 255

Cys Leu Val Lys Gly Phe Tyr Pro Ser Asp Ile Ala Val Glu Trp Glu
            260                 265                 270

Ser Asn Gly Gln Pro Glu Asn Asn Tyr Lys Thr Thr Pro Pro Val Leu
        275                 280                 285

Asp Ser Asp Gly Ser Phe Phe Leu Tyr Ser Lys Leu Thr Val Asp Lys
    290                 295                 300

Ser Arg Trp Gln Gln Gly Asn Val Phe Ser Cys Ser Val Met His Glu
305                 310                 315                 320

Ala Leu His Asn His Tyr Thr Gln Lys Ser Leu Ser Leu Ser Pro Gly
                325                 330                 335

Lys

<210>  17
<211>  28
<212>  DNA
<213>  Artificial

<220>
<223>  PCR Primer

<400>  17
ggggaagctt ttccccgcag ccacctcc                                          28

<210>  18
<211>  45
<212>  DNA
<213>  Artificial

<220>
<223>  PCR Primer

<400>  18
gggggaattc aaacgacctt cgatgtcccc gaagaggcca cagtg                       45

<210>  19
<211>  34
<212>  DNA
<213>  Artificial

<220>
<223>  PCR Primer

<400>  19
ggggaagctt ccgtgcaaga tcctcaagtg caac                                   34

<210>  20
<211>  26
<212>  DNA
<213>  Artificial

<220>
<223>  PCR Primer

<400>  20
ccccgaattc aaggccgtcc gccgcg                                            26

<210>  21
<211>  33
<212>  DNA
<213>  Artificial

<220>
<223>  PCR Primer

<400>  21
ccccgaattc aagtccccga agaggccaca gtg                                    33


<210>  22
<211>  26
<212>  DNA
<213>  Artificial

<220>
<223>  PCR Primer

<400>  22
ggggaagctt ctgcggggct gccccc                                            26


<210>  23
<211>  31
<212>  DNA
<213>  Artificial

<220>
<223>  PCR Primer

<400>  23
ccccgaattc aagcccgtgg tgaggaggtc g                                      31


<210>  24
<211>  20
<212>  DNA
<213>  Artificial

<220>
<223>  PCR Primer

<400>  24
atgcagccgc caagggagag                                                   20


<210>  25
<211>  21
<212>  DNA
<213>  Artificial

<220>
<223>  PCR Primer

<400>  25
gcagaacaca gggagcaggg c                                                 21


<210>  26
<211>  9
<212>  PRT
<213>  Artificial

<220>
<223>  Consensus sequence


<220>
<221>  MISC_FEATURE
<222>  (2)..(2)
<223>  Xaa is any amino acid

<220>
<221>  MISC_FEATURE
<222>  (5)..(5)

<223>  Xaa is Val or Leu

<220>
<221>  MISC_FEATURE
<222>  (6)..(6)
<223>  Xaa is any amino acid

<400>  26

Leu Xaa Leu Cys Xaa Xaa Gly Cys Pro
1               5


<210>  27
<211>  28
<212>  PRT
<213>  Artificial

<220>
<223>  Consensus sequence


<220>
<221>  MISC_FEATURE
<222>  (3)..(5)
<223>  Xaa is any amino acid

<220>
<221>  MISC_FEATURE
<222>  (7)..(7)
<223>  Xaa is Lys or His

<220>
<221>  MISC_FEATURE
<222>  (9)..(9)
<223>  Xaa is any amino acid

<220>
<221>  MISC_FEATURE
<222>  (10)..(10)
<223>  Xaa is Leu or Met

<220>
<221>  MISC_FEATURE
<222>  (13)..(13)
<223>  Xaa is any amino acid

<220>
<221>  MISC_FEATURE
<222>  (15)..(15)
<223>  Xaa is any amino acid

<220>
<221>  MISC_FEATURE
<222>  (17)..(17)
<223>  Xaa is Phe or Tyr

<220>
<221>  MISC_FEATURE
<222>  (18)..(18)
<223>  Xaa is His or Gln

<220>
<221>  MISC_FEATURE
<222>  (19)..(19)
<223>  Xaa is Ala or Ser

<220>
<221>  MISC_FEATURE

<222>  (24)..(24)
<223>  Xaa is Val or Leu

<220>
<221>  MISC_FEATURE
<222>  (26)..(26)
<223>  Xaa is any amino acid

<220>
<221>  MISC_FEATURE
<222>  (27)..(27)
<223>  Xaa is Thr or Ser

<400>  27

Thr Ala Xaa Xaa Xaa Cys Xaa Glu Xaa Xaa Pro Val Xaa Asp Xaa Tyr
1               5                   10                  15

Xaa Xaa Xaa Cys Val Phe Asp Xaa Leu Xaa Xaa Gly
            20                  25